# EUROPEAN PATENT SPECIFICATION

(11) **EP 0 914 339 B1**
(45) Date of publication and mention of the grant of the patent: **02.08.2006**
(21) Application number: 97930981.2
(22) Date of filing: 07.05.1997
(51) Int. Cl.: C07K 14/47, C12N 15/12, C12Q 1/68, A61K 48/00, C12N 5/10, C12N 15/62, C07K 16/18, C12N 5/06

(54) **RET LIGAND 3 (RetL3) FOR STIMULATING NEURAL AND RENAL GROWTH**
RET-LIGAND 3 (RetL3), UM NEURALES UND RENALES WACHSTUM ZU STIMULIEREN
RET LIGAND 3 (RetL3) POUR STIMULER LA CROISSANCE NEUTRALE ET RENALE

(30) Priority: 08.05.1996 US 17427 P; 07.06.1996 US 19300 P; 16.07.1996 US 21859 P; 11.04.1997 US 43533
(43) Date of publication of application: 12.05.1999
(73) Proprietor: Biogen Idec MA Inc., Cambridge, Massachusetts 02142 (US)
(72) Inventor: SANICOLA-NADEL, Michele, Winchester, MA 01890 (US); HESSION, Catherine, Hingham, MA 02043 (US); CATE, Richard, L., Weston, MA 02193 (US)
(74) Representative: Pohlman, Sandra M.
(86) International application number: PCT/US1997/007726
(87) International publication number: WO 1997/044356

(56) References cited:
- WO-A-95/16709
- WO-A-97/18240
- WO-A-97/33912
- HILLIER, L. ET AL.: "The WashU-Merck EST Project - y170a10.s1 Homo sapiens cDNA clone 43207 3' " EMBL DATABASE ENTRY HS619153; ACCESSION NUMBER H05619, 23 June 1995, XP002047612
- HILLIER, L. ET AL.: "The WashU-Merck EST Project - ye83h05.s1 Homo sapiens cDNA clone 124377 3' " EMBL DATABASE ENTRY HS13571; ACCESSION NUMBER R02135 , 17 April 1995, XP002047613
- PANDEY A ET AL: "The Ret receptor protein tyrosine kinase associates with the SH2-containing adapter protein Grb10." JOURNAL OF BIOLOGICAL CHEMISTRY, (1995 SEP 15) 270 (37) 21461-3., XP002047614
- CAO T.: "c-ret and signal transduction." CANCER BULLETIN, (1995) 47/2 (119-124)., XP002047615
- PASINI B ET AL: "RET mutations in human disease" TRENDS IN GENETICS, vol. 12, no. 4, April 1996, page 138-144 XP004037254 cited in the application
- JING S ET AL: "GDNF-INDUCED ACTIVATION OF THE RET PROTEIN TYROSINE KINASE IS MEDIATED BY GDNFR-ALPHA, A NOVEL RECEPTOR FOR GDNF" CELL, vol. 85, 28 June 1996, pages 1113-1124, XP002036435
- TREANOR J J ET AL: "Characterization of a multicomponent receptor for GDNF." NATURE, (1996 JUL 4) 382 (6586) 80-3., XP002047616
- HILLIER, ET AL.: "The WashU-Merck EST Project - mj11d08.r1 Soares mouse embryo MbME13.5 14.5 Mus musculus cDNA clone 475791 5' " EMBL DATABASE ENTRY MMAA49894; ACCESSION NUMBER AA049894 (VERSION 2), 31 December 1996, XP002047617 cited in the application
- MASON, I.: "The GDNF receptor: Recent progress and unanswered question" MOL. CELL. NEUROSCI., (1996) VOL. 8, NO. 2-3, PP. 112-119., XP002047618
- ROBERTSON K ET AL: "The GDNF-RET signalling partnership" TRENDS IN GENETICS, vol. 13, no. 1, January 1997, page 1-3 XP004015047

## Description

### FIELD OF THE INVENTION

This invention relates to nucleotide sequences which encode a Ret ligand (RetL), as well as to methods of stimulating neural and renal growth by treating cells with RetL DNA or protein.

### BACKGROUND OF THE INVENTION

One of the goals of current research on cell signaling and receptor activation is to enable therapeutic modulation of processes involved in cell growth and survival. Such processes determine the outcome in diverse medical conditions, including organ failure, fetal development, and tumor growth, among others. Each of these conditions is of worldwide clinical importance, and has limited efficacious treatment options. It is an object of the invention to provide compositions and methods for promoting regeneration or survival of damaged tissue, as well as compositions for treating disorders involving the aberrant growth and development of tissues.

Tissue loss or end-stage organ failure affects millions of people worldwide each year and adds substantially to health care costs. Organ or tissue loss is usually treated by transplanting organs from donors, by surgical reconstruction, or with mechanical devices. Each of these remedies has shortcomings. Transplantation is limited by donor shortage, surgical reconstruction can create other long-term problems, and mechanical devices cannot perform all the functions of a single organ, and therefore cannot prevent progressive deterioration. Thus, a real medical need exists for new solutions to these problems.

Protein factors that affect the growth, differentiation and/or survival of cells may be useful in the treatment of disorders of organs which contain responsive cells. Factors or ligands that interact with receptors of the receptor protein tyrosine kinase (RPTK) family are of particular interest in this regard. These receptors are involved in many cellular programs including cell growth and differentiation, and the genesis of many neoplasias. Thus the factors or ligands that interact with these receptors may prove useful in treating disorders of certain organs where the tissue has been damaged. Alternatively, it may be useful to block the interaction of these factors with their receptors in order to block tumor growth.

The Ret proto-oncogene encodes a receptor tyrosine kinase that is expressed during development in a variety of tissues, including the peripheral and central nervous systems and the kidney. The abnormalities present in ret null mice suggest that Ret is critical for the migration and innervation of enteric neurons to the hindgut, and for proliferation and branching of the ureteric bud epithelium during kidney development (Nature 367, 380-383, 1994). The search for a key component of the Ret signaling pathway, the Ret ligand, has been an area of intensive research.

### SUMMARY OF THE INVENTION

The invention provides an isolated nucleic acid encoding a polypeptide with at least 80% sequence identity to an amino acid sequence selected from the group consisting of murine RetL3 (SEQ ID NO: 17) and human RetL3 (SEQ ID NO:21), wherein said polypeptide interacts with a receptor protein Ret to trigger dimerization of the receptor protein Ret or autophosphorylation of a tyrosine kinase domain of the receptor protein Ret. In one embodiment, this isolated nucleic acid retains at least 80% of the cysteines when aligned with SEQ ID NO:17 or SEQ ID NO:21.

In another embodiment, the encoded polypeptide has at least 90% sequence identity to an amino acid sequence selected from the group consisting of SEQ ID NO: 17 and SEQ ID NO:21. The encoded polypeptide may be SEQ ID NO: 17 or SEQ ID NO:21. The invention further provides an isolated nucleic acid comprising a nucleotide sequence selected from the group consisting of murine retL3 cDNA (SEQ ID NO:16) and human retL3 cDNA (SEQ ID NO:20).

The invention further provides a vector comprising an insert comprising any one of the nucleic acids mentioned above, or a host cell comprising such a vector. A method for producing a polypeptide, comprising the steps of culturing said host cell and recovering the polypeptide expressed from the insert within said host cell, is also encompassed.

In another embodiment of the invention, a polypeptide encoded by one of said nucleic acids is also covered, as is an isolated monoclonal antibody that binds to a polypeptide selected from the group consisting of partial murine RetL3 amino acid (SEQ ID NO:15), murine Ret L3 amino acid (SEQ ID NO:17), partial human RetL3 amino acid (SEQ ID NO:19) and human RetL3 amino acid (SEQ ID NO:21). The invention further relates to a composition comprising said antibody associated to a toxin, an imageable compound or a radionuclide.

The invention further relates to a fusion protein comprising said polypeptide encoded by one of the nucleic acids of the invention fused to an immunoglobulin, a toxin, an imageable compound, or a radionuclide. Finally, any one of the nucleic acids, polypeptides, vectors, antibodies, or fusion proteins of the invention can be for use in therapy or diagnosis.

Disclosed is a purified and isolated DNA molecule coding for a RetL. having the nucleotide sequence of any RetL, but specifically including rat retL1 cDNA (SEQ ID NO:1), partial human retL1 cDNA (SEQ ID NO:8), full-length human retL1 cDNA (SEQ ID NO:10), human retL2 cDNA (SEQ ID NO:12), murine retL3 cDNA (SEQ ID NO:16), partial human retL3 cDNA (SEQ ID NO:18) or human retL3 cDNA (SEQ ID NO:20). Further disclosed is a RetL protein, with an amino acid sequence comprising that of rat RetL1 (SEQ ID NO:2), partial human RetL1 (SEQ ID NO:9), full-length human RetL1 (SEQ ID NO:11), human RetL2 (SEQ ID NO:13), murine RetL3 (SEQ ID NO:17), partial human RetL3 (SEQ ID NO:19) or human RetL3 (SEQ ID NO:21).

A DNA sequence which encompasses the sequence (partial human retL1 cDNA (SEQ ID NO:8)) of the insert DNA of clone HRL20, which is ATCC No. 97604, or the sequence of the insert DNA of clone #230-5A-86-17 (rat retL1 cDNA (SEQ ID NO: 1)), which is ATCC No. 98047, is also described.

Further, a purified and isolated DNA molecule for use in securing expression in a prokaryotic or eukaryotic host cell of a polypeptide product has at least a part of the primary structural conformation and the biological activity of RetL is disclosed; the DNA may be a) a DNA molecule which comprises rat retL1 cDNA, partial human retL1 cDNA, full-length human retL1 cDNA, human retL2 cDNA, murine retL3 cDNA or human retL3 cDNA, or the complementary strand of rat retL1 cDNA, partial human retL1 cDNA, full-length human retL1 cDNA, human retL2 cDNA, murine retL3 cDNA or human retL3 cDNA; b) DNA molecules which hybridize under stringent conditions to the DNA molecules defined in a) or fragments thereof; or c) DNA molecules which, but for the degeneracy of the genetic code, would hybridize to the DNA molecules defined in a) and b). A purified and isolated DNA molecule coding for a polypeptide fragment or variant of a human REtL having the biological activity of a RetL is also described.

Any of the recombinant DNA molecules mentioned above may be operably linked to an expression control sequence.

Also included are vectors and delivery systems which encompass the DNA molecules or constructs defined elsewhere in this specification. The vector may encompass a DNA molecule encoding a RetL or a variant of a RetL.

The invention includes prokaryotic or eukaryotic host cells stably transformed or transfected by a vector comprising a DNA molecule encoding a native or variant RetL.

A purified and isolated human RetL substantially free of other human proteins is specifically disclosed, as is a process for the production of a polypeptide product having part or all of the primary structural conformation and the biological activity of a RetL. Such a process may include the steps of growing, under suitable culture conditions, prokaryotic or eukaryotic host cells transformed or transfected with any DNA molecule disclosed herein, in a manner allowing expression of such polypeptide product, and recovering a RetL. The polypeptide product of the expression in a procaryotic or eukaryotic host cell of a DNA is also included.

Also disclosed are proteins and protein fragments, variants and derivatives, whether soluble or membrane bound. In some cases, the protein has an amino acid sequence which comprises rat RetL1, partial human RetL1, full-length human RetL1. human RetL2, murine RetL3, or human RetL3, or is a variant of one of these sequences. In other cases, the protein is a fusion protein including Ret or a RetL, fused to another molecule or molecular fragment, such as an immunoglobulin, toxin, imageable compound or radionuclide. Also included are chimeric molecules of RetL.

Further disclosed are specific monoclonal antibodies to a RetL of the invention. Such an antibody may be associated with a toxin, imageable compound or radionuclide. Such antibodies may be produced by hybridoma cell lines, including AA.FF9, AA.HE3, AF.E9, BA.B1, BB.B6, AA.GE7, CD.F11, AH.E3, CD.G4, AG.E7, BD.G6 and BH.G8, as well as subclones of these hybridomas. Antibodies produced by these hybridomas or subclones of these hybridomas are also described.

The disclosure futher includes the use of a therapeutically effective amount of a compound which interacts with cellular Ret and thereby induces autophosphorylation of Ret for promoting growth of new tissue, or promoting survival of damaged tissue in a subject. The compound may be RetL1, RetL2, or RetL3, a fragment of a full-length RetL, or an antibody which binds to Ret. The compound may be administered concurrently with a therapeutically effective amount of a second compound, such as GDNF, neurturin or a GDNF-related molecule. While tissues of interest may include any tissue, preferred tissues include renal tissue, neural tissue, heart, stomach, small intestine, spinal cord, or lung. In one embodiment, the RetL is a soluble RetL. The above subject may be human.

In another use disclosed, Ret signal transduction between a first cell expressing a RetL and a second cell is inhibited by contacting the first cell with a soluble Ret protein or with an antibody to the RetL. The soluble Ret protein may be a fusion protein.

Also described is a method for targeting a toxin, imageable compound or radionuclide to a cell expressing Ret, encompassing contacting the cell with a RetL fusion protein or an anti-Ret antibody conjugated to a toxin, imageable compound or radionuclide. The RetL can be RetL1. RetL2 or RetL3. In another method, growth of a tumor cell which expresses Ret is suppressed, with a step of the method being contacting the cell with a fusion protein of a RetL and a toxin or radionuclide, or an anti-Ret antibody conjugated to a toxin or radionuclide. The cell may be within a subject, and the protein or the conjugated antibody is administered to the subject.

Also disclosed is a method for targeting a toxin, imageable compound or radionuclide to a cell expressing a RetL, comprising contacting the cell with a fusion protein comprising Ret and a toxin, imageable compound or radionuclide, or an anti-RetL antibody conjugated to a toxin, imageable compound or radionuclide. Another embodiment includes the method of suppressing growth of a tumor cell which expresses a RetL, comprising contacting the cell with a fusion protein of Ret and a toxin or radionuclide or with an anti-RetL antibody conjugated to a toxin or radionuclide; the cell may be within a subject, and the protein administered to the subject.

The RetL disclosed is RetL1, RetL2 or RetL3, or a variant or fragment of RetL1, RetL2 or RetL3.

Use of the disclosed substances for gene therapy are also described. One embodiment is the use of a vector comprising a DNA molecule encoding a RetL for treating a subject with a disorder of Ret metabolism, as well as the use for promoting growth of new tissue in a subject. Another embodiment includes the use of a therapeutically effective amount of a vector encoding a RetL for promoting survival of damaged tissue in a subject.

### BRIEF DESCRIPTION OF THE DRAWINGS

FIGURE is a cDNA sequence (SEQ ID NO: 1) and deduced amino acid sequence (SEQ ID NO:2) of rat RetL1. The nucleotide sequence extends from base pair 201 through base pair 1700 of SEQ ID NO:1, and contains the entire open reading frame.
FIGURE 2A is a partial cDNA sequence (SEQ ID NO:8) and deduced amino acid sequence (SEQ ID NO:9) of human RetL1. This sequence is that of the insert of clone HRL20, deposited as ATCC No. 97604.
FIGURE 2B is a composite full-length DNA sequence (SEQ ID NO:10) and deduced amino acid sequence (SEQ ID NO:11) of human RetLI.
FIGURE 3A is a comparison of the nucleotide sequence of human RetLI (upper line of sequence) with that of rat RetL1 sequence (lower line of sequence). Vertical lines between nucleotides show identity at a position, while a dot indicates a gap at that position.
FIGURE 3B is a comparison of the amino acid sequence of human RetL1 (upper line of sequence) with that of rat RetL1 sequence (lower line of sequence). Vertical lines between corresponding amino acids show identity at a residue, while a dot indicates a conservative substitution at that residue.
FIGURE 4A is a schematic diagram of a possible role for Ret and RetL in the interaction between a metanephric mesenchyme cell and a ureteric bud cell.
FIGURE 4B is a schematic diagram of a method of screening transfectants of a cDNA library for clones that express a RetL. The presence of expressed RetL on transfectants is detected by assessing the binding by those transfectants either of Ret/IgG fusion protein or of Ret/alkaline phosphatase fusion protein.
FIGURE 5 is a schematic diagram showing the construction of the plasmids used to express the rat Ret/IgG fusion protein.
FIGURE 6 is a schematic diagram showing the construction of the plasmids used to express the human Ret/IgG fusion protein.
FIGURE 7 is a cDNA sequence (SEQ ID NO:12) and deduced amino acid sequence (SEQ ID NO:13) of human retL2, as found in clone DSW240. The protein reading frame is contained within nucleotides 25 to 141b.
FIGURE 8 is a comparison of the amino acid sequence of human RetL2 (upper line of sequence) with that of human RetL1 sequence (lower line of sequence). Vertical lines between amino acids show identity at a position, while a dot indicates a gap at that position.
FIGURE 9 is a cDNA sequence (SEQ ID NO:16) and deduced amino acid sequence (SEQ ID NO:17) of murine RetL3.
FIGURE 10 is a cDNA sequence (SEQ ID NO:20) and deduced amino acid sequence (SEQ ID NO:21) of human RetL3.

### DETAILED DESCRIPTION OF THE INVENTION

### Sequence Identification Numbers

Nucleotide and amino acid sequences referred to in the specification have been given the following sequence identification numbers:
SEQ ID NO:1- rat retL1 cDNA
SEQ ID NO:2 - rat RetL1 aa
SEQ ID NO:3 - oligomer kid-13
SEQ ID NO:4 - oligomer kid-14
SEQ ID NO:5 - oligomer kid-15
SEQ ID NO:6 - extracellular rat ret cDNA
SEQ ID NO:7 - extraretlular rat Ret aa
SEQ ID NO:8 - partial human retL1 cDNA
SEQ ID NO:9 - panial human RetL1 aa
SEQ ID NO:10 - human retL1 cDNA
SEQ ID NO:11 - human RetL1 aa
SEQ ID NO:12 - human retL2 cDNA
SEQ ID NO:13 - human RetL2 aa
SEQ ID NO:14 - partial murine retL3 cDNA (EST AA50083)
SEQ ID NO:15 - partial murine RetL3 aa
SEQ ID NO:16 - murine retL3 cDNA
SEQ ID NO:17 - murine RetL3 aa
SEQ ID NO: 18 - partial human retL3 cDNA
SEQ ID NO:19 - partial human RetL3 aa
SEQ ID NO:20 - human retL3 cDNA
SEQ ID NO:21 - human retL3 aa

### Definitions

As used herein, the term "RetL" means any protein which specifically interacts with the receptor protein Ret, and which when it interacts with Ret triggers Ret dimerization and/or autophosphorylation of the tyrosine kinase domain of Ret. The DNA sequences which code for RetL and for Ret are termed "retL" and "ret", respectively. A ligand may be soluble, or present as a membrane-bound molecule on the same or on a different cell as the Ret molecule for which it is triggering autophosphorylation. In certain uses or interactions with Ret, the ligand may require additional molecules to trigger autophosphorylation. Ligands include co-receptors or accessory ligand cofactors. Ligands further include anti-Ret mAbs which act as Ret antagonists, triggering Ret dimerization and autophosphorylation. The ligand may also be modified in various ways, such as incorporated as a portion of a fusion protein, such as with a toxin or radionuclide.

By "alignment of sequences" is meant the positioning of one sequence, either nucleotide or amino acid, with that of another, to allow a comparison of the sequence of relevant portions of one with that of the other. An example of one method of this procedure is given in Needleman et al. (J. Mol. Biol. 48:443-453, 1970). The method may be implemented conveniently by computer programs such as the Align program (DNAstar. Inc.). As will be understood by those skilled in the art, homologous or functionally equivalent sequences include functionally equivalent arrangements of the cysteine residues within the conserved cysteine skeleton, including amino acid insertions or deletions which alter the linear arrangement of these cysteines, but do not materially impair their relationship in the folded structure of the protein. Therefore, internal gaps and amino acid insertions in the candidate sequence are ignored for purposes of calculating the level of amino acid sequence homology or identity between the candidate and reference sequences. One characteristic frequently used in establishing the homology of proteins is the similarity of the number and location of the cysteine residues between one protein and another.

By "cloning" is meant the use of in vitro recombination techniques to insert a particular gene or other DNA sequence into a vector molecule. In order to successfully clone a desired gene, it is necessary to employ methods for generating DNA fragments, for joining the fragments to vector molecules, for introducing the composite DNA molecule into a host cell in which it can replicate, and for selecting the clone having the target gene from amongst the recipient host cells.

By "cDNA" is meant complementary or copy DNA produced from an RNA template by the action of RNA-dependent DNA polymerase (reverse transcriptase). Thus a "cDNA clone" means a duplex DNA sequence complementary to an RNA molecule of interest, carried in a cloning vector.

By "cDNA library" is meant a collection of recombinant DNA molecules containing cDNA inserts which together comprise a representation of the mRNA molecules present in an entire organism or tissue, depending on the source of the RNA templates. Such a cDNA library may be prepared by methods known to those of skill, and described, for example, in Maniatis et al., Molecular cloning: A Laboratory Manul, *supra.* Generally, RNA is first isolated from the cells of an organism from whose genome it is desired to clone a particular gene. Preferred for the purposes of the present invention are mammalian, and particularly human, cell lines. Alternatively, RNA may be isolated from a tumor cell, derived from an animal tumor, and preferably from a human tumor. Thus, a library may be prepared from, for example, a human adrenal tumor, but any tumor may be used.

As used herein, the term "DNA polymorphism" refers to the condition in which two or more different nucleotide sequences can exist at a particular site in DNA.

"Expression vector" includes vectors which are capable of expressing DNA sequences contained therein, i.e., the coding sequences are operably linked to other sequences capable of effecting their expression. It is implied, although not always explicitly stated, that these expression vectors must be replicable in the host organisms either as episomes or as an integral part of the chromosomal DNA. A useful, but not a necessary, element of an effective expression vector is a marker encoding sequence, which is a sequence encoding a protein which results in a phenotypic property (e.g. tetracycline resistance) of the cells containing the protein which permits those cells to be readily identified. In sum, "expression vector" is given a functional definition, and any DNA sequence which is capable of effecting expression of a specified contained DNA code is included in this term, as it is applied to the specified sequence. Such vectors are frequently in the form of plasmids, so "plasmid" and "expression vector" are often used interchangeably. However, the invention is intended to include such other forms of expression vectors, including phage, which serve equivalent functions and which may from time to time become known in the art.

Similarly, a "functional derivative" of a gene of any of the proteins of the present invention is meant to include "fragments", "variants", and "analogues" of the gene, which may be "substantially similar" in nucleotide sequence, and which encode a molecule possessing similar activity.

"GDNF-related molecule" means any moleule which is at least 40% homologous to either GDNF or neurturin, and is also capable of specifically binding a RetL.

The term "gene" means a polynucleotide sequence encoding a peptide.

By "homogeneous" is meant, when referring to a peptide or DNA sequence, that the primary molecular structure (i.e., the sequence of amino acids or nucleotides) of substantially all molecules present in the composition under consideration is identical.

The term "oligonucleotide" as used herein in referring to probes, oligomer fragments to be detected, oligomer controls, unlabeled blocking oligomers and primers for amplification of sequences is defined as a molecule comprised of more than three deoxyribonucleotides or ribonucleotides. Its exact size will depend on many factors, which in turn depend on the ultimate function or use of the oligonucleotide.

The term "probe" refers to a ligand of known qualities capable of selectively binding to a target antiligand. As applied to nucleic acids, the term "probe" refers to a strand of nucleic acid having a base sequence complementary to a target strand.

"Recombinant host cells" refers to cells which have been transformed with vectors constructed using recombinant DNA techniques. As defined herein, the antibody or modification thereof produced by a recombinant host cell is by virtue of this transformation, rather than in such lesser amounts, or more commonly, in such less than detectable amounts, as would be produced by the untransformed host.

As used herein, the terms "restriction endonucleases" and "restriction enzymes" refer to bacterial enzymes each of which cut double-stranded DNA at or near a specific nucleotide sequence.

As used herein, the term "restriction fragment length polymorphism" ("RFLP") refers to the differences among individuals in the lengths of a particular restriction fragment.

A molecule is said to be "substantially similar" to another molecule if the sequence of amino acids in both molecules is substantially the same, and if both molecules possess a similar biological activity. Thus, provided that two molecules possess a similar activity, they are considered variants as that term is used herein even if one of the molecules contains additional amino acid residues not found in the other, or if the sequence of amino acid residues is not identical. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed, for example, in Remington's Pharmaceutical Sciences, 16th ed., Mack Publishing Co., Easton, Penn. (1980).

By "vector" is meant a DNA molecule, derived from a plasmid or bacteriophage, into which fragments of DNA may be inserted or cloned. A vector will contain one or more unique restriction sites, and may be capable of autonomous replication in a defined host or vehicle organism such that the cloned sequence is reproducible.

By "substantially pure" is meant any protein of the present invention, or any gene encoding any such protein, which is essentially free of other proteins or genes, respectively, or of other contaminants with which it might normally be found in nature, and as such exists in a form not found in nature.

### Compounds of the Invention

The invention includes cDNA coding for a RetL. such as the nucleotide sequence of murine retL3 cDNA or human retL3 cDNA. In addition, the compounds of the invention include sequences which include the above sequences, or are derivatives of one of these sequences. The invention also includes vectors, liposomes and other carrier vehicles which encompass one of these sequences or a derivative of one of these sequences. The invention also includes proteins transcribed and translated from murine retL3 cDNA or human retL3 cDNA, including but not limited to murine RetL3, or human RetL3, and their derivatives and variants.

One embodiment of the invention includes soluble variants of a RetL. Soluble variants lack at least a portion of the intramembrane section of the native RetL. In some examples, the soluble variant lacks the phosphatidylinositol glycan linkage of the native RetL. Soluble variants include fusion proteins which encompass derivatives of RetL that lack a phosphatidylinositol motif.

Variants can differ from naturally occurring RetL in amino acid sequence or in ways that do not involve sequence, or both. Variants in amino acid sequence are produced when one or more amino acids in naturally occurring RetL is substituted with a different natural amino acid, an amino acid derivative or non-native amino acid. Particularly preferred variants include naturally occurring RetL, or biologically active fragments of naturally occurring RetL, whose sequences differ from the wild type sequence by one or more conservative amino acid substitutions, which typically have minimal influence on the secondary structure and hydrophobic nature of the protein or peptide. Variants may also have sequences which differ by one or more non-conservative amino acid substitutions, deletions or insertions which do not abolish the RetL biological activity. Conservative substitutions typically include the substitution of one amino acid for another with similar characteristics such as substitutions within the following groups: valine, glycine; glycine, alanine; valine, isoleucine; aspartic acid, glutamic acid; asparagine, glutamine; serine, threonine; lysine, arginine: and phenylalanine, tyrosine. The non-polar (hydrophobic) amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid.

Other conservative substitutions can be taken from the table below, and yet others are described by Dayhoff in the Atlas of Protein Sequence and Structure (1988).

**TABLE 1: CONSERVATIVE AMINO ACID REPLACEMENTS**

| For Amino Acid | Code | Replace with any of |
|---|---|---|
| Alanine | A | D-Ala, Gly, beta-Ala, L-Cys, D-Cys |
| Arginine | R | D-Arg, Lys, homo-Arg, D-homo-Arg, Met, D-Met, Ile, D-Ile, Orn, D-Om |
| Asparagine | N | D-Asn, Asp, D-Asp, Glu, D-Glu, Gin, D-Gln |
| Aspartic Acid | D | D-Asp, D-Asn, Asn, Glu,D-Glu, Gin, D-Gln |
| Cysteine | C | D-Cys, S-Me-Cys,Met,D-Met,Thr, D-Thr |
| Glutamine | Q | D-Gln.Asn, D-Asn,Glu,D-Glu, Asp, D-Asp |
| Glutamic Acid | E | D-Glu, D-Asp, Asp, Asn, D-Asn. Gin. D-Gln |
| Glycine | G | Ala, D-Ala,Pro. D-Pro, Beta-Ala, Acp |
| Isoleucine | I | D-Ile. Val. D-Val, Leu, D-Leu. Met. D-Met |
| Leucine | L | D-Leu, Val, D-Val, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val, Norleu |
| Phenylalanine | F | D-Phe, Tyr, D-Thr, L-Dopa, His, D-His, Trp, D-Trp, Trans 3,4 or 5-phenylproline, cis 3,4 or 5 phenylproline |
| Proline | P | D-Pro, L-I-thioazolidine-4-carboxylic acid, D- or L-1-oxazolidine-4-carboxylic acid |
| Serine | S | D-Ser, Thr, D-Thr, allo-Thr, Met, D-Met, Met(O), D-Met(O), Val, D-Val |
| Threonine | T | D-Thr, Ser, D-Ser, allo-Thr, Met, D-Met, Met)O, D-Met(O), Val, D-Val |
| Tyrosine | Y | D-Tyr,Phe, D-Phe, L-Dopa, His,D-His |
| Valine | V | D-Val, Leu,D-Leu,Ile,D-Ile, Met, D-Met |

Other variants within the invention are those with modifications which increase peptide stability. Such variants may contain, for example, one or more non-peptide bonds (which replace the peptide bonds) in the peptide sequence. Also included are: variants that include residues other than naturally occurring L-amino acids, such as D-amino acids or non-naturally occurring or synthetic amino acids such as beta or gamma amino acids and cyclic variants. Incorporation of D- instead of L-amino acids into the polypeptide may increase its resistance to proteases. See, e.g., U.S. Patent 5,219,990.

The peptides of this invention may also be modified by various changes such as insertions, deletions and substitutions, either conservative or nonconservative where such changes might provide for certain advantages in their use. Splice variants are specifically included in the invention.

In addition to substantially full-length polypeptides, the present invention provides for biologically active fragments of the polypeptides. A RetL polypeptide or fragment is biologically active if it exhibits a biological activity of naturally occurring RetL. Such biological activities include the ability to specifically bind the extracellular portion of Ret, with an affinity that is at least 50% of, and preferably at least equal to, the affinity of naturally occurring RetL for the extracellular portion of Ret. Another biological activity is the ability to bind to an antibody which is directed at an epitope which is present on naturally occurring RetL.

In other embodiments, variants with amino acid substitutions which are less conservative may also result in desired derivatives, e.g., by causing changes in charge, conformation and other biological properties. Such substitutions would include for example, substitution of hydrophilic residue for a hydrophobic residue, substitution of a cysteine or proline for another residue, substitution of a residue having a small side chain for a residue having a bulky side chain or substitution of a residue having a net positive charge for a residue having a net negative charge. When the result of a given substitution cannot be predicted with certainty, the derivatives may be readily assayed according to the methods disclosed herein to determine the presence or absence of the desired characteristics.

Generally, substitutions that may be expected to induce changes in the functional properties of Ret polypeptides are those in which: (I) a hydrophilic residue, e.g., serine or threonine, is substituted by a hydrophobic residue, e.g., leucine, isoleucine, phenylalanine, or alanine; (ii) a cysteine residue is substituted for (or by) any other residue; (iii) a residue having an electropositive side chain, e.g., lysine, arginine or histidine, is substituted for (or by) a residue having an electronegative charge, e.g., glutamic acid or aspartic acid; or (iv) a residue having a bulky side chain, e.g., phenylalanine, is substituted for (or by) one not having such a side chain, e.g., glycine.

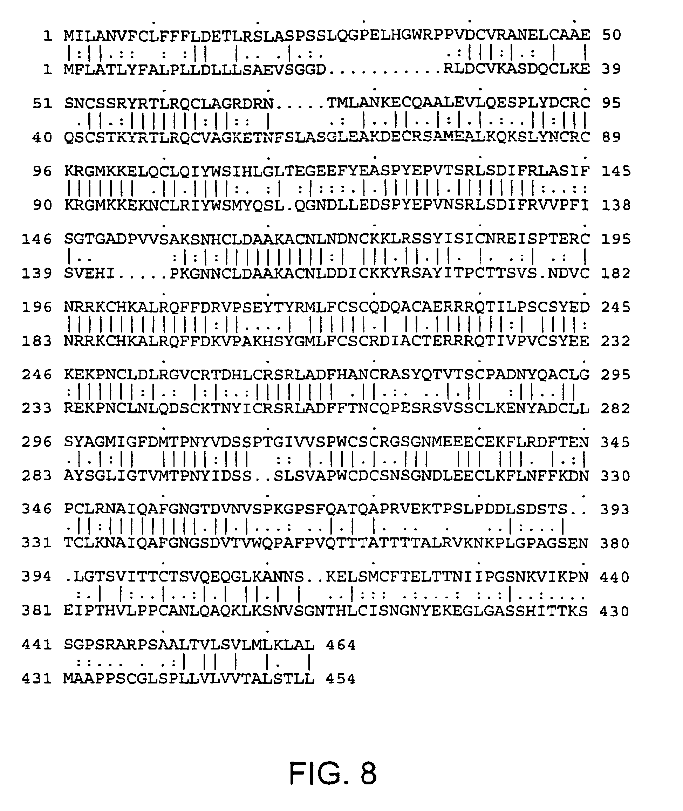

Variants disclosed herein include proteins and peptides with amino acid sequences having at least sixty percent homology with rat RetL1 (SEQ ID NO:2), partial human RetL1 (SEQ ID NO:9), full-length human RetL1 (SEQ ID NO:11), human RetL2 (SEQ ID NO:13), murine RetL3 (SEQ ID NO:17), partial human RetL3 (SEQ ID NO:19) or human RetL3 (SEQ ID NO:21). More preferably the sequence homology is at least eighty, at least ninety percent, or at least ninety-five percent. For the purposes of determining homology the length of comparison sequences will generally be at least 8 amino acid residues, usually at least 20 amino acid residues. Variants of the compounds of the invention also includes any protein which 1) has an amino acid sequence which is at least forty percent homologous to a RetL protein of the invenion, and also which 2) after being placed in an optimal alignment with the RetL sequence (as depicted for RetL1 and RetL2 in Figure 8), has at least 80% of its cysteine residues alligned with cysteines in the RetL protein of the invention.

Just as it is possible to replace substituents of the scaffold, it is also possible to substitute functional groups which are bound to the scaffold with groups characterized by similar features. Such modifications do not alter primary sequence. These will initially be conservative, i.e.. the replacement group will have approximately the same size, shape, hydrophobicity and charge as the original group. Non-sequence modifications may include, for example, in vivo or in vitro chemical derivatization of portions of naturally occurring RetL, as well as changes in acetylation, methylation, phosphorylation, carboxylation or glycosylation.

Also included within the invention are agents which specifically bind to a protein of the invention, or a fragment of such a protein. These agents include Ig fusion proteins and antibodies (including single chain, double chain, Fab fragments, and others, whether native, humanized, primatized, or chimeric). Additional descriptions of these categories of agents are in PCT application 95/16709.

### EXPERIMENTAL PROCEDURE

### Overview of Strategy

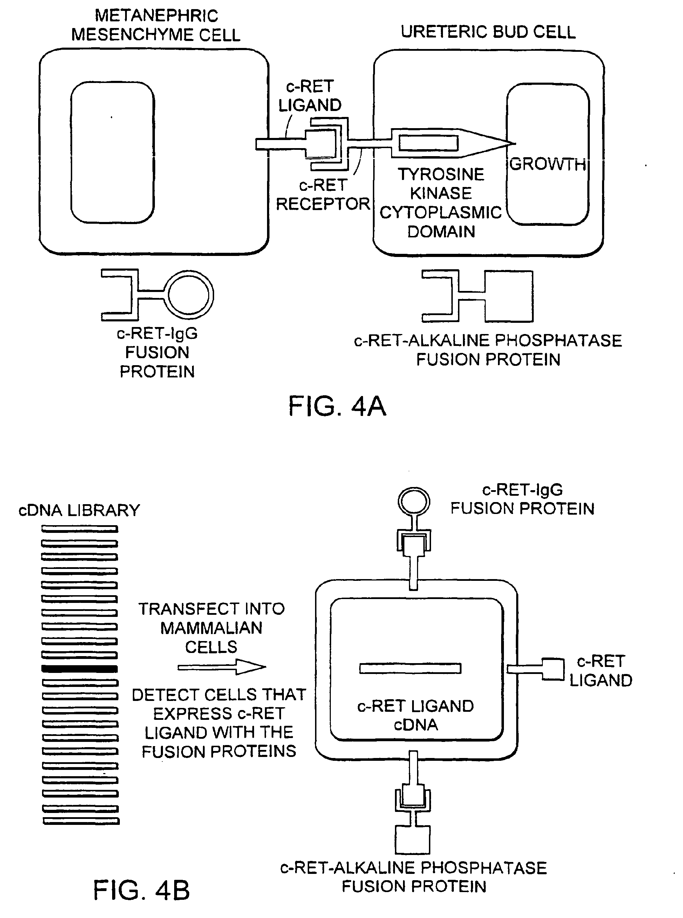

The general strategy used to clone RetL1 is shown in Figures 4A and 4B. Our strategy was based on the premise that at least a RetL is expressed on the metanephric mesenchyme of the developing kidney as a membrane protein (although it is possible that the ligand is also expressed in a soluble form; Figure 4A). The RetL interacts with the Ret receptor on the ureteric bud cell, activating its tyrosine kinase cytoplasmic domain and sending a signal to the nucleus, which in turn activates genes involved in the growth and branching of the ureteric bud. Therefore, proteins containing the extracellular domain of Ret fused to either the F_{c} portion of human immunoglobulin G1 (IgG1) or alkaline phosphatase (AP) can be used as part of a strategy to clone RetL as shown in Figure 4B. The fusion proteins, the expression libraries and other reagents used in the cloning of RetL1 are described below.

We first isolate a cDNA for rat RetL1 and then use it as a probe to isolate a cDNA for human RetL1. cDNAs are subsequently isolated for RetL2 and RetL3.

### Generation of Reagents Required for Direct Expression Cloning of Ret Ligands

### 1. Isolation of cDNA Encoding Rat Ret Extracellular Domain

To identify RetL1. fusion proteins are generated consisting of the extracellular domains of either rat or human Ret fused to a protein, in one example the human Fc portion of IgG1, and in another example alkaline phosphatase. Both fusion partners can be easily assayed to detect cells which express the ligand as illustrated in Figure 4B.

Since a cDNA coding for rat Ret has never been disclosed, we isolate a cDNA encoding the extracellular domain of the rat Ret receptor using the Reverse Transcriptase-Polymerase Chain Reaction (RT-PCR) method. We compare the two nucleotide sequences for human (Genbank Accession numbers M57464 and X15262) and murine (Genbank Accession number X67812) ret and design oligonucleotide primers from regions of high identity between the two sequences. A sense oligomer called kid-013 (SEQ ID NO:3; contains nucleotides 150-169 of Genbank sequence X15262) is chosen from the 5' end of the human ret cDNA sequence overlapping the ATG initiation codon. It includes nucleotides on its 5' end encoding a Not 1 restriction site for the purpose of cloning. Two antisense oligomers called kid-014 (SEQ ID

NO:4; contains the complement of nucleotides 1819-1839 of Genbank sequence M57464) and kid-015 (SEQ ID NO:5; contains the complement of nucleotides 1894-1914 of Genbank sequence X67812) are chosen, respectively, from the human and murine cDNA sequences immediately 5' to the sequences that encode the transmembrane domains. Oligomers kid-014 and kid-015 contain additional nucleotides at their 5' ends that encode a Sall restriction site for the purpose of cloning.

Total RNA is isolated from the day 14 embryonic rat kidney and mRNA is purified using oligo-dT chromatography. mRNA is converted to cDNA using AMV reverse transcriptase and the cDNA is converted to double stranded cDNA and amplified using Taq polymerase in a standard polymerase chain reaction with oligomers kid-013 and kid-015. The synthesis of a 1942 bp PCR fragment is confirmed by running an aliquot of the PCR reaction on a 1% agarose gel. The rest of the PCR fragment is digested with Not1 and Sal1 and cloned into pSAE132 previously digested with Not1 and Sal1. The resulting plasmid is called pJC011. The entire insert of plasmid pJC011 contained between the Not1 and Sal1 sites is sequenced, and is shown as extracellular rat ret cDNA. SEQ ID NO:6. A translation of this sequence reveals the peptide sequence (SEQ ID NO:7) for extracellular rat Ret. Because oligomers for PCR were chosen from human and mouse sequences of ret, it is possible that the nucleotide sequence shown as that of extracellular rat ret cDNA, and the peptide sequence shown as that of extracellular rat Ret, may differ from the natural rat ret nucleotide and Ret peptide sequences in the regions of kid-013 and kid-015 sequences. Subsequently, ret cDNA clones are isolated from a day 18 rat embryonic kidney cDNA library and a few nucleotide changes are observed in the primer regions resulting in two amino acid changes. One change is in the signal sequence (arginine at position 5 to threonine) and one change is near the end of the extracellular domain (glutamic acid at position 633 to alanine). Both of these changes should not affect ligand binding.

### 2. Ret/IgG Fusion Proteins

Fusion proteins are generated consisting of the extracellular domains of the rat (aa residues #1-637) and human (aa residues #1-636) Ret receptors fused to the Fc portion of human IgG1.

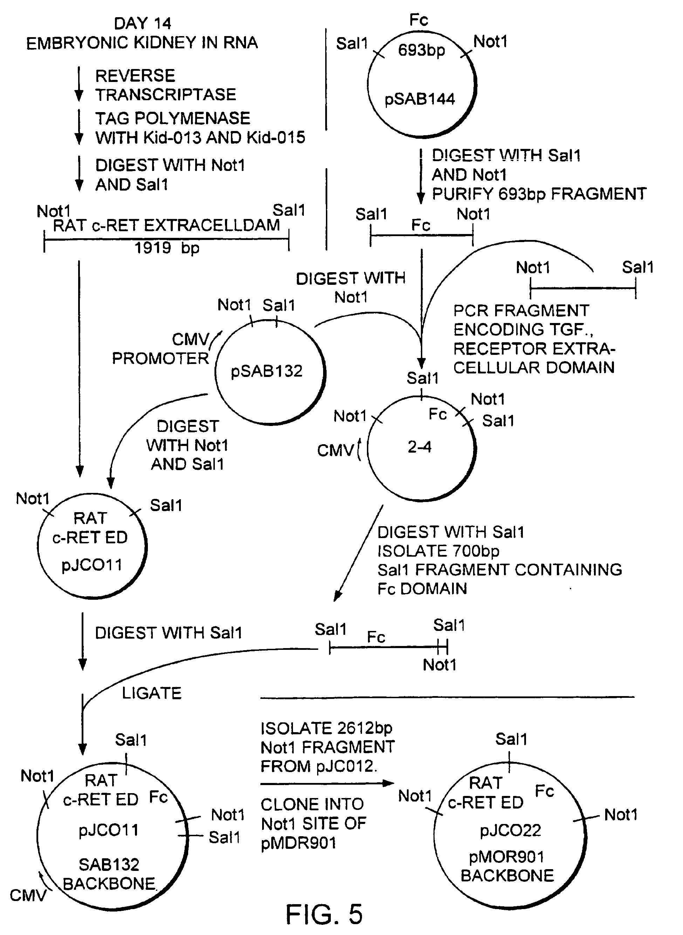

The construction of the plasmids used to express the rat Ret/IgG fusion protein is shown schematically in Figure 5. In order to construct a gene encoding the rat Ret/IgG fusion protein, we digest pJC011 (described above) containing the rat Ret extracellular domain with Sal1, and ligate it to a 700 bp Sal fragment from plasmid 2-4, to create plasmid pJC012. This Sal1 fragment contains part of the Fc domain of human IgG1 originally derived from plasmid pSAB144. Plasmid 2-4 was created previously via a three way ligation: a Not1 - Sal1 fragment generated by PCR containing the extracellular domain of the rabbit TGF-beta type II receptor; a 693 bp Sal1- Not1 fragment from pSAB144 containing part of the Fc domain of human IgG1; and Not1 digested pSAB132. As shown in Figure 5, a fragment containing the Fc domain can be released from the 2-4 plasmid as a 700 bp Sal1 fragment. pJC012 is transfected into COS cells and the rat Ret/IgG fusion protein is purified from the medium 48 hrs later using Protein-A Sepharose chromatography. In order to make a stable cell line producing the rat Ret/IgG protein, the 2612 bp Not1 fragment from pJC012 containing the entire rat Ret/IgG fusion protein is isolated and cloned into the Not1 site of expression vector pMDR901. The resulting plasmid is called pJC022. Plasmid pJC022 is transfected into CHO cells to generate stable cell lines. The highest producing cell line is suspension adapted. Typical yields for the rat Ret/IgG CHO line are 75mg/L.

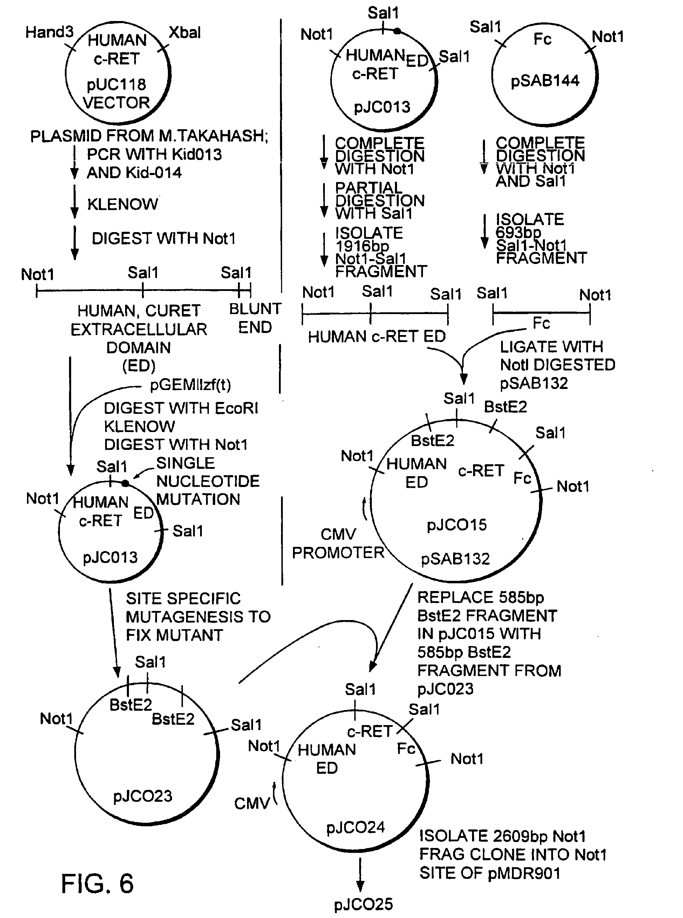

The construction of the plasmids used to express the human Ret/IgG fusion protein is shown schematically in Figure 6. In order to construct a gene encoding the human Ret/IgG fusion protein, we obtain a plasmid containing a cDNA encoding the human Ret receptor from Dr. M.Takahashi (Department of Pathology, Nagoya University. School of Medicine. Nagoya, Japan). A PCR fragment is generated from this plasmid using oligomers kid-013 and kid-014. The PCR fragment is treated with Klenow fragment followed by digestion with Not1 to produce a PCR fragment with a sticky Not1 end and one blunt end. This fragment is cloned into the vector pGEM11zf(+) previously digested with EcoR1, treated with Klenow fragment, and digested with Not1, in order to generate a sticky Not1 end and one blunt end. The resulting plasmid is called pJC013. The 1916 bp Not1 - Sal1 fragment from pJC013 is isolated after a complete digestion with Not1 and a partial digestion with Sal1, and ligated to the 693 bp Sal1 - Not1 fragment from pSAB144 containing part of the Fc domain of human IgG1, and the pSAB132 expression vector digested with Not1. The resulting plasmid is called pJC015. The insert in plasmid pJC013 is sequenced and found to contain a single nucleotide difference which changes one amino acid in the extracellular domain of human Ret (Genbank sequence M57464 has a C at position 812, whereas pJC013 has a T at the corresponding position; this results in a change in amino acids from alanine to valine at position 294 of the human Ret protein sequence). This nucleotide is corrected back to the C residue specified by Genbank sequence M57464 by site specific mutagenesis of plasmid pJC013, producing plasmid pJC023. A 585 bp BstE2 fragment from pJC023 containing the repaired nucleotide sequence is isolated and cloned into plasmid pJC015 from which the 585 bp BstE2 fragment containing the variant nucleotide has been removed. The new plasmid is called pJC024. The 2609 bp Not1 fragment from pJC024 containing the entire human Ret/IgG fusion protein is isolated and cloned into the Not1 site of expression vector pMDR901. The resulting plasmid is called pJC025. Plasmid pJC025 is transfected into CHO cells to generate stable cell lines. The highest producing cell line is suspension adapted. Typical yields for the human Ret/IgG CHO line are 6 mg/L.

Further details on production of the vectors employed in the methods of the invention are given in PCT applications 94/01456 and 92/02050, the specifications of which are herein incorporated by reference.

### 3. Bioactivity of the Ret/IgG Fusion Proteins

To determine if the Ret/IgG fusion proteins that we produce are bioactive and therefore would be good screening reagents for the cloning of a RetL, we perform several organ culture assays for bioactivity. The organ culture assay consists of growing day 13-14 embryonic rat kidneys in organ culture for 3-5 days in the presence of the Ret/Ig fusion protein at a concentration of 50 ug/ml. Kidneys are also cultured in the presence of LFA-3TIP/IgG or vehicle buffer. After the culture period, some of the kidneys are stained with the fluorescent lectin Dolichos Biflorus Agglutinin (DB lectin) which stains the collecting duct tissues, which are epithelial cells derived from the ureteric bud. These "DB" positive cells mark the Ret-positive cells, since Ret is expressed in the ureteric bud and its epithelial derivatives. This provides a gross assessment of the Ret/IgG fusion protein on the growth and development of the embryonic kidney. There is a clear difference in collecting duct morphology and growth between kidneys which have been cultured with LFA-3TIP and those cultured with the rat Ret/IgG fusion protein.. The Ret/IgG-treated kidneys have collecting ducts which show significantly less branching and are typically smaller overall.

Paraffin sections are prepared from other kidneys for histological examination. Embryonic kidneys are treated with control buffer or with Ret/IgG, then stained with hematoxylin and eosin. The Ret/Ig-treated embryonic kidney exhibits less branching of the collecting ducts than the control buffer-treated embryonic kidneys. In addition, Ret/IgG-treated kidneys have fewer tubules. We have also observed this effect with the human Ret/IgG fusion protein. These observations are consistent with the fusion proteins blocking the inductive signal between the mesenchyme and the ureteric bud. Therefore we conclude that the fusion protein is a good reagent for cloning a RetL.

### 4. Ret/alkaline phosphatase Fusion Protein

Receptor/alkaline phosphatase (AP) fusion proteins have been used successfully to identify and clone ligands for c-kit (Cell 63:185. 1990), ligands for members of the eph family of orphan receptors (Cell 79:157, 1994), and recently to clone a receptor for leptin, the product of the ob gene (Cell 83:1263, 1995). Plasmids encoding the rat Ret/AP fusion protein are constructed and the rat Ret/AP protein is produced in COS7 cells in cell factories. Subsequently, a stable NIH3T3 cell line is generated expressing on average 10 mg/L of fusion protein. SDS-PAGE analysis of the rat Ret/AP protein indicates that its size is consistent with the predicted molecular weight, and gel filtration analysis indicates that it is produced as a dimer. Partial purification is achieved by affinity chromatography on an anti-AP column.

### 5. Anti-Ret Antibodies

A rabbit polyclonal antibody is generated against the rat Ret/IgG fusion protein. The antibody works on Western blots, FACS analysis of Ret positive cell lines, and immunohistochemistry of embryonic kidney sections.

A panel of hamster anti-rat Ret monoclonal antibodies is generated. Rat Ret/IgG fusion protein, coupled to Protein A Sepharose, is used to immunize Armenian hamsters. 316 clones are obtained after the fusion and screened for their ability to bind rat Ret fusion proteins and/or human IgG in an ELISA assay. 11 clones produce antibodies that bind only to rat Ret/IgG (and rat Ret/AP), but not human IgG. The cross reactivity to human Ret is assayed by FACS; four clones produce antibodies that can bind to the Ret positive human cell line THP-1. The following table summarizes the Ret binding properties of twelve monoclonal antibodies.

| Clone | ELISA rat Ret/Ig | FACS human THP-1 |
|---|---|---|
| AA.FF9.5 | + | - |
| AA.HE3.7 | + | + |
| AF.E9.5 | + | - |
| BA.B1.16 | + | - |
| BB.B6 | + | - |
| AA.GE7.3 | + | - |
| CD.F11.2 | + | - |
| AH.E3.11 | + | + |
| CD.G4.2 | + | + |
| AG.E7.9 | + | - |
| BD.G6 | + | + |
| BH.G8 | - | - |

### 6. cDNA Expression Libraries

We prepare cDNA libraries from rat embryonic kidney, one in the CDM8 vector which utilizes the SV40 origin for amplification, and one in a modified InVitrogen vector, pCEP4, which utilizes the EBV origin for amplification. This modified vector, CH269, has the EBNA-1 gene sequence removed. The EBNA-1 protein interacts with the EBV origin, but the gene is not needed on the vector when cells are used which stably express the EBNA protein. The library in the CDM8 vector contains 1.5 X 10⁶ clones with an average insert size of 1.18 kb, while the library in the CH269 vector contains approximately 1 X10⁶ clones with an average insert size of 1.5 kb.

### Expression Cloning of Ret Ligand RetL1

### A. Cloning of Rat Ret Ligand RetL1

### 1. Initial Attempts at Expression Cloning of Ret Ligand RetL1 -

A number of direct expression methods have been tried to clone RetL1. All of these methods are based on the concept illustrated in Figure 4B. cDNAs from a cDNA library are introduced into mammalian cells; cells that receive RetL1 can be identified using the Ret fusion proteins. Although the three approaches described below were unsuccessful, important knowledge and expertise was acquired, which was deployed in a subsequent approach that met with success.
a. Panning Method with Ret/IgG - The rat Ret/IgG fusion protein is used in an attempt to isolate RetL1 by direct expression cloning using a panning method (Aruffo and Seed, Proc. Natl. Acad. Sci. 84: 8753-8757 (1987)). A day 18 embryonic rat kidney cDNA library in CDM8 is used for the panning effort. Pools of cDNAs from this library (5,000 - 10,000 cDNAs per pool) are introduced into COS cells using the DEAE-dextran method. After 48 hours, the cells are removed from the plates with EDTA, incubated with the fusion protein, and subsequently panned on plates coated with anti-human IgG₁ antibody. DNA is recovered from cells that adhered, transformed back into E. coli, and subsequently isolated for a second round of panning. We are unable to see any cells bind after the third round of panning, and very few clones are obtained after transformation of the Hirt DNA back into E. Coli. A VCAM cDNA, used in conjunction with an anti-VCAM monoclonal antibody as a positive control, could only be diluted to a ratio of 1:100 and still be detected, indicating that our pool sizes are probably too large. Analysis of some of the clones that are obtained after the second round of panning, indicates that the clones are undergoing rearrangement and deletion.
b. Preparative FACS Method with Ret/IgG - 80,000 cDNA clones from the day 18 embryonic rat kidney library (CDM8 vector) are introduced into COS7 cells and subjected to preparative FACS using the rat Ret/IgG protein followed by a fluor-tagged secondary antibody. The top 0.5% and 0.9% of fluorescing cells are collected and the plasmid DNA is recovered by Hirt lysis. The DNA is electroporated back into E. coli: 228 clones are obtained for the 0.5% pool and 752 clones for the 0.9% pool. DNA is recovered from the bacterial clones and a second round of preparative FACS is performed. Plasmids recovered from bacterial clones at the end of the second round are analyzed and found to contain large deletions and rearrangements.
c. Colorimetric Detection Method with Ret/AP- COS cells are transfected with 400 pools of the cDNA clones (1000 clones per pool) from the day 18 rat embryonic kidney cDNA library (CDM8 vector) and stained with the Ret/AP protein and a colorimetric substrate for alkaline phosphatase. The transfected cells are inspected under a microscope for positive signals. In one experiment, five potential positives were re-analyzed, but all were negative.
   As a control for the Ret/AP protein, a VCAM/AP protein is produced by fusing the first two domains of human VCAM to the N-terminus of placental AP. (VCAM binds to the integrin VLA4, which is composed of two chains, alpha-4 and beta-1). Transient transfections of COS cells produces sufficient VCAM/AP protein for control experiments. The VCAM/AP protein is compared to VCAM/IgG directly coupled to AP, and to VCAM/IgG plus an AP coupled secondary antibody, in order to assess their ability to detect VLA4 on COS cells transfected with the alpha-4 chain cDNA (COS cells already express the beta-1 chain). The results show that while the VCAM/AP protein could detect VLA4 on transfected cells, the best detection is afforded by the VCAM/IgG protein in combination with an AP coupled secondary antibody.
d. Methodological Conclusions:
   Three major conclusions emerged from these initial cloning efforts:
   1) Methods which require that plasmid DNA be recovered for subsequent rounds (i.e. panning and preparative FACS) are not suitable when the abundance of the target cDNA is low, because of rearrangements and deletions that occur during these subsequent rounds. Based on the low expression of Ret, there is good reason to suspect that the expression of RetL1 is also low. The preferred approach is to transfect in pools and use a detection method that allows a positive pool to be identified. The original pool can then be broken down, with no need to recover the transiently expressed DNA from transfected cells.
   2) The Ret/IgG protein when coupled to a secondary reagent affords better detection capability than the Ret/AP protein.
   3) Control experiments with a VCAM/IgG control protein (and an AP coupled secondary antibody) and the alpha-4 integrin cDNA (diluted into CDM8 vector and transfected into COS cells) indicate that our detection capability is just about one in a thousand (i.e. the pool size cannot exceed 1000 clones). To attain an improved level of sensitivity, we changed from an
   SV40 origin based vector (expressed in COS cells) to an EBV origin based vector (expressed in EBNA positive cell lines). EBV origin based vectors are maintained as episomes and are not as toxic to the cell as the SV40 origin based vectors after amplification. Considerable evidence exists that genes can be expressed at higher levels in these vectors and that cDNAs can be diluted much further (i.e. up to 1 to 80,000) and still be detected.

### 2. Screening of Pools from the EBV Origin Based cDNA library

We screen pools of clones from the day 18 rat embryonic kidney cDNA library (CH269 vector with the EBV origin) with the rat Ret/IgG fusion protein. In one experiment, 256 pools, each containing 5000 clones from the library, are generated. Briefly, an aliquot of the cDNA library is titered, 5000 cells are plated (256 times), and are allowed to grow overnight. The colonies are scraped into medium: part of the culture is used to generate a glycerol stock for the pool (stored at -70) and part is used for a plasmid preparation. DNAs from the 256 pools are individually transfected into 293/EBNA cells (8 X 10⁵ on a 60 mm plate) using the lipofection method. After 48 hr. the cells are washed two times with HBHA buffer (0.5 mg/ml BSA, 0.1% NaN₃, 20 mM HEPES (pH 7.0)) and incubated with 20 ug/ml rat Ret/IgG in Tris-buffered saline plus 1 mM MgCl₂ and CaCl₂ for 60-90 min at RT. Following this incubation, the cells are washed four times with HBHA buffer and then fixed with 60% acetone/3% formaldehyde/20 mM HEPES (pH 7.0) for 30 sec. Following two washes with HBS buffer (150 mM NaCl, 20 mM HEPES (pH 7.0)), the cells are incubated with an AP-coupled secondary antibody (goat anti-human IgG Fc-gamma-specific F(ab'), (Jackson Immuno Research Laboratories; catalog # 109-056-098; 1:5000 dilution in Tris-buffered saline plus 1 mM MgCl₂ and CaCl₂) for 60 min at RT. The cells are then washed twice with HBS buffer and twice with AP substrate buffer (100 mM Tris-HCl (pH 9.5), 100 mM NaCl, 5 mM MgCl₂) containing 2X Pierce Immuno Pure^{R} Phosphatase suppressor (catalog #35002). The last wash is left for 15 min. The AP substrates NBT (0.33 mg/ml) and BCIP (0.17 mg/ml) are then added in AP substrate buffer containing the Pierce AP inhibitor and incubated with the cells for 5-20 min. The plates are then washed twice with water. The plates are then inspected under a dissecting microscope for the presence of purple stained cells.

From an analysis of the 256 pools, 17 positive pools are identified in the primary screen. DNA from each positive pool is re-transfected into 293/EBNA cells and the above procedure repeated along with some additional control experiments to confirm that the staining observed is Ret/IgG specific. 10 out of the 17 positive pools only show staining with Ret/IgG fusion protein and not with another IgG fusion protein.

### 3. Breakdown of Pool #230

As an example, one of the above-described positive pools, designated #230, is broken down into smaller subpools in order to identify the cDNA within the pool that is conferring binding to the Ret/IgG fusion protein. 600 cells from the glycerol stock for pool #230 are plated (10 times) and grown overnight. Colonies on these plates are scraped into medium: one tenth of the culture is used to generate a glycerol stock and the remaining portion is used for a DNA preparation. The ten subpools of 600 clones are designated 230-1A through 230-5A and 230-1B through 230-5B. DNAs from these subpools are transfected into 293/EBNA cells and the procedure described above for staining with the Ret/IgG fusion protein is repeated. One subpool #230-5A is positive for staining with the Ret/IgG protein.

Pool #230-5A is further broken down in order to identify the cDNA with this subpool that is conferring binding to the Ret/IgG fusion protein. Cells from the glycerol stock of pool 230-5A are plated and grown overnight. Colonies are picked into the wells of seven 96-well Bioblocks® and grown overnight. From each 96-well Bioblock, 4 pools of 20 clones and 1 pool of 16 clones are made. Thus 35 pools are generated from the seven Bioblocks® designated 230-5A-71 through 230-5A-105. DNAs are prepared from each of these pools and transfected into 293/EBNA cells and re-assayed with the Ret/IgG fusion protein as described above. Pool #230-5A-86 is positive.

Pool #230-5A-86 is broken down by going back to the Bioblock and identifying the 20 clones that were mixed together to make this pool. DNAs are made from all twenty clones and transfected individually into 293/EBNA cells and re-assayed for Ret/IgG as described above. Pool #230-5A-86-17 is found to be positive.

### 4. Characterization of Clone #230-5A-86-17

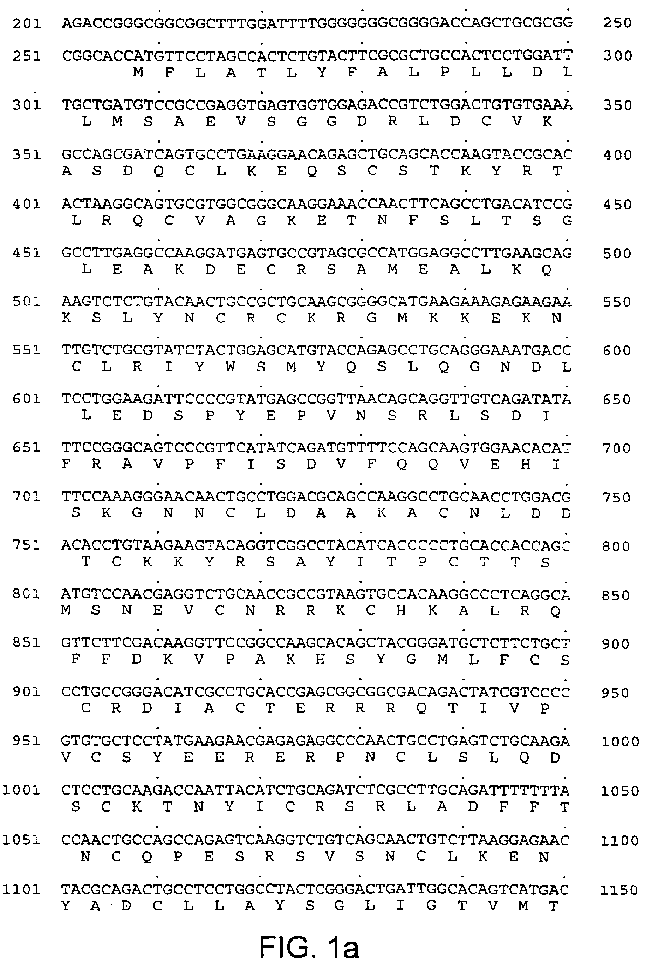

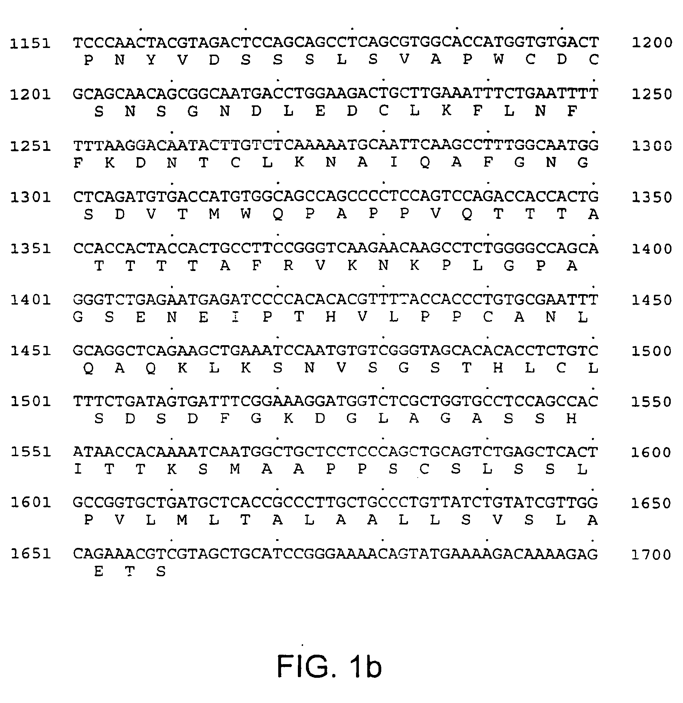

Clone #230-5A-86-17 (called retL-17 or clone 17 and deposited as ATCC 98047) is further analyzed by DNA sequencing. The entire nucleotide sequence of the insert of this clone is SEQ ID NO:1 (rat retL1 cDNA), and part of the nucleotide sequence is shown in Figure 1. Within this nucleotide sequence, we find a reading frame coding for a protein of 468 amino acids (rat RetL1). The predicted protein has a signal sequence with a predicted cleavage after amino acid 24 (Von Heijne et al., Nucl. Acid Res. 14:14683 (1986)). The hydrophobic C-terminus indicates that the protein may be linked to the cell via a phosphatidylinositol glycan linkage. There are three predicted N-linked glycosylation sites. These properties are consistent with those expected for a ligand for Ret.

We can express soluble forms of the rat RetL1 protein by truncating the gene prior to the hydrophobic C terminus. For example, this could be done by truncating after Lysine 435 (rat RetL1). Truncation upstream of this amino acid should also result in the expression of a soluble form of the rat RetL1 protein. The soluble rat RetL1 protein can be expressed by itself or as a part of a fusion with human immunoglobulin, a histidine tag, or a small epitope that is recognized by an antibody.

### B. Cloning of Human Ret Ligand RetL1

A human embryonic kidney cDNA library in the vector lambda gt10 is purchased from Clontech (catalog #HL5004A). One million plaque forming units from the phage stock are plated on 10 Nunc™ plates. Duplicate plaque lifts are made on Schleicher and Schuell Optitran™ filters.

A probe is generated by digesting plasmid rat RetL1 with the restriction enzyme PvuII, followed by agarose gel isolation of a 1.34 kb fragment which corresponds to nt 242-1582 of the rat RetL nucleotide sequence (rat retL1 cDNA). This coding region probe is P³² labeled by random priming (Feinberg and Vogelstein, Anal. Biochem. 137:266-267. 1984). The filters are hybridized overnight in 300 ml plaque screen PSB buffer (50 mM Tris pH 7.5, 1 M NaCl, 0.1% sodium pyrophosphate, 0.2% PVP, and 0.2% Ficoll) containing 10% dextran sulphate, 100 ug/ml tRNA, and 6.7 X 10⁷ CPM of the rat probe, at 55C. They are washed twice with plaque screen buffer and twice with 2XSSC/1%SDS at 55C and exposed to film at -70C with an intensifying screen.

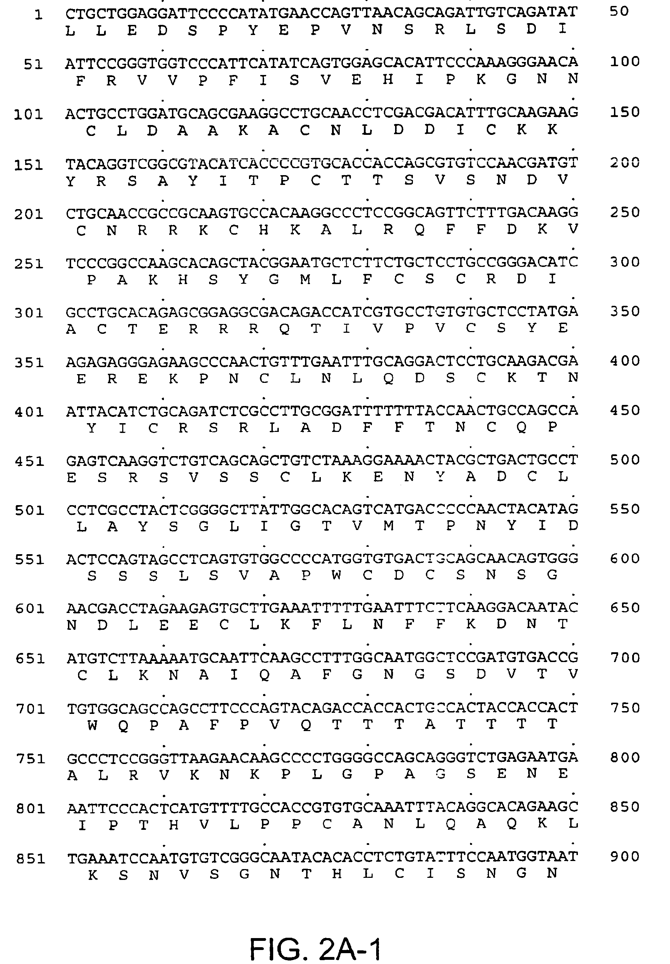

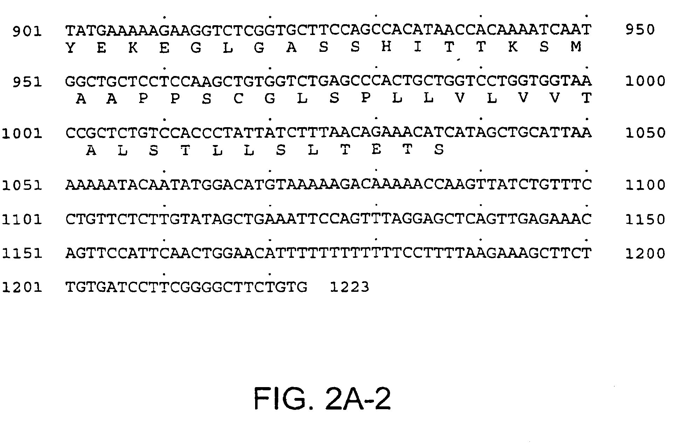

Duplicate positives are cored from the master plates into SM (100 mM NaCl, 10 mM SO₄, 50 mM Tris pH 7.5) plus gelatin. 24 of these positives are plaque purified. Lambda miniprep DNA from the purified candidate plaques is digested with Not1, electrophoresed on 1% agarose gel and Southern blotted. The Southern blot is hybridized with the rat rat RetL1 coding region probe. Clone HRL20 has the longest insert (4.4 kb) which hybridizes intensely to the rat probe. DNA sequence (partial human retL1 cDNA; SEQ ID NO: 8; Figure 2A) and deduced peptide sequence (partial human RetL1; SEQ ID NO: 9; Figure 2A) have been obtained from this clone , confirming that it is the human homologue. This clone encodes most of the coding region, including the 3' end of the coding region.

To obtain the 5' end of the human cDNA, a human fetal kidney Marathon-Ready™ cDNA kit is purchased from Clontech (catalog #7423-1). Antisense oligonucleotides Kid-155, corresponding to the complement of nucleotides 62-81 of SEQ ID NO: 8 (partial human retL1 cDNA) and Kid-154, corresponding to the complement of nucleotides 17-43 of SEQ ID NO: 8 (partial human retL1 cDNA) are synthesized. PCR is performed using the Advantage™ cDNA PCR kit (Clontech catalog #8417-1) combined with Marathon™ cDNA reagents and the oligonucleotides Kid-155 or Kid-154. The first PCR reaction is set up as follows: 35.5 ul H₂O; 5.0 ul 10X KlenTaq Buffer: 1.0 ul 10mM dNTP mix, 1.0 ul 50X Advantage™ KlenTaq Polymerase mix. These reagents are combined and mixed. Then 5.0 ul Marathon-Ready™ Fetal Kidney cDNA; 1.0 ul 10uM AP1 primer and 1.5 ul 6.4uM Kid-155 are added (final volume = 50 ul). PCR is carried out in a Perkin-Elmer Cetus DNA Thermal Cycler 480 with the following cycle conditions: 1 cycle of 94C for 1 min; 30 cycles of 94C for 30 sec, 55C for 30 sec. 68C for 4 min. A nested PCR is performed using the product of the first PCR reaction. First, 5 ul of PCR product #1 is diluted 50 fold with TE (final volume 250 ul). The nested PCR reaction contains 35.5 ul H₂O; 5.0 ul 10X KlenTaq Buffer; 1.0 ul 10mM dNTP mix; 1.0 ul 50X Advantage™ KlenTaq Polymerase mix. These reagents are mixed as above. 5.0 ul diluted PCR product #1: 1.0 ul 10uM AP2 primer and 1.5 ul 6.9uM Kid-154 are then added. Cycle conditions are the same as above. The resultant product of approximately 700 bp is purified on a 1% low-melt agarose gel and phenol extracted. The purified DNA is cloned into the EcoR5 site of pZErO™ (Invitrogen catalog #K2510-01). Sequence information is obtained from multiple isolates, including clones called HRL7G6 and HRL7G8.

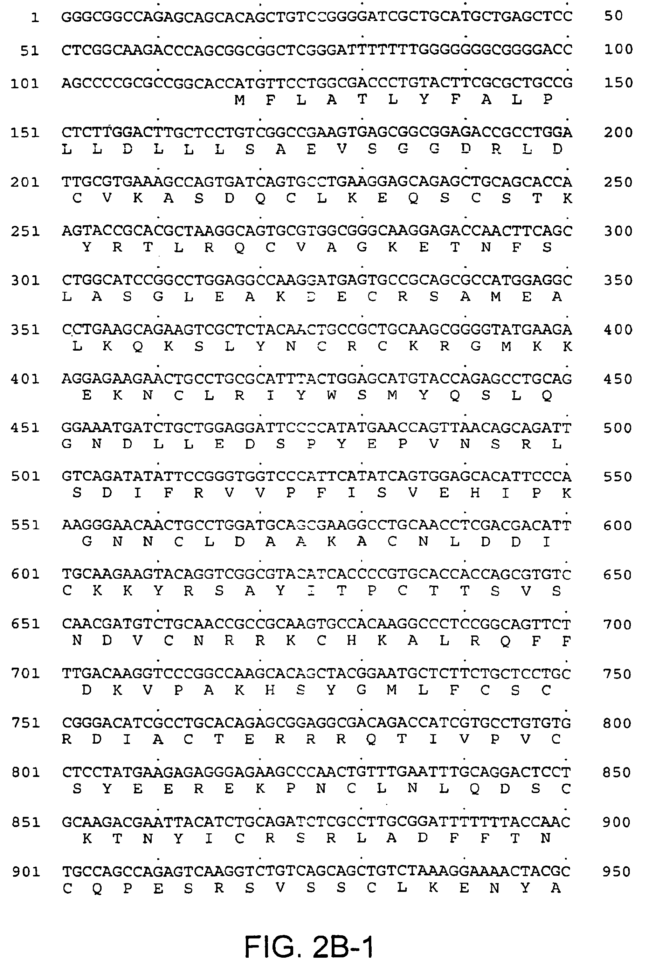

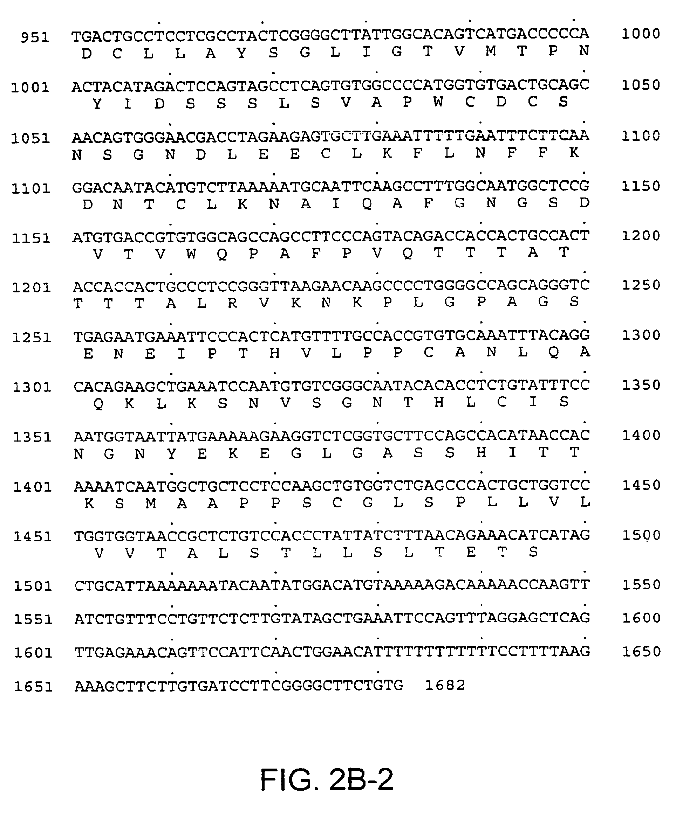

The sequence obtained from clone HRL7G8 is found to overlap with the sequence of clone HRL20 (partial human retL1 cDNA) and is used to generate a full-length sequence of human RetL1 (full-length human retL1 cDNA), also shown in Figure 2B. The nucleotide sequence obtained from clone HRL7G8 represents nucleotides 1 to 502 of full-length human retL1 cDNA; the nucleotide sequence from clone HRL20 represents nucleotides 460 to 1682 of full-length human retL1 cDNA. The sequence from clone HRL7G8 is confirmed by sequencing another cDNA clone (GJ102) isolated from the human embryonic kidney lambda gt10 cDNA library described above, using a probe derived from clone HRL7G6. Nucleotides 118 to 1497 comprise the protein reading frame of full-length human retL1 cDNA.

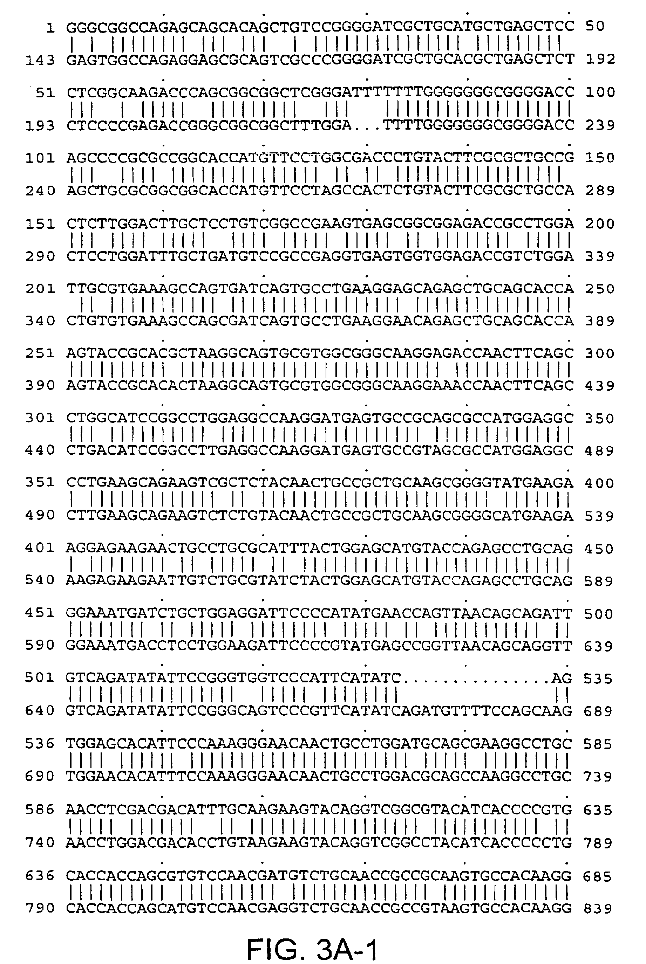

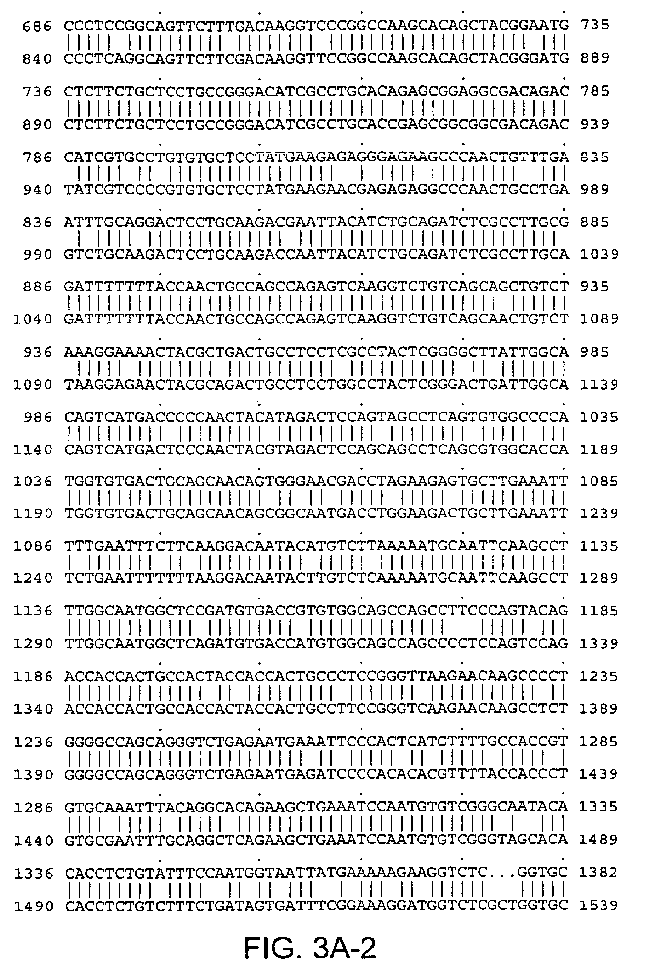

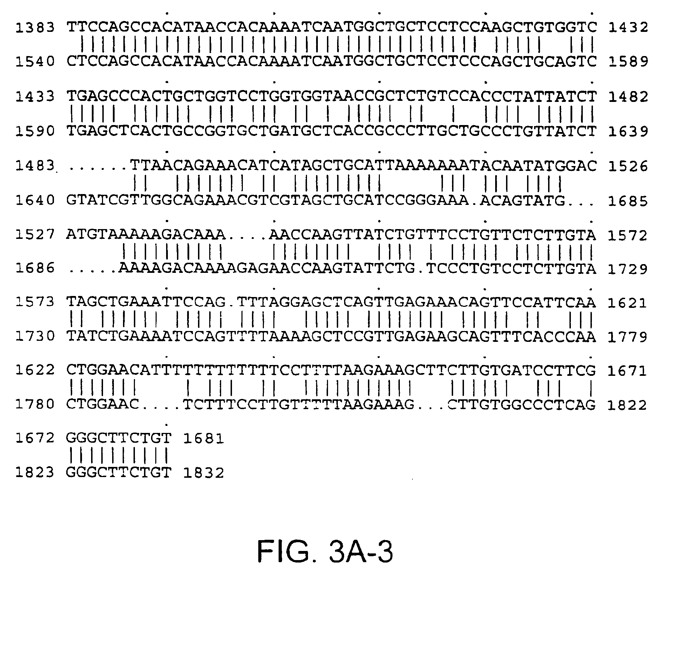

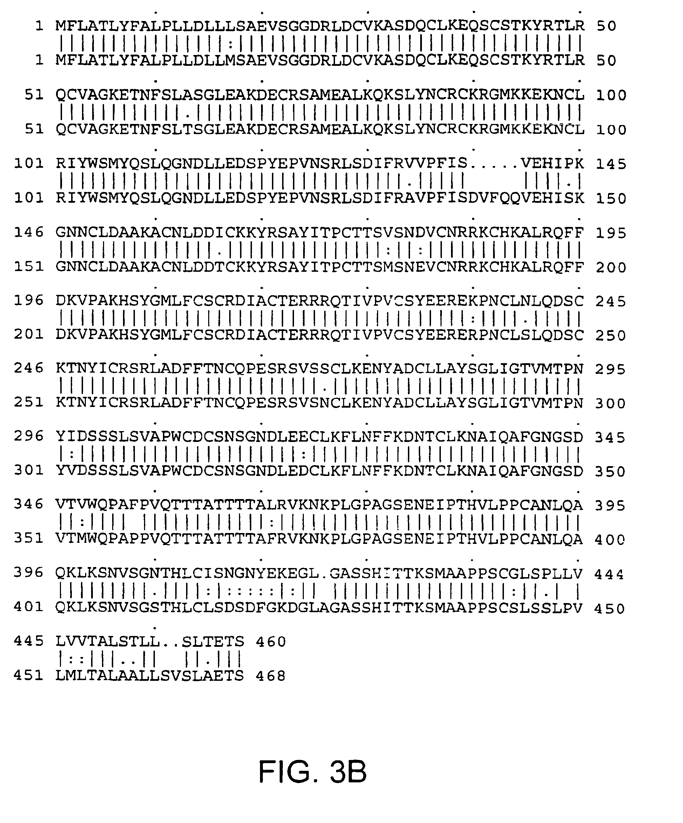

The complete amino acid sequence of human RetL1 is also shown in Figure 2B. As shown by the BESTFIT analysis depicted in Figure 3A, the human retL1 cDNA is 88.2% identical to the rat retL1 cDNA. The peptide comparison (Figure 3B) shows the human putative peptide sequence to be 93.3% identical, and 97.2% similar, to that of the rat.

### Cloning of Ret Ligand RetL2

### A. Cloning of Human RetL2

The peptide sequence of rat RetL1 (rat RetL1) is used to search the GenBank database with the program BLAST in order to identify related proteins (i.e. isologs). BLAST. or Basic Local Alignment Search Tool, uses the method of Altschul et al. (J. Mol. Biol. 215: 403-410, 1990) to search for similarities between a query sequence and all the sequences in the sequence database. The query sequence and the database to be searched can be either peptide or nucleotide in any combination. When the rat RetL1 peptide sequence is queried against the Expressed Sequence Tag (EST) nucleotide database, two significant matches are obtained. One is with GenBank Accession # R02249, a 229 bp EST from a combined human fetal liver and spleen cDNA library, and the other is with Genbank Accession # H12981, a 521 bp EST from a human infant brain cDNA library. The two ESTs share 99% identity in a region of overlap indicating that they are from the same cDNA. Oligonucleotides are generated from the H12981 EST: KID-228 (GAA TGA CAA CTG CAA GAA GCT GCG CTC CTC; corresponding to nucleotides 38-67 and also to nucleotides 534-563 of SEQ ID N0:12), and antisense oligonucleotide KID-229 (GTG TAC TCG CTG GGC ACC CG: corresponding to the complement of nucleotides 156-175 and also to the complement of nucleotides 652-671 of SEQ ID NO:12).

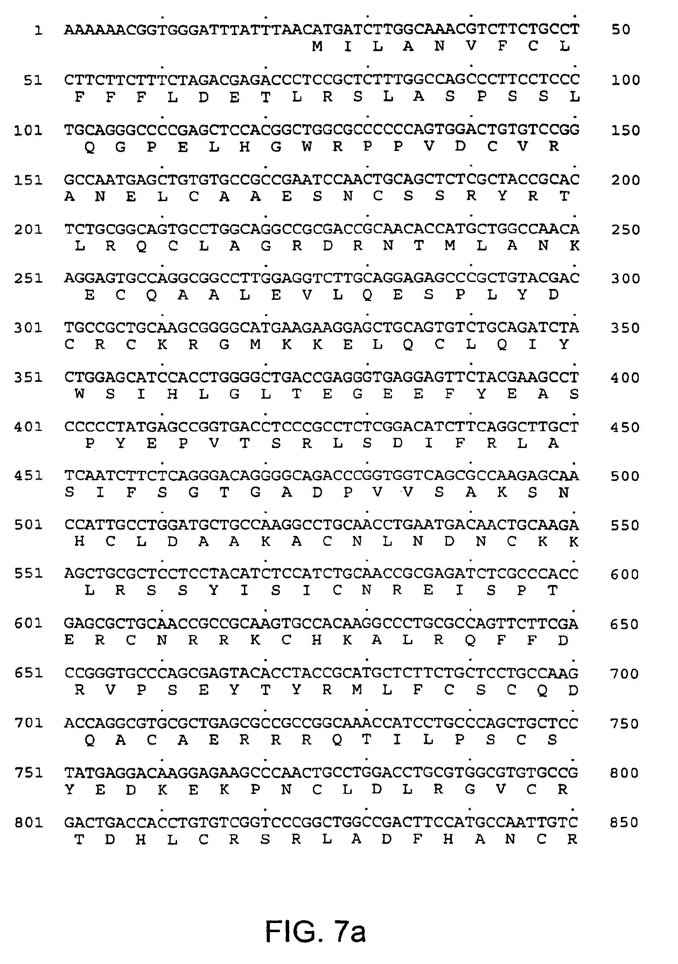

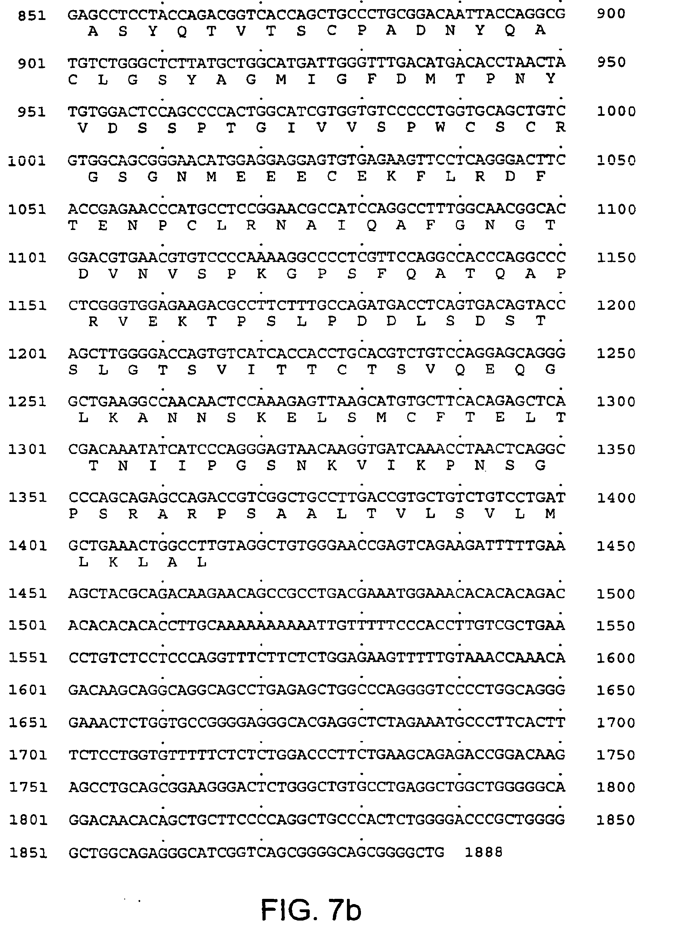

1X10⁶ plaque forming units from a Clontech Human Fetal Liver 5' -Stretch Plus lambda GT10 cDNA library (cat# HL5003a) are screened in duplicate on OPTITRAN™ filters. The filters are hybridized with ³²P-labeled oligonucleotides KID-228 and KID-229 in 400 mls plaque screen buffer (50mM Tris pH 7.5, 1M NaCl, 0.1% sodium pyrophosphate, 0.2% Polyvinylpryrolidine and 0.2% Ficoll) containing 10% Dextran sulfate and 100ug/ml tRNA and 80 pmole each ³²P-labeled oligonucleotide at 65C overnight. They are washed twice with 2X SSC/1% SDS and twice with 1X SSC/1% SDS and exposed to film. 11 duplicate positives are purified. DNA from each of these clones is analyzed by restriction enzyme digest followed by agarose gel electrophoresis and Southern blotting. The filters are hybridized to KID-228 and KID-229 to confirm that the inserts hybridize to the probe. The insert of clone DSW240 is completely sequenced (human retL2 cDNA, SEQ ID NO:12) and is shown in Figure 7.

Nucleotides 25-1416 comprise the protein reading frame of human retL2 cDNA, which encodes a protein of 464 amino acids (human RetL2; SEQ ID NO:13), and is shown in Figure 7. As shown by the BESTFIT analysis depicted in Figure 8, the human RetL2 protein is 49.1% identical and 63.7% similar to the human RetL1 protein. It shares in common with human RetL1 a hydrophobic N-terminus indicative of a signal sequence and a hydrophobic C-terminus indicative of a phosphatidylinositol glycan linkage motif. In addition, 30 cysteines out of the 31 that are present in each protein are conserved.

### B. Demonstration that RetL2 is a Ligand for Ret

We demonstrate that RetL2 is a ligand for Ret by transfecting 293/EBNA cells with an expression plasmid that contains the insert of clone DSW240 and by showing that the cells can bind a soluble Ret/IgG fusion protein.

The insert of DSW240 is removed using Not1 and cloned into the expression vector CH269 which contains an EBV origin and allows for high expression in EBNA positive cell lines. Restriction digests are performed to identify clones that have the correct orientation. Plasmid DNA is prepared from a clone having the correct orientation.

Plasmid DNAs (the retL2 expression plasmid, a retL1 expression plasmid for a positive control, and an expression plasmid containing an unrelated protein for a negative control) are transfected into 293/EBNA cells (8 X 10⁵ on a 60 mm plate) using the lipofection method. After 48 hr, the cells are washed two times with HBHA buffer (0.5 mg/ml BSA, 0.1% NaN₃, 20 mM HEPES (pH 7.0)) and incubated with 20 ug/ml rat Ret/IgG in Tris-buffered saline plus 1 mM MgCl₂ and CaCl₂ for 60-90 min at room temperature. Following this incubation, the cells are washed four times with HBHA buffer and then fixed with 60% acetone/3% formaldehyde/20 mM HEPES (pH 7.0) for 30 sec. Following two washes with HBS buffer (150 mM NaCl, 20 mM HEPES (pH 7.0)), the cells are incubated with an AP-coupled secondary antibody (goat anti-human IgG Fc-gamma-specific F(ab')₂ (Jackson Immuno Research Laboratories; catalog # 109-056-098; 1:5000 dilution in Tris-buffered saline plus 1 mM MgCl₂ and CaCl₂) for 60 min at RT. The cells are then washed twice with HBS buffer and twice with AP substrate buffer (100 mM Tris-HCl (pH 9.5), 100 mM NaCl, 5 mM MgCl₂) containing 2X Pierce Immuno Pure® Phosphatase suppressor (catalog #35002). The last wash is left for 15 min. The AP substrates NBT (0.33 mg/ml) and BCIP (0.17 mg/ml) are then added in AP substrate buffer containing the Pierce AP inhibitor and incubated with the cells for 5-20 min. The plates are then washed twice with water. The plates are then inspected under a dissecting microscope for the presence of purple stained cells. The presence of purple stained cells indicates that the Ret/fusion protein has bound to the cells and that the RetL2 protein is a ligand for Ret. Purple stained cells are also observed after trasfection with the retL1 expression vector but not with the negative control vector.

### Cloning of Ret Ligand RetL3

### A. Murine RetL3

A search of the EST data base with rat RetL1 amino acid sequence discloses two murine ESTs with homology to Ret ligands. These ESTs are AA049894, and AA050083 (which is partial murine retL3 cDNA, SEQ ID NO: 14). Plasmids encoding these ESTs are obtained from Genome Systems Inc. (Catalog# 475791 and #475497) as bacterial stabs. Plasmid DNA is prepared from single colonies obtained by streaking the stabs onto LB Amp plates. The inserts from these plasmids are sequenced in their entirety. Comparison of the two sequences demonstrates that AA049894, which has a 1.4 kb insert, is contained within AA050083, which has a 1.9 kb insert. Translation of the DNA sequence from AA050083 indicates there is a continuous open reading frame from NT205 to NT1242 (partial murine RetL3; SEQ ID NO:15). This ORF had 37.5% identity to that of rat retL1 and 40.2% identity to rat retL2. However, the open reading frame does not encode a Met or a signal sequence at the 5' end. We examine the 5' ORFs upstream of this region and find a Met in the context of a Kozak consensus sequence for translation iniation and a potential signal sequence for surface expression/secretion. This ORF is out of frame with the downstream ORF indicating that EST AA050083 contains a potential mutation, such as an insertion, deletion, intron or cloning artifact, at its 5' end.

In order to obtain the correct 5' end, we employ Marathon RACE. Mouse 11-day embryo Marathon-Ready™ cDNA (cat.#7458-1) and an Advantage™ Kit (cat.# 8417-1) is purchased from Clontech. Antisense oligonucleotides, Kid-366, corresponding to the complement of nucleotides 847-866 of SEQ ID NO:14 and Kid-365, corresponding to the complement of nucleotides 589-615 of SEQ ID NO:14 are synthesized. PCR is performed using and an Advantage™ cDNA PCR kit (Clontech cat.#8417-1) combined with Marathon™ cDNA reagents and oligonucleotide Kid-366. The first PCR reaction is set up as follows: 35.3 ul H₂O; 5.0 ul 10X KlenTaq Buffer; 1.0ul 10mM dNTP mix; 1.0 ul 50X Advantage™ KlenTaq Polymerase mix. These reagents are combined and mixed. Then 5.0 ul Marathon-Ready™ mouse 11-day embryo cDNA; 1.0 ul 10uM AP1 primer and 1.7ul 5.88uM Kid-366 are added (final volume = 50ul). PCR is carried out in a Perkin-Elmer Cetus DNA Thermal Cycler 480 with the following cycle conditions: 1 cycle of 94C for 1 min; 5 cycles of 94C for 30 sec, 72C for 4 min; 5 cycles of 94C for 30 sec, 70C for 4 min; 25 cycles 94C for 30 sec, 68C for 4 min. A nested PCR is performed using the product of the first PCR reaction. First, 5 ul of PCR product#1 is diluted 50 fold with TE(Final volume 250 ul). The nested PCR reaction contains 35.5 ul H₂O 5.0 ul 10X KlenTaq Buffer; 1.0 ul 10mM dNTP mix; 1.0 ul 50X Advantage™ KlenTaq Polymerase mix. These reagents are mixed as above. 5.0 ul diluted PCR product #1; 1.0 ul 10uM AP2 primer and 3.6 ul 2.8uM Kid-365 are then added. Cycle conditions are the same as above. The resultant product of approximately 665 bp is purified on a 1% low-melt agarose gel and Qiaex II (Qiagen cat#20021) extracted. The purified DNA is cloned into pNoTA/T7™ using PRIME PCR CLONER™ cloning system (5 Prime-> 3 Prime cat.#1-725029). Sequence information is obtained from multiple isolates, including clones called DSW252 and DSW253.

The sequence of DSW252 is found to overlap with SEQ ID NO: 14 except that an additional T is present between NT 252 and NT 253 of the SEQ ID NO:14 sequence. This T is also present in the other isolates DSW251 and DSW253. Insertion of this additional base corrects the ORF such that a single 1191 bp ORF (counting from the first Met) encoding 397 amino acids is obtained. This ORF encodes a Met in the context of a canonical translation initiation consensus sequence (Kozak) and includes a signal sequence for surface expression/secretion.

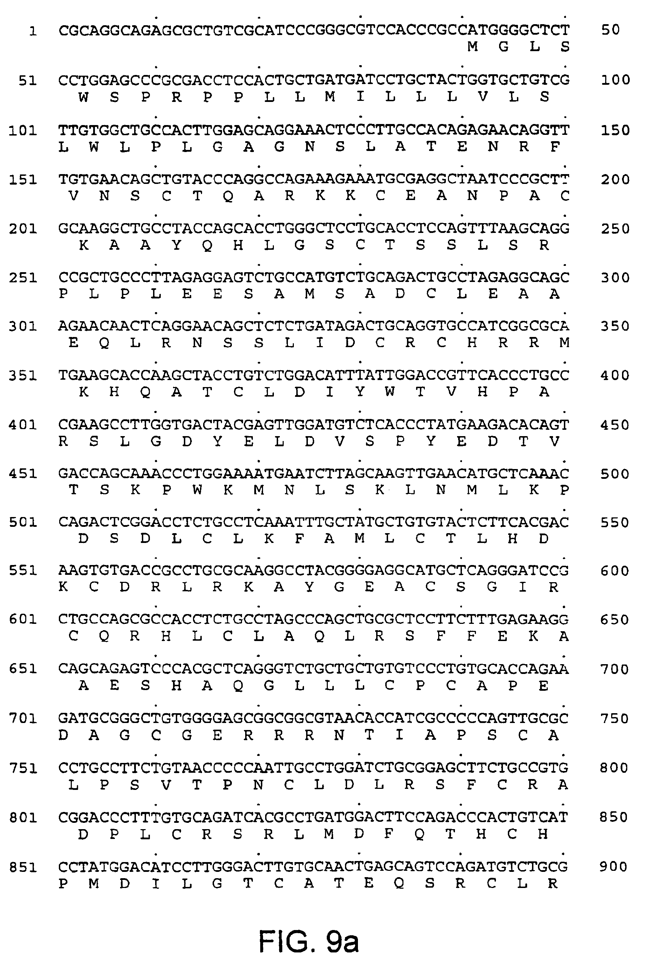

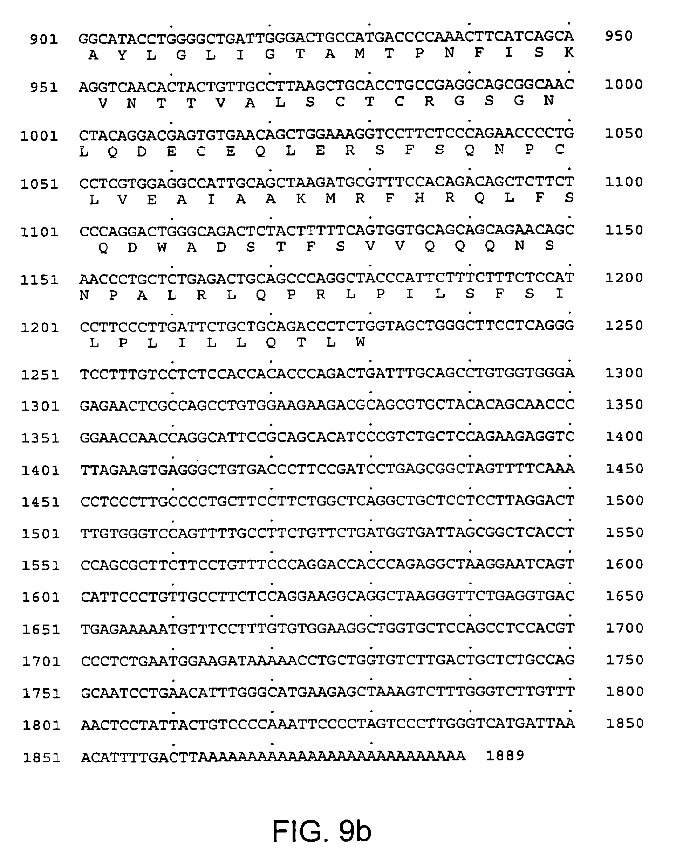

To obtain a full-length murine clone which is capable of being expressed, a 630 bp Not1-BamH1 fragment of DSW252 and a 1308 bp BamH1-Not1 fragment of AA050083 is purified and ligated to Not I digested expression vector CH269. The ligation is transformed into E.coli XL1-Blue (Strategene cat. #200236). Qiawell Ultra minipreps are performed on resultant transformants. These are analysed by restriction digest and gel electrophoresis for correct size and for orientation. This construct is called DSW254. The insert of DSW254 is sequenced in its entirety (murine retL3; SEQ ID NO:16) and the ORF is confirmed as encoding a protein of 397 amino acids (murine RetL3; SEQ ID NO:17). These sequences are also shown in Figure 9. The C-terminus of RetL3 is hydrophobic and indicative of a phosphatidylinositol glycan linkage motif.

### B. Human RetL3

In order to find a candidate tissue source for cloning human RetL3, we utilize northern blots of mouse tissues to determine the expression pattern of murine RetL3. Of the tissues surveyed, expression of RetL3 is highest in heart tissue. A human adult heart cDNA library in the vector lambda gt10 is purchased from Clontech (catalog# HL3026a). One million plaque forming units from the phage stock are plated on 10 Nunc plates. Duplicate plaque lifts are made on Schleicher and Schuell Optitran™ filters.

A probe is generated by PCR with primers Kid-366 and Kid-367. corresponding to nucleotides 397-420 of the AA050083 sequence. PCR reaction is set up as follows: 10 ul 10X PFU Buffer. 2.0 ul 10mM dNTP mix, 72.1 ul H₂O, 3.1 ul 13.2 uM Kid-367, 6.8 ul 5.88 uM Kid-366, 5.0 ul 0.1 ug/ ul AA050083 DNA and 2.0 ul 2.5 Units/ul PFU (Stategene catalog # 600154) are mixed. PCR is carried out in Perkin-Elmer Cetus DNA Thermal Cycler 480 with the following conditions: 25 cycles of 94 C for 1 min, 53C for 1 min.. 72C for 4 min. The product is purified by extraction with phenol, chloroform, isoamyl alcohol 50:49:1 followed by low-melt agarose gel electrophoresis and QiaexII purification of the excised fragment. This coding region probe is P³² labeled by random priming (Feinberg and Vogelstein). The filters are hybridized overnight in 200 ml Plaque screen buffer () containing 10% dextran sulphate, 100 ug/ml tRNA and 1.8 X 10⁸ CPM of the mouse probe at 65C. They are washed twice with plaque screen buffer, twice with 2XSSC/1% SDS, twice with 1XSSC/1%SDS at 65C and exposed to film at - 70C with an intensifying screen. Duplicate positives are plaque purified. Lambda miniprep DNA from the purified candidate plaques is digested with EcoR1, electrophoresed on a 1% agarose gel and Southern blotted. The Southern blot is hybridized with the mouse probe. Clone GJ128 , which has a 1.3 kb insert, hybridizes intensly to the mouse coding region probe. DNA sequence (partial human retL3 cDNA; SEQ ID NO:18) and deduced peptide sequence (partial human RetL3; SEQ ID NO:19) are obtained from this clone, confirming that it is the human homologue. This clone encodes most of the coding region, including the 3' end of the coding region.

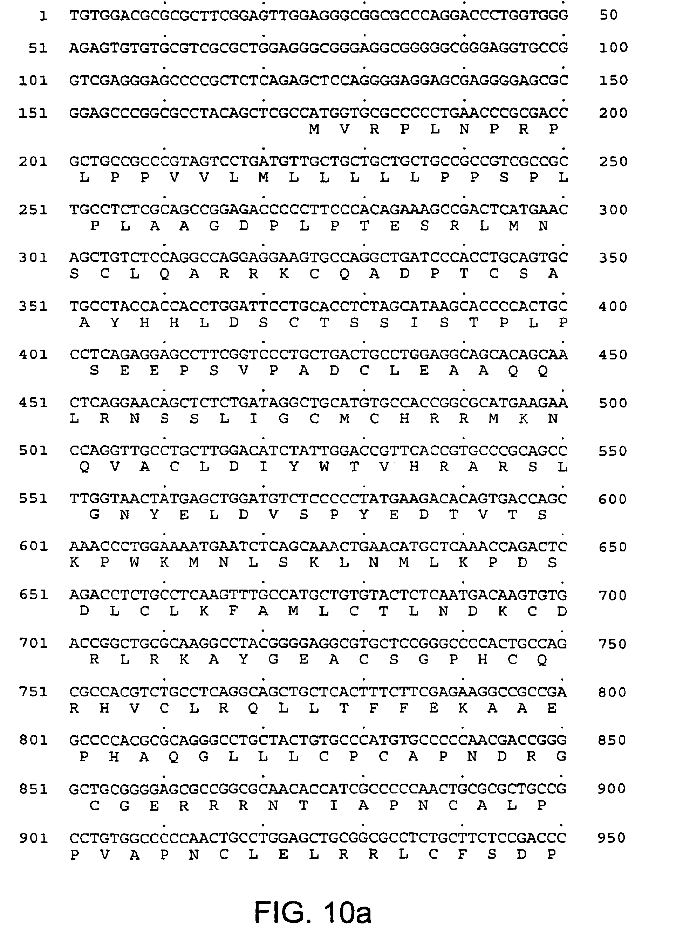

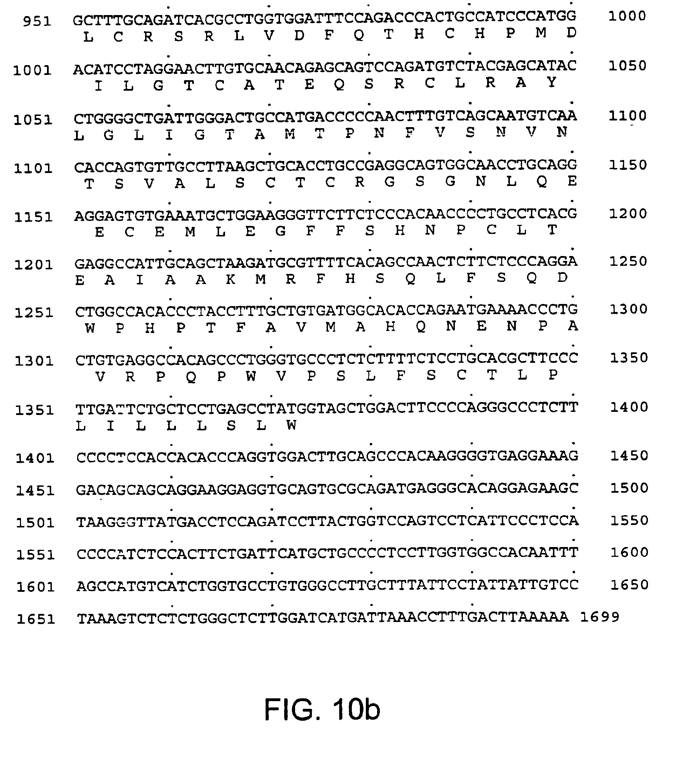

The 1.3 kb insert from GJ128 is purified, labeled with P³² and used to screen the Clontech human adult heart library in order to obtain a clone with the 5' end. No clones containing the 5' end are obtained in a screen of 2 X 10⁶ plaques from this library. Northern analysis of human adult tissue mRNA blots (Clontech catalog # 7760-1, 7759-1 and 7767-1) hybridized with the same probe, using protocols supplied by manufacturer, indicates that human RetL3 is expressed in human adult spinal cord, stomach, heart, pancreas, small intestine, colon, prostate and testis. A Clontech human adult spinal cord cDNA library (catalog # 5001a) is screened with GJ128 insert. 3 independant clones are purified and the longest, GJ135 is sequenced. The sequence of the insert of GJ135 overlaps with the insert of GJ128. allowing the generation of a composite sequence of the full-length human retL3 cDNA (SEQ ID NO:20) and the determination of the full-length human RetL3 (SEQ ID NO:21). These sequences are also shown in Figure 10. Human RetL3 is 34.3% and 34.9% identical to human RetL1 and human RetL2, respectively. It has 76.8% identity with murine RetL3.

### THERAPEUTIC USES OF THE COMPOUNDS OF THE INVENTION

Native and variant RetL's, anti-RetL antibodies, anti-Ret antibodies, and fusion proteins of Ret and of RetL's may have therapeutic utility in situations where it is desirable to block or to activate the Ret signaling pathway, to stimulate renal and/or neuronal cell growth or survival in disease situations where these cells are lost or damaged, or to suppress growth of or to kill undesirable cells such as tumor cells that express Ret or a RetL.

In general, compounds of the invention that bind to Ret, inducing dimerization and/or autophosphorylation of Ret, are useful for stimulating growth of or limiting damage to Ret-expressing tissues. The compounds of the invention are useful for stimulating renal tissue growth and/or survival, supporting renal function, and in minimizing damage to renal tissue after various insults. Particular conditions which may be beneficially treated with the compounds of the invention include acute renal failure, acute nephritis, chronic renal failure, nephrotic syndrome, renal tubule defects, kidney transplants, toxic injury, hypoxic injury, and trauma. Renal tubule defects include those of either hereditary or acquired nature, such as polycystic renal disease, medullary cystic disease, and medullary sponge kidney. This list is not limited, and may include many other renal disorders (see, e.g., Harrison's Principles of Internal Medicine, 13th ed., 1994).

In other applications, the genes and proteins of the invention may be used to treat conditions where neural growth and regeneration is desirable. This would include any conditions involving disorders of neural degeneration, such as Alzheimer's disease, Parkinson's, Huntington's, Tourette's, amyotrophic lateral sclerosis, as well as motor neuron disease, demyelinating diseases such as multiple sclerosis, bacterial diseases such as meningitis, abscess, or empyema, viral diseases such as HIV-associated myelopathy, prion diseases including Creutzfeldt-Jakob disease. Also included are disorders of damage to neural tissue, whether caused by neoplastic impingement, trauma, or cerebrovascular events such as hemorrhage or emboli. Diseases of the cranial nerves and of the spinal cord, including disorders involving traumatic, inflammatory, congenital or vascular etiologies, are specifically included, as are disorders affecting the autonomic nervous system. Also included are developmental neural disorders such as mental retardation, autism, fetal alcohol syndrome, Down's syndrome, and cerebral palsy. The compounds of the invention may also be used to treat syndromes involving the peripheral nervous system. These disorders include those caused by any of the factors previously listed, and specifically include Lyme disease. HIV-associated neuropathies, polymyositis, muscular dystrophy, and myasthenia gravis.

Anti-RetL antibodies and Ret fusion proteins of the invention, which specifically bind to the protein of murine RetL3 or human RetL3, or fragments of these proteins, are useful in several methods. The compounds may be used therapeutically to inhibit or block Ret receptor signaling, such as for blocking growth of tumors which depend on activation of Ret signaling for growth. These agents may also be fused to detectable markers, such as fluoroscopically or radiographically opaque substances, and administered to a subject to allow imaging of tissues which express a RetL. The agents may also be bound to substances, such as horseradish peroxidase, which can be used as immunocytochemical stains to allow visualization of areas of RetL-positive cells on histological sections. A specific antibody could be used alone in this manner, and sites where it is bound can be visualized in a sandwich assay using an anti-immunoglobulin antibody which is itself bound to a detectable marker. Specific antibodies to any RetL are also useful in immunoassays to quantify the substance for which a given antibody has specificity. Specific antibodies to a RetL may also be bound to solid supports, such as beads or dishes, and used to remove the ligand from a solution, either for use in purifying the protein or in clearing it from the solution. Each of these techniques is routine to those of skill in the immunological arts.

Other methods of the invention include modulating Ret-RetL signaling by contacting Ret with an anti-Ret monoclonal antibody. The effect of such a mAb-Ret contact can be to either block or to stimulate activation of the Ret signaling pathway, depending on the characteristics of the interaction of each particular mAb with Ret. Certain mAbs interact with Ret as agonists, with the agonist mAb-Ret binding triggering the dimerization and autophosphorylation of Ret. Other mAbs act as Ret antagonists. The interaction of Ret with an antagonist mAb prevents Ret signaling activation by other RetL's, or by complexes comprising RetL's, which would otherwise activate the Ret signaling pathway.

A RetL and/or antibodies to Ret or to a Ret fusion protein can be used to allow imaging of tissues which express Ret, or in the immunohistological or preparative methods described above for antibodies to a RetL.

Fusion proteins encompassing a RetL and/or anti-Ret antibodies can be used to specifically target medical therapies against cancers and tumors which express Ret. Such tumors might include the several different tumor phenotypes which have been associated with mutations in Ret (N. Engl. J. Med. 335:943-951, 1996; Nature 367: 319-320. 1996; Trends Gen. 12:138-144, 1996). Therapeutic interventions against neoplasias which express a RetL utilize fusion proteins which incorporate Ret and/or an anti-RetL antibody. The anti-Ret antibody or anti-RetL antibody may be effective by itself through antibody-dependent and complement-dependent cytolysis mediated by the Fc domain. Such hybrid ligands and antibodies can be made more effective as cancer therapeutics by using them as delivery vehicles for antineoplastic drugs, toxins and cytocidal radionuclides, such as yttrium 90. Cytotoxic effector cells may be targeted to tumor cells using heteroconjugate antibodies, where an antibody specific for either Ret or for a RetL expressed by a tumor is covalently coupled to an antibody directed against a surface protein on cytotoxic effector cells, such as NK cells or CTLs.

One example of an anti-Ret antibody or RetL therapy is to conjugate the toxic A chain of ricin or a modified full-length form of ricin (which can no longer bind cells) to a RetL or to an antibody directed against the Ret polypeptide expressed on the surface of malignant cells. In another embodiment, a toxin is conjugated to Ret or to an anti-RetL antibody to selectively target and kill RetL-positive cells, such as a tumor expressing a RetL. Such an approach has proved successful with blocked ricin conjugated to a monoclonal antibody against the CD19 antigen expressed on most neoplastic cells (Grossbard et al., Blood 79:576,1992). Other toxins are equally useful, as known to those of skill in the art. Such toxins include, but are not limited to, pseudomonas exotoxin, diphtheria toxin, and saporin. This approach should prove even more successful using a RetL or anti-Ret antibody, as contrasted to the known anti-CD19 antigen approach, because Ret is expressed in a very limited number of tissues.

The above approaches, using fusions of ricin or other toxins, are equally applicable to toxic conjugates of RetL or of an anti-Ret antibody; these are useful for selectively targeting and killing Ret-positive cells, such as tumor cells expressing Ret.

Another approach to such medical therapies is to use radioisotope labeled RetL or anti-Ret antibodies. Such radiolabeled compounds will preferentially target radioactivity to tumor sites in cells expressing Ret, sparing normal tissues. Depending on the radioisotope employed, the radiation emitted from a radiolabeled antibody bound to a tumor cell may also kill nearby malignant tumor cells that do not express Ret. A variety of radionuclides may be used. Isotopes that emit β particles (for example, ¹³¹I) have been successful when employed with monoclonal antibodies against CD20 present on B-cell lymphomas (Kaminski et al., N. Engl. J. Med. 329: 459 (1993); Press et al., N. Engl. J. Med. 329: 1219 (1993). Radionuclides emitting β particles generate radioactive emissions that are tumoricidal over distances spanning several cell diameters, permitting the eradication of antigen negative cells and diminishing the consequences of nonhomogenous deposition of antibody or ligand in tumors.

Radionuclides emitting a particles may also be employed. The low dose rate irradiation generated by radionuclide labeled RetL or anti-Ret antibodies may be more therapeutically effective than the instantaneous irradiation delivered externally in conventional radiation therapy. Low dose rate irradiation can induce apoptosis (programmed cell death) in certain cell lines (Macklis et al., Radiat. Res. 130: 220 (1992); Maklis et al., Radiopharm. 5: 339 (1992).

The compounds of the invention are administered in therapeutically-effective amounts, which means an amount of a compound which produces a medically desirable result or exerts an influence on the particular condition being treated.

The term "subject" used herein is taken to mean any mammal to which Ret ligand or gene may be administered. Subjects specifically intended for treatment with the method of the invention include humans, as well as nonhuman primates, sheep, horses, cattle, goats, pigs, dogs, cats, rabbits, guinea pigs, hamsters, gerbils, rats and mice, as well as the organs, tumors, and cells derived or originating from these hosts.

### Use of Compounds of the Invention in Gene Therapy

The RetL genes of the invention are introduced into damaged tissue to stimulate production of a RetL by the transfected cells, to promote cell growth and/or survival of cells that express Ret.

In a specific embodiment of a gene therapy use a RetL gene may be introduced into a renal or neural target tissue of choice. A RetL would then be stably expressed and stimulate Ret receptor-positive cells to grow, divide, differentiate, and/or potentiate cell survival. Furthermore, RetL genes may be introduced into a target cell using a variety of well-known methods that use either viral or non-viral based strategies.

Non-viral methods include electroporation, membrane fusion with liposomes, high velocity bombardment with DNA-coated microprojectiles, incubation with calcium-phosphate-DNA precipitate, DEAE-dextran mediated transfection, and direct micro-injection into single cells. For instance, a RetL gene may be introduced into a cell by calcium phosphate coprecipitation (Pillicer et al., Science, 209: 1414-1422 (1980); mechanical microinjection and/or particle acceleration (Anderson et al., Proc. Natl. Acad. Sci. USA, 77: 5399-5403 (1980); liposome based DNA transfer (e.g., LIPOFECTIN-mediated transfection- Fefgner et al., Proc. Nat. Acad. Sci., USA, 84: 471-477,1987; Gao and Huang, Biochim. Biophys. Res. Comm., 179: 280-285,1991; DEAE Dextran-mediated transfection; electroporation (U.S. Patent 4.956.288); or poly lysine-based methods in which DNA is conjugated to deliver DNA preferentially to liver hepatocytes (Wolff et al., Science, 247: 465-468,1990; Curiel et al., Human Gene Therapy 3: 147-154,1992).

Target cells may be transfected with the genes of the invention by direct gene transfer. See, e.g., Wolff et al., "Direct Gene Transfer Into Moose Muscle In Vivo", Science 247:1465-68, 1990. In many cases, vector-mediated transfection will be desirable. Any of the methods known in the art for the insertion of polynucleotide sequences into a vector may be used. See, for example, Sambrook et al., Molecular Cloning: A Laboratory Manual, Cold Spring Harbor Laboratory, Cold Spring Harbor, NY (1989) and Ausubel et al.. Current Protocols in Molecular Biology, J. Wiley & Sons, NY (1992). Promoter activation may be tissue specific or inducible by a metabolic product or administered substance. Such promoters/enhancers include, but are not limited to, the native RetL promoter, the cytomegalovirus immediate-early promoter/enhancer (Karasuyama et al., J. Exp. Med., 169: 13 (1989)); the human beta-actin promoter (Gunning et al., Proc. Nat. Acad. Sci. USA, 84: 4831 (1987); the glucocorticoid-inducible promoter present in the mouse mammary tumor virus long terminal repeat (MMTV LTR) (Klessig et al., Mol. Cell. Biol., 4: 1354 (1984)); the long terminal repeat sequences of Moloney murine leukemia virus (MuLV LTR) (Weiss et al.. RNA Tumor Viruses, Cold Spring Harbor Laboratory, Cold Spring Harbor. NY (1985)); the SV40 early region promoter (Bernoist and Chambon, Nature, 290:304 (1981)); the promoter of the Rous sarcoma virus (RSV) (Yamamoto et al., Cell, 22:787 (1980)); the herpes simplex virus (HSV) thymidine kinase promoter (Wagner et at., Proc. Nat. Acad. Sci. USA. 78: 1441 (1981)); the adenovirus promoter (Yamada et al., Proc. Nat. Acad. Sci. USA, 82: 3567 (1985)).

The RetL genes may also be introduced by specific viral vectors for use in gene transfer systems which are now well established. See for example: Madzak et al., J. Gen. Virol., 73: 1533-36, 1992 (papovavirus SV40); Berkner et al., Curr. Top. Microbiol. Immunol., 158: 39-61, 1992 (adenovirus); Hofmann et al., Proc. Natl. Acad. Sci. 92: 10099-10103,1995 (baculovirus); Moss et al., Curr. Top. Microbiol. Immunol., 158: 25-38,1992 (vaccinia virus); Muzyczka, Curr. Top. Microbiol. Immunol., 158: 97-123,1992 (adeno-associated virus); Margulskee, Curr. Top. Microbiol. Immunol., 158: 67-93, 1992 (herpes simplex virus (HSV) and Epstein-Barr virus (HBV)); Miller, Curr. Top. Microbiol. Immunol., 158: 1-24,1992 (retrovirus); Brandyopadhyay et al., Mol. Cell. Biol., 4: 749-754,1984 (retrovirus); Miller et al., Nature, 357: 455-450,1992 (retrovirus); Anderson, Science, 256: 808-813, 1992 (retrovirus), Current Protocols in Molecular Biology: Sections 9.10-9.14 (Ausubel et al., Eds.), Greene Publishing Associates, 1989.

Preferred vectors are DNA viruses that include adenoviruses (preferably Ad-2 or Ad-5 based vectors), baculovirus, herpes viruses (preferably herpes simplex virus based vectors), and parvoviruses (preferably "defective" or non-autonomous parvovirus based vectors, more preferably adeno-associated virus based vectors, most preferably AAV-2 based vectors). See, e.g., Ali et al., Gene Therapy 1:367-384, 1994; U.S. Patent 4,797,368 and 5,399,346 and discussion below.

The choice of a particular vector system for transferring, for instance, a RetL sequence will depend on a variety of factors. One important factor is the nature of the target cell population. Although retroviral vectors have been extensively studied and used in a number of gene therapy applications, they are generally unsuited for infecting cells that are not dividing but may be useful in cancer therapy since they only integrate and express their genes in replicating cells. They are useful for ex vivo approaches and are attractive in this regard due to their stable integration into the target cell genome.

Adenoviruses are eukaryotic DNA viruses that can be modified to efficiently deliver a therapeutic or reporter transgene to a variety of cell types. The general adenoviruses types 2 and 5 (Ad2 and Ad5, respectively), which cause respiratory disease in humans, are currently being developed for gene therapy of Duchenne Muscular Dystrophy (DMD)and Cystic Fibrosis (CF). Both Ad2 and Ad5 belong to a subclass of adenovirus that are not associated with human malignancies. Adenovirus vectors are capable of providing extremely high levels of transgene delivery to virtually all cell types, regardless of the mitotic state. High titers (10¹³ plaque forming units/ml) of recombinant virus can be easily generated in 293 cells (an adenovirus-transformed, complementation human embryonic kidney cell line: ATCC CRL1573) and cryo-stored for extended periods without appreciable losses. The efficacy of this system in delivering a therapeutic transgene *in vivo* that complements a genetic imbalance has been demonstrated in animal models of various disorders. See Y. Watanabe, Atherosclerosis, 36: 261-268 (1986); K. Tanzawa et al, FEBS Letters. 118(1):81-84 (1980); J.L. Golasten et al, New Engl.J. Med., 309 (11983): 288-296 (1983); S. Ishibashi et al, J. Clin. Invest., 92: 883-893 (1993); and S. Ishibashi et al, J. Clin. Invest., 93: 1889-1893 (1994). Indeed, recombinant replication defective adenovirus encoding a cDNA for the cystic fibrosis transmembrane regulator (CFTR) has been approved for use in at least two human CF clinical trials. See, e.g., J. Wilson, Nature, 365: 691-692 (Oct., 21, 1993). Further support of the safety of recombinant adenoviruses for gene therapy is the extensive experience of live adenovirus vaccines in human populations.

Human adenoviruses are comprised of a linear, approximately 36 kb double-stranded DNA genome, which is divided into 100 map units (m.u.), each of which is 360 bp in length. The DNA contains short inverted terminal repeats (ITR) at each end of the genome that are required for viral DNA replication. The gene products are organized into early (E1 through E4) and late (L1 through L5) regions, based on expression before or after the initiation of viral DNA synthesis. See, e.g., Horwitz, Virology, 2d edit., ed. B.N. Fields, Raven Press Ltd., New York (1990).

The first-generation recombinant, replication-deficient adenoviruses which have been developed for gene therapy of DMD and other inherited disorders contain deletions of the entire E1a and part of the E1b regions. This replication-defective virus is grown in 293 cells containing a functional adenovirus E1a gene which provides a transacting E1a protein. E1-deleted viruses are capable of replicating and producing infectious virus in the 293 cells, which provide E1a and E1b region gene products in *trans.* The resulting virus is capable of infecting many cell types and can express the introduced gene (providing it carries its own promoter), but cannot replicate in a cell that does not carry the E1 region DNA unless the cell is infected at a very high multiplicity of infection. Adenoviruses have the advantage that they have a broad host range, can infect quiescent or terminally differentiated cells such as neurons, and appear essentially non-oncogenic. Adenoviruses do not appear to integrate in to the host genome. Because they exist extrachromasomally, the risk of insertional mutagenesis is greatly reduced. Ali et al., supra, at 373. Recombinant adenoviruses (rAdV) produce very high titers, the viral particles are moderately stable, expression levels are high, and a wide range of cells can be infected. Their natural host cells are airway epithelium, so they are useful for therapy of lung cancers.

Baculovirus-mediated transfer has several advantages. Baculoviral gene transfer can occur in replicating and nonreplicating cells, and can occur in renal cells, as well as in hepatocytes, neural cells, spleen, skin, and muscle. Baculovirus is non-replicating and nonpathogenic in mammalian cells. Humans lack pre-existing antibodies to recombinant baculovirus which could block infection. In addition, baculovirus is capable of incorporating and transducing very large DNA inserts.

Adeno-associated viruses (AAV) have also been employed as vectors for somatic gene therapy. AAV is a small, single-stranded (ss) DNA virus with a simple genomic organization (4.7 kb) that makes it an ideal substrate for genetic engineering. Two open reading frames encode a series of *rep* and *cap* polypeptides. *Rep* polypeptides (*rep78, rep68, rep 62 and rep 40)* are involved in replication, rescue and integration of the AAV genome. The cap proteins (VP1, VP2 and VP3) form the virion capsid. Flanking the rep and *cap* open reading frames at the 5' and 3' ends are 145 bp inverted terminal repeats (ITRs), the first 125 bp of which are capable of forming Y- or T-shaped duplex structures. Of importance for the development of AAV vectors, the entire rep and *cap* domains can be excised and replaced with a therapeutic or reporter transgene. See B.J. Carter, in Handbook of Parvoviruses, ed., P. Tijsser, CRC Press, pp. 155-168 (1990). It has been shown that the ITRs represent the minimal sequence required for replication, rescue, packaging, and integration of the AAV genome.

The AAV life cycle is biphasic, composed of both latent and lytic episodes. During a latent infection, AAV virions enter a cell as an encapsilated ssDNA, and shortly thereafter are delivered to the nucleus where the AAV DNA stably integrates in to a host chromosome without the apparent need for host cell division. In the absence of a helper virus, the integrated AAV genome remains latent but capable of being activated and rescued. The lytic phase of the life cycle begins when a cell harboring an AAV provirus is challenged with a secondary infection by a herpesvirus or adenovirus which encodes helper functions that are recruited by AAV to aid in its excision from host chromatin (B.J. Carter, supra). The infecting parental ssDNA is expanded to duplex replicating form (RF) DNAs in a rep dependent manner. The rescued AAV genomes are packaged into preformed protein capsids (icosahedral symmetry approximately 20 nm in diameter) and released as infectious virions that have packaged either + or - ssDNA genomes following cell lysis.

Adeno-associated viruses (AAV) have significant potential in gene therapy. The viral particles are very stable and recombinant AAVs (rAAV)have "drug-like" characteristics in that rAAV can be purified by pelleting or by CsCl gradient banding. They are heat stable and can be lyophilized to a powder and rehydrated to full activity. Their DNA stably integrates into host chromosomes so expression is long-term. Their host range is broad and AAV causes no known disease so that the recombinant vectors are non-toxic.

Once introduced into a target cell, sequences of interest can be identified by conventional methods such as nucleic acid hybridization using probes comprising sequences that are homologous/complementary to the inserted gene sequences of the vector. In another approach, the sequence(s) may be identified by the presence or absence of a "marker" gene function (e.g, thymidine kinase activity, antibiotic resistance, and the like) caused by introduction of the expression vector into the target cell.

### Formulations and Administration

The compounds of the invention may be administered in any manner which is medically acceptable. This may include injections, by parenteral routes such as intravenous, intravascular, intraarterial, subcutaneous, intramuscular, intratumor, intraperitoneal, intraventricular, intraepidural, or others as well as oral, nasal, ophthalmic, rectal, or topical. Sustained release administration is also specifically included in the invention, by such means as depot injections or erodible implants. Localized delivery is particularly contemplated, by such means as delivery via a catheter to one or more arteries, such as the renal artery or a vessel supplying a localized tumor.

The term "pharmaceutically acceptable carrier" means one or more organic or inorganic ingredients, natural or synthetic, with which the mutant proto-oncogene or mutant oncoprotein is combined to facilitate its application. A suitable carrier includes sterile saline although other aqueous and non-aqueous isotonic sterile solutions and sterile suspensions known to be pharmaceutically acceptable are known to those of ordinary skill in the art. In this regard, the term "carrier" encompasses liposomes and the HIV-1 tat protein (See Chen et al., Anal. Biochem. 227: 168-175, 1995) as well as any plasmid and viral expression vectors. An "effective amount" refers to that amount which is capable of ameliorating or delaying progression of the diseased, degenerative or damaged condition. An effective amount can be determined on an individual basis and will be based, in part, on consideration of the symptoms to be treated and results sought. An effective amount can be determined by one of ordinary skill in the art employing such factors and using no more than routine experimentation.

The liposome system may be any variety of unilamellar vesicles, multilamellar vesicles, or stable plurilamellar vesicles, and may be prepared and administered according to methods well known to those of skill in the art, for example in accordance with the teachings of United States Patents 5,169,637, 4,762,915, 5,000,958 or 5,185,154. In addition, it may be desirable to express the novel polypeptides of this invention, as well as other selected polypeptides, as lipoproteins, in order to enhance their binding to liposomes. As an example, treatment of human acute renal failure with liposome-encapsulated RetL may be performed in vivo by introducing a RetL into cells in need of such treatment using liposomes . The liposomes can be delivered via catheter to the renal artery. The recombinant RetL protein is purified, for example, from CHO cells by immunoaffinity chromatography or any other convenient method, then mixed with liposomes and incorporated into them at high efficiency. The encapsulated protein may be tested in vitro for any effect on stimulating cell growth.

This invention also contemplates that the novel polypeptide of this invention may be administered to an animal via liposome delivery system in order to enhance their stability and/or immunogenicity. Delivery of the novel polypeptides via liposomes may be particularly advantageous because the liposome may be internalized by phagocytic cells in the treated animal. Such cells, upon ingesting the liposomal membrane and subsequently present the polypeptides to the immune system in conjunction with other molecules required to elicit a strong immune response.

Any of the novel RetL polypeptides of this invention may be used in the form of a pharmaceutically acceptable salt. Suitable acids and bases which are capable of forming salts with the polypeptides of the present invention are well known to those of skill in the art, and include inorganic and organic acids and bases.

## Claims

1. An isolated nucleic acid encoding a polypeptide with at least 80% sequence identify to an amino acid sequence selected from the group consisting of SEQ ID NO: 17 and SEQ ID NO:21, wherein said polypeptide interacts with a receptor protein Ret to trigger dimerization of the receptor protein Ret or autophosphorylation of a tyrosine kinase domain of the receptor protein Ret.

2. The nucleic acid of claim 1, wherein the amino acid sequence of the encoded polypeptide retains at least 80% of the cysteines when aligned with SEQ ID NO:17 or SEQ ID NO:21.

3. The nucleic acid of claim 1, wherein the encoded polypeptide has at least 90% sequence identity to an amino acid sequence selected from the group consisting of SEQ ID NO:17 and SEQ ID NO:21.

4. The nucleic acid of claim 3, wherein the encoded polypeptide is SEQ ID NO: 17 or SEQ ID NO:21.

5. An isolated nucleic acid comprising a nucleotide sequence selected from the group consisting of SEQ ID NO:16 and SEQ ID NO:20.

6. A vector comprising an insert comprising the nucleic acid according to any one of claims 1-5.

7. A host cell comprising the vector according to claim 6.

8. A method for producing a polypeptide, comprising the steps of: culturing the host cell according to claim 7; and recovering the polypeptide expressed from the insert within said host cell.

9. A polypeptide encoded by the nucleic acid according to any one of claims 1-5.

10. An isolated monoclonal antibody that binds to a polypeptide selected from the group consisting of SEQ ID NO:15, SEQ ID NO:17, SEQ ID NO:19 and SEQ ID NO:21.

11. The antibody of claim 10, wherein the antibody binds to the polypeptide of SEQ ID NO:19 or SEQ ID NO:21.

12. A composition comprising: (a) an antibody according to claims 10 or 11, associated to (b) a toxin, imageable compound or radionuclide.

13. A fusion protein comprising a polypeptide according to claim 9, fused to an immunoglobulin, a toxin, an imageable compound, or a radionuclide.

14. The nucleic acid according to any one of claims 1-5, the polypeptide according to claim 9, the vector according to claim 6, the antibody according to claims 10 or 11, the composition according to claim 12, or the fusion protein according to claim 13 for use in therapy or diagnosis.

## Patentansprüche

1. Eine isolierte Nukleinsäure, welche ein Polypeptid enkodiert, das mindestens 80% Sequenzidentität mit einer Aminosäuresequenz hat, die aus der Gruppe bestehend aus SEQ ID NO:17 und SEQ ID NO:21 ausgewählt ist, wobei besagtes Polypeptid mit einem Ret Rezeptorprotein interagiert, um die Dimerisierung des Ret Rezeptorproteins oder die Autophosphorylierung einer Tyrosinkinase Domäne des Ret Rezeptorproteins auszulösen.

2. Die Nukleinsäure gemäß Anspruch 1, wobei die Aminosäuresequenz des enkodierten Polypeptids in einem Alignment mit SEQ ID NO:17 oder SEQ ID NO:21 mindestens 80% der Cysteine beibehält.

3. Die Nukleinsäure gemäß Anspruch 1, wobei das enkodierte Polypeptid mindestens 90% Sequenzidentität zu einer Aminosäuresequenz ausgewählt aus der Gruppe bestehend aus SEQ ID NO:17 und SEQ ID NO:21 hat.

4. Die Nukleinsäure gemäß Anspruch 3, wobei das enkodierte Polypeptid SEQ ID NO:17 oder SEQ ID NO:21 ist.

5. Eine isolierte Nukleinsäure unfassend eine Nukleotidsequenz ausgewählt aus der Gruppe bestehend aus SEQ ID NO:16 und SEQ ID NO:20.

6. Ein Vektor umfassend einen Insert umfassend die Nukleinsäure gemäß einem beliebigen der Ansprüche 1-5.

7. Eine Wirtszelle umfassend den Vektor gemäß Anspruch 6.

8. Ein Verfahren zur Herstellung eines Polypeptids, umfassend die Schritte der: Kultivierung der Wirtszelle gemäß Anspruch 7; und Gewinnung des Polypeptids, das von dem Insert innerhalb besagter Wirtszelle exprimiert wird.

9. Ein Polypeptid welches durch die Nukleinsäure gemäß einer beliebigen der Ansprüche 1-5 enkodiert wird.

10. Ein isolierter monoklonaler Antikörper, welcher ein Polypeptid ausgewählt aus der Gruppe bestehend aus SEQ ID NO:15, SEQ ID NO:17, SEQ ID NO:19 und SEQ ID NO:21 bindet.

11. Der Antikörper gemäß Anspruch 10, wobei der Antikörper an das Polypeptid von SEQ ID NO:19 oder SEQ ID NO:21 bindet.

12. Eine Zusammensetzung umfassend: (a) einen Antikörper gemäß Anspruch 10 oder 11, verbunden mit (b) einem Toxin, einer bildgebenden Verbindung oder einem Radionuklid.

13. Ein Fusionsprotein umfassend ein Polypeptid gemäß Anspruch 9 fusioniert mit einem Immunoglobulin, einem Toxin, einer bildgebenden Verbindung oder einem Radionuklid.

14. Die Nukleinsäure gemäß einem beliebigen der Ansprüche 1-5, das Polypeptid gemäß Anspruch 9, der Vektor gemäß Anspruch 6, der Antikörper gemäß Anspruch 10 oder 11, die Zusammensetzung gemäß Anspruch 12 oder das Fusionsprotein gemäß Anspruch 13 zur Verwendung in der Therapie oder in der Diagnose.

## Revendications

1. Acide nucléique isolé codant pour un polypeptide ayant une identité de séquence d'au moins 80 % avec une séquence d'aminoacides choisie dans le groupe consistant en la SEQ ID NO: 17 et la SEQ ID NO: 21, ledit polypeptide interagissant avec une protéine réceptrice Ret pour déclencher la dimérisation de la protéine réceptrice Ret ou l'autophosphorylation d'un domaine de tyrosine-kinase de la protéine réceptrice Ret.

2. Acide nucléique suivant la revendication 1, dans lequel la séquence d'aminoacides du polypeptide codé conserve au moins 80 % des cystéines lors de l'alignement avec la SEQ ID NO: 17 ou la SEQ ID NO: 21.

3. Acide nucléique suivant la revendication 1, dans lequel le polypeptide codé présente une identité de séquence d'au moins 90 % avec une séquence d'aminoacides choisie dans le groupe consistant en la SEQ ID NO: 17 et la SEQ ID NO: 21.

4. Acide nucléique suivant la revendication 3, dans lequel le polypeptide codé est la SEQ ID NO: 17 ou la SEQ ID NO: 21.

5. Acide nucléique isolé comprenant une séquence de nucléotides choisie dans le groupe consistant en la SEQ ID NO:16 et la SEQ ID NO: 20.

6. Vecteur comprenant un segment d'insertion comprenant l'acide nucléique suivant l'une quelconque des revendications 1 à 5.

7. Cellule hôte comprenant le vecteur suivant la revendication 6.

8. Procédé pour la production d'un polypeptide, comprenant les étapes consistant : à cultiver la cellule hôte suivant la revendication 7 et à recueillir le polypeptide exprimé à partir du segment d'insertion dans ladite cellule hôte.

9. Polypeptide codé par l'acide nucléique suivant l'une quelconque des revendications 1 à 5.

10. Anticorps monoclonal isolé qui se lie à un polypeptide choisi dans le groupe consistant en les SEQ ID NO:15, SEQ ID NO:17, SEQ ID NO:19 et SEQ ID NO: 21.

11. Anticorps suivant la revendication 10, l'anticorps se liant au polypeptide de la SEQ ID NO: 19 ou de la SEQ ID NO: 21.

12. Composition comprenant: (a) un anticorps suivant la revendication 10 ou 11, associé à (b) une toxine, un composé apte à l'imagerie ou un radionucléide.

13. Protéine de fusion comprenant un polypeptide suivant la revendication 9, fusionné à une immunoglobuline, une toxine, un composé apte à l'imagerie ou un radionucléide.

14. Acide nucléique suivant l'une quelconque des revendications 1 à 5, polypeptide suivant la revendication 9, vecteur suivant la revendication 6, anticorps suivant la revendication 10 ou 11, composition suivant la revendication 12 ou protéine de fusion suivant la revendication 13, destiné à être utilisé en thérapeutique ou en diagnostic.


### SEQUENCE LISTING

(1) GENERAL INFORMATION:
   (i) APPLICANT: doherty, jodi
   (ii) TITLE OF INVENTION: biogen patent application title
   (iii) NUMBER OF SEQUENCES: 21
   (iv) CORRESPONDENCE ADDRESS:
      (A) ADDRESSEE: Biogen, Inc.
      (B) STREET: 14 Cambridge Center
      (C) CITY: Cambridge
      (D) STATE: MA
      (E) COUNTRY: USA
      (F) ZIP: 02142
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE: 07-MAY-1997
      (C) CLASSIFICATION:
   (vii) PRIOR APPLICATION DATA:
      (A) APPLICATION NUMBER: US 08/999,999
      (B) FILING DATE: 01-JAN-1996
   (viii) ATTORNEY/AGENT INFORMATION:
      (A) NAME: Levine, Leslie M.
      (B) REGISTRATION NUMBER: 35,245
      (C) REFERENCE/DOCKET NUMBER: A008 PCT CIP
   (ix) TELECOMMUNICATION INFORMATION:
      (A) TELEPHONE: 617-679-2400
      (B) TELEFAX: 617-679-2838
(2) INFORMATION FOR SEQ ID NO:1:
   (i) SEQUENCE CHARACTERISTICS:
      (A) LENGTH: 3616 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 257..1660
   (xi) SEQUENCE DESCRIPTION: SEQ ID NO:1:
   GCGGCCGCAG GTTGGGTCGG AACTGAACCC CTGAAAGCGG GTCCGCCTCC CGCCCTCGCG      60
   CCCGCCCGGA TCTGAGTCGC TGGCGGCGGT GGGCGGCAGA GCGACGGGGA GTCTGCTCTC     120
   ACCCTGGATG GAGCTGAACT TTGAGTGGCC AGAGGAGCGC AGTCGCCCGG GGATCGCTGC     180
   ACGCTGAGCT CTCTCCCCGA GACCGGGCGG CGGCTTTGGA TTTTGGGGGG GCGGGGACCA     240
   GCTGCGCGGC GGCACC ATG TTC CTA GCC ACT CTG TAC TTC GCG CTG CCA         289
                     Met Phe Leu Ala Thr Leu Tyr Phe Ala Leu Pro
                      1               5                  10
   CTC CTG GAT TTG CTG ATG TCC GCC GAG GTG AGT GGT GGA GAC CGT CTG       337
   Leu Leu Asp Leu Leu Met Ser Ala Glu Val Ser Gly Gly Asp Arg Leu
               15                  20                  25
   GAC TGT GTG AAA GCC AGC GAT CAG TGC CTG AAG GAA CAG AGC TGC AGC       385
   Asp Cys Val Lys Ala Ser Asp Gln Cys Leu Lys Glu Gln Ser Cys Ser
           30                  35                  40
   ACC AAG TAC CGC ACA CTA AGG CAG TGC GTG GCG GGC AAG GAA ACC AAC       433
   Thr Lys Tyr Arg Thr Leu Arg Gln Cys Val Ala Gly Lys Glu Thr Asn
       45                  50                  55
   TTC AGC CTG ACA TCC GGC CTT GAG GCC AAG GAT GAG TGC CGT AGC GCC       481
   Phe Ser Leu Thr Ser Gly Leu Glu Ala Lys Asp Glu Cys Arg Ser Ala
   60                  65                  70                  75
   ATG GAG GCC TTG AAG CAG AAG TCT CTG TAC AAC TGC CGC TGC AAG CGG       529
   Met Glu Ala Leu Lys Gln Lys Ser Leu Tyr Asn Cys Arg Cys Lys Arg
                   80                  85                  90
   GGC ATG AAG AAA GAG AAG AAT TGT CTG CGT ATC TAC TGG AGC ATG TAC       577
   Gly Met Lys Lys Glu Lys Asn Cys Leu Arg Ile Tyr Trp Ser Met Tyr
               95                  100                 105
   CAG AGC CTG CAG GGA AAT GAC CTC CTG GAA GAT TCC CCG TAT GAG CCG       625
   Gln Ser Leu Gln Gly Asn Asp Leu Leu Glu Asp Ser Pro Tyr Glu Pro
           110                 115                 120
   GTT AAC AGC AGG TTG TCA GAT ATA TTC CGG GCA GTC CCG TTC ATA TCA       673
   Val Asn Ser Arg Leu Ser Asp Ile Phe Arg Ala Val Pro Phe Ile Ser
       125                 130                 135
   GAT GTT TTC CAG CAA GTG GAA CAC ATT TCC AAA GGG AAC AAC TGC CTG        721
   Asp Val Phe Gln Gln Val Glu His Ile Ser Lys Gly Asn Asn Cys Leu
   140                 145                 150                 155
   GAC GCA GCC AAG GCC TGC AAC CTG GAC GAC ACC TGT AAG AAG TAC AGG        769
   Asp Ala Ala Lys Ala Cys Asn Leu Asp Asp Thr Cys Lys Lys Tyr Arg
                   160                 165                 170
   TCG GCC TAC ATC ACC CCC TGC ACC ACC AGC ATG TCC AAC GAG GTC TGC        817
   Ser Ala Tyr Ile Thr Pro Cys Thr Thr Ser Met Ser Asn Glu Val Cys
               175                 180                 185
   AAC CGC CGT AAG TGC CAC AAG GCC CTC AGG CAG TTC TTC GAC AAG GTT        865
   Asn Arg Arg Lys Cys His Lys Ala Leu Arg Gln Phe Phe Asp Lys Val
           190                 195                 200
   CCG GCC AAG CAC AGC TAC GGG ATG CTC TTC TGC TCC TGC CGG GAC ATC        913
   Pro Ala Lys His Ser Tyr Gly Met Leu Phe Cys Ser Cys Arg Asp Ile
       205                 210                 215
   GCC TGC ACC GAG CGG CGG CGA CAG ACT ATC GTC CCC GTG TGC TCC TAT        961
   Ala Cys Thr Glu Arg Arg Arg Gln Thr Ile Val Pro Val Cys Ser Tyr
   220                 225                 230                 235
   GAA GAA CGA GAG AGG CCC AAC TGC CTG AGT CTG CAA GAC TCC TGC AAG       1009
   Glu Glu Arg Glu Arg Pro Asn Cys Leu Ser Leu Gln Asp Ser Cys Lys
                   240                 245                 250
   ACC AAT TAC ATC TGC AGA TCT CGC CTT GCA GAT TTT TTT ACC AAC TGC       1057
   Thr Asn Tyr Ile Cys Arg Ser Arg Leu Ala Asp Phe Phe Thr Asn Cys
               255                 260                 265
   CAG CCA GAG TCA AGG TCT GTC AGC AAC TGT CTT AAG GAG AAC TAC GCA       1105
   Gln Pro Glu Ser Arg Ser Val Ser Asn Cys Leu Lys Glu Asn Tyr Ala
           270                 275                 280
   GAC TGC CTC CTG GCC TAC TCG GGA CTG ATT GGC ACA GTC ATG ACT CCC       1153
   Asp Cys Leu Leu Ala Tyr Ser Gly Leu Ile Gly Thr Val Met Thr Pro
       285                 290                 295
   AAC TAC GTA GAC TCC AGC AGC CTC AGC GTG GCA CCA TGG TGT GAC TGC       1201
   Asn Tyr Val Asp Ser Ser Ser Leu Ser Val Ala Pro Trp Cys Asp Cys
   300                 305                 310                 315
   AGC AAC AGC GGC AAT GAC CTG GAA GAC TGC TTG AAA TTT CTG AAT TTT       1249
   Ser Asn Ser Gly Asn Asp Leu Glu Asp Cys Leu Lys Phe Leu Asn Phe
                   320                 325                 330
   TTT AAG GAC AAT ACT TGT CTC AAA AAT GCA ATT CAA GCC TTT GGC AAT       1297
   Phe Lys Asp Asn Thr Cys Leu Lys Asn Ala Ile Gln Ala Phe Gly Asn
               335                 340                 345
   GGC TCA GAT GTG ACC ATG TGG CAG CCA GCC CCT CCA GTC CAG ACC ACC       1345
   Gly Ser Asp Val Thr Met Trp Gln Pro Ala Pro Pro Val Gln Thr Thr
           350                 355                 360
   ACT GCC ACC ACT ACC ACT GCC TTC CGG GTC AAG AAC AAG CCT CTG GGG      1393
   Thr Ala Thr Thr Thr Thr Ala Phe Arg Val Lys Asn Lys Pro Leu Gly
       365                 370                 375
   CCA GCA GGG TCT GAG AAT GAG ATC CCC ACA CAC GTT TTA CCA CCC TGT      1441
   Pro Ala Gly Ser Glu Asn Glu Ile Pro Thr His Val Leu Pro Pro Cys
   380                 385                 390                 395
   GCG AAT TTG CAG GCT CAG AAG CTG AAA TCC AAT GTG TCG GGT AGC ACA      1489
   Ala Asn Leu Gln Ala Gln Lys Leu Lys Ser Asn Val Ser Gly Ser Thr
                   400                 405                 410
   CAC CTC TGT CTT TCT GAT AGT GAT TTC GGA AAG GAT GGT CTC GCT GGT      1537
   His Leu Cys Leu Ser Asp Ser Asp Phe Gly Lys Asp Gly Leu Ala Gly
               415                 420                 425
   GCC TCC AGC CAC ATA ACC ACA AAA TCA ATG GCT GCT CCT CCC AGC TGC      1585
   Ala Ser Ser His Ile Thr Thr Lys Ser Met Ala Ala Pro Pro Ser Cys
           430                 435                 440
   AGT CTG AGC TCA CTG CCG GTG CTG ATG CTC ACC GCC CTT GCT GCC CTG      1633
   Ser Leu Ser Ser Leu Pro Val Leu Met Leu Thr Ala Leu Ala Ala Leu
       445                 450                 455
   TTA TCT GTA TCG TTG GCA GAA ACG TCG TAGCTGCATC CGGGAAAACA            1680
   Leu Ser Val Ser Leu Ala Glu Thr Ser
   460                 465
   GTATGAAAAG ACAAAAGAGA ACCAAGTATT CTGTCCCTGT CCTCTTGTAT ATCTGAAAAT   1740
   CCAGTTTTAA AAGCTCCGTT GAGAAGCAGT TTCACCCAAC TGGAACTCTT TCCTTGTTTT   1800
   TAAGAAAGCT TGTGGCCCTC AGGGGCTTCT GTTGAAGAAC TGCTACAGGG CTAATTCCAA   1860
   ACCCATAAGG CTCTGGGGCG TGGTGCGGCT TAAGGGGACC ATTTGCACCA TGTAAAGCAA   1920
   GCTGGGCTTA TCATGTGTTT GATGGTGAGG ATGGTAGTGG TGATGATGAT GGTAATTTTA   1980
   ACAGCTTGAA CCCTGTTCTC TCTACTGGTT AGGAACAGGA GATACTATTG ATAAAGATTC   2040
   TTCCATGTCT TACTCAGCAG CATTGCCTTC TGAAGACAGG CCCGCAGCCT AGTGTGAATG   2100
   ACAAGTGGAG GTTGGCCTCA AGAGTGGACT TGGCAGACTC TACCTTGTAG TAATGTTCAC   2160
   CTTTCCGTGT ATGGTCTCCA CAGAGTGTTT ATGTATTTAC AGACTGTTCT GTGATCCCCC   2220
   AACAACAACA ACCACAAATT CCTTGGTCAC CTCCAAATGT AACCGGTCCT TTAGCCCAGT   2280
   AGAGGAGGGT GGGTGTGGCC CTGGCACAGC TCCCGGATTG TTGATGGGCA CTCTCCTGAG   2340
   CTTTGCTTGA GTGAGAAGCT GAATGTAGCT GAAAATCAAC TCTTCTTACA CTTCTTACTG   2400
   CTTCGTTCAC TTACGAGGTC ACATATAGAA CAAACATCAC CAACTATTAG CTTACCGTTA   2460
   GCTTCCCAAC TATTAGCTTT CTATGTTTTG AAAGCAGTGT TGCTGACCCC ATGTTTTAAT    2520
   GATGGTTTAA TACATGCAGC CCTTTCCTCT CATCGGTAAC ACTAGCTCCA ACATCAACTT    2580
   CATGCATGTG GCTCTCAAAA GCAGGCCCCA AGAAGCCCAG TTCTTTAGGA GAAAGCTGCG    2640
   TCCTGTTTCT GTGGACAGGC AGGAGGAAAC AGAGCAGCCT GCCCGTGGTG TCTTTATCTG    2700
   TTTTGAAATC AAGGCTGCCT GTGTGTAAGG AATGGTTCAA TTCTTATAAA GGGTGCCACT    2760
   GTTGATGCCA CAACTGGCAG TTGGTCTAGC TCCAGGACAC CGGTTTCCAT GTTGCCTGGC    2820
   AGAGACAGCT TTGATTGGGA CTGGCTGGCC ACAAGGGATG GGATGAAGAT GTGCTGCCCT    2880
   CTCTTTCAAA GTTGAGCCCT GCCAGGGCAC ATAGAAGCAT CTTTGCTCCT GACCACAACG    2940
   TAGAACAGCT TGGATTCAAG GTCATCAAGC GTCTCCTGTA CATTGCTCTG TGACCTTCAT    3000
   AACAGACTGT CCCGCACAAA AGGAACGGCA GTTTATGGAT CTAGAGTGGG AGCACAGGGT    3060
   CTGGAAAGGT GAACCGATTG GCAAAATACA CAGAACAGGA GGGAGAGTCT CAAGCCGAGA    3120
   CATCTTGCTT ACTAGCCACA CACCATCTCC TGGAGCCCTC CTCCTGACCT GGGCAGACCC    3180
   TTAGGTGTAT ATCTAAAGAC CTCTTCAATG TTCAGGTTCA GAATCTGTAA ATGGTTGCGT    3240
   CCTGGCACCC ATTCCTGAAA ACTGAACAAA GGAGAGGATA TCTTTCCTCC ATTGAGCCCT    3300
   GAAAGTATGA CTGGCTTCTC ACCCTCCCAC AGAGCAGGGA GCCCTGGTGC ACACAGTCTC    3360
   CTGATATCCT CCCTGCTCTT TGAGGTTTGC CTTGGGAGAA AATGATTCAC CTCGGGAGGG    3420
   GACGCTTTGG TGTCTGAAGT ACGTTTATAT CGAAATGTTA ATGAATACCC ATGTAAAATA    3480
   CTCAATAGCC ACCTTTCTTC CCTTCACAAT GTTTTCGAGG GGAATGCATC CAACATCCAA    3540
   GTGTACCTGG TCAGTGGGAA GTTCCATGAA GACTCATACA TTGAATAAAC ATATTCGATG    3600
   TGCCGAAAGC GGCCGC                                                    3616
(2) INFORMATION FOR SEQ ID NO:2:
   (i) SEQUENCE CHARACTERISTICS:
      (A) LENGTH: 468 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Phe Leu Ala Thr Leu Tyr Phe Ala Leu Pro Leu Leu Asp Leu Leu
    1               5                  10                  15
   Met Ser Ala Glu Val Ser Gly Gly Asp Arg Leu Asp Cys Val Lys Ala
               20                  25                  30
   Ser Asp Gln Cys Leu Lys Glu Gln Ser Cys Ser Thr Lys Tyr Arg Thr
           35                  40                  45
   Leu Arg Gln Cys Val Ala Gly Lys Glu Thr Asn Phe Ser Leu Thr Ser
       50                  55                  60
   Gly Leu Glu Ala Lys Asp Glu Cys Arg Ser Ala Met Glu Ala Leu Lys
   65                  70                  75                  80
   Gln Lys Ser Leu Tyr Asn Cys Arg Cys Lys Arg Gly Met Lys Lys Glu
                   85                  90                  95
   Lys Asn Cys Leu Arg Ile Tyr Trp Ser Met Tyr Gln Ser Leu Gln Gly
               100                 105                 110
   Asn Asp Leu Leu Glu Asp Ser Pro Tyr Glu Pro Val Asn Ser Arg Leu
           115                 120                 125
   Ser Asp Ile Phe Arg Ala Val Pro Phe Ile Ser Asp Val Phe Gln Gln
       130                 135                 140
   Val Glu His Ile Ser Lys Gly Asn Asn Cys Leu Asp Ala Ala Lys Ala
   145                 150                 155                 160
   Cys Asn Leu Asp Asp Thr Cys Lys Lys Tyr Arg Ser Ala Tyr Ile Thr
                   165                 170                 175
   Pro Cys Thr Thr Ser Met Ser Asn Glu Val Cys Asn Arg Arg Lys Cys
               180                 185                 190
   His Lys Ala Leu Arg Gln Phe Phe Asp Lys Val Pro Ala Lys His Ser
           195                 200                 205
   Tyr Gly Met Leu Phe Cys Ser Cys Arg Asp Ile Ala Cys Thr Glu Arg
       210                 215                 220
   Arg Arg Gln Thr Ile Val Pro Val Cys Ser Tyr Glu Glu Arg Glu Arg
   225                 230                 235                 240
   Pro Asn Cys Leu Ser Leu Gln Asp Ser Cys Lys Thr Asn Tyr Ile Cys
                   245                 250                 255
   Arg Ser Arg Leu Ala Asp Phe Phe Thr Asn Cys Gln Pro Glu Ser Arg
               260                 265                 270
   Ser Val Ser Asn Cys Leu Lys Glu Asn Tyr Ala Asp Cys Leu Leu Ala
           275                 280                 285
   Tyr Ser Gly Leu Ile Gly Thr Val Met Thr Pro Asn Tyr Val Asp Ser
       290                 295                 300
   Ser Ser Leu Ser Val Ala Pro Trp Cys Asp Cys Ser Asn Ser Gly Asn
   305 310 315 320
   Asp Leu Glu Asp Cys Leu Lys Phe Leu Asn Phe Phe Lys Asp Asn Thr
   325 330 335
   Cys Leu Lys Asn Ala Ile Gln Ala Phe Gly Asn Gly Ser Asp Val Thr
   340 345 350
   Met Trp Gln Pro Ala Pro Pro Val Gln Thr Thr Thr Ala Thr Thr Thr
   355 360 365
   Thr Ala Phe Arg Val Lys Asn Lys Pro Leu Gly Pro Ala Gly Ser Glu
   370 375 380
   Asn Glu Ile Pro Thr His Val Leu Pro Pro Cys Ala Asn Leu Gln Ala
   385 390 395 400
   Gln Lys Leu Lys Ser Asn Val Ser Gly Ser Thr His Leu Cys Leu Ser
   405 410 415
   Asp Ser Asp Phe Gly Lys Asp Gly Leu Ala Gly Ala Ser Ser His Ile
   420 425 430
   Thr Thr Lys Ser Met Ala Ala Pro Pro Ser Cys Ser Leu Ser Ser Leu
   435 440 445
   Pro Val Leu Met Leu Thr Ala Leu Ala Ala Leu Leu Ser Val Ser Leu
   450 455 460
   Ala Glu Thr Ser
   465
(2) INFORMATION FOR SEQ ID NO:3:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
      AAGGAAAAAA GCGGCCGCCA TGGCGAAGGC GACGTCCGG 39
(2) INFORMATION FOR SEQ ID NO:4:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
      AGTTTTGTCG ACCGTGCGGC ACAGCTCGTC 33
(2) INFORMATION FOR SEQ ID NO:5:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
      AGTTTTGTCG ACCGTGCGGC ACAGCGCATC ACA 33
(2) INFORMATION FOR SEQ ID NO:6:
   (i) SEQUENCE CHARACTERISTICS:
      (A) LENGTH: 1926 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 10..1920
   (xi) SEQUENCE DESCRIPTION: SEQ ID NO:6:
   GCGGCCGCC ATG GCG AAG GCG ACG TCC GGC GCC GCA GGG CTG GGG CTG      48
             Met Ala Lys Ala Thr Ser Gly Ala Ala Gly Leu Gly Leu
                 470                 475                 480
   AAG CTG TTT TTG CTG CTG CCG CTA CTG GGA GAA GCC CCG CTG GGT CTC    96
   Lys Leu Phe Leu Leu Leu Pro Leu Leu Gly Glu Ala Pro Leu Gly Leu
               485                 490                 495
   TAC TTC TCA AGG GAT GCT TAC TGG GAG AGG CTG TAT GTG GAC CAG CCA      144
   Tyr Phe Ser Arg Asp Ala Tyr Trp Glu Arg Leu Tyr Val Asp Gln Pro
           500                 505                 510
   GCT GGC ACA CCT CTG CTC TAT GTC CAT GCC CTA CGG GAT GCC CCT GGA      192
   Ala Gly Thr Pro Leu Leu Tyr Val His Ala Leu Arg Asp Ala Pro Gly
       515                 520                 525
   GAA GTG CCC AGC TTC CGC CTG GGC CAG TAT CTC TAT GGC GTC TAC CGC      240
   Glu Val Pro Ser Phe Arg Leu Gly Gln Tyr Leu Tyr Gly Val Tyr Arg
   530                 535                 540                 545
   ACG CGT CTG CAT GAG AAT GAC TGG ATC CAC ATC GAT GCG GGC ACT GGC      288
   Thr Arg Leu His Glu Asn Asp Trp Ile His Ile Asp Ala Gly Thr Gly
                   550                 555                 560
   CTC CTC TAC CTC AAT CAG AGC CTG GAC CAT AGT TCC TGG GAG CAG CTC      336
   Leu Leu Tyr Leu Asn Gln Ser Leu Asp His Ser Ser Trp Glu Gln Leu
               565                 570                 575
   AGC ATC CGA AAT GGC GGC TTC CCC TTG CTC ACC GTC TTC CTC CAG GTC      384
   Ser Ile Arg Asn Gly Gly Phe Pro Leu Leu Thr Val Phe Leu Gln Val
           580                 585                 590
   TTC CTG GGG TCC ACA GCC CAG AGA GAG GGA GAG TGT CAT TGG CCA GGC      432
   Phe Leu Gly Ser Thr Ala Gln Arg Glu Gly Glu Cys His Trp Pro Gly
       595                 600                 605
   TGT GCC CGT GTG TAC TTC TCC TTC ATC AAC GAC ACC TTC CCA AAT TGT      480
   Cys Ala Arg Val Tyr Phe Ser Phe Ile Asn Asp Thr Phe Pro Asn Cys
   610                 615                 620                 625
   AGC TCC TTC AAA GCC CGG GAT CTC TGC ACC CCA GAG ACG GGT GTG TCC      528
   Ser Ser Phe Lys Ala Arg Asp Leu Cys Thr Pro Glu Thr Gly Val Ser
                   630                 635                 640
   TTC CGC ATC AGG GAG AAC AGG CCC CCT GGC ACC TTC TAC CAG TTC CGC      576
   Phe Arg Ile Arg Glu Asn Arg Pro Pro Gly Thr Phe Tyr Gln Phe Arg
               645                 650                 655
   ATG CTA CCT GTG CAG TTC CTT TGT CCT AAC ATC AGT GTG AAG TAC AAA      624
   Met Leu Pro Val Gln Phe Leu Cys Pro Asn Ile Ser Val Lys Tyr Lys
           660                 665                 670
   CTC TTA GAA GGG GAC GGT CTG CCC TTC CGT TGT GAC CCC GAC TGT CTG      672
   Leu Leu Glu Gly Asp Gly Leu Pro Phe Arg Cys Asp Pro Asp Cys Leu
       675                 680                 685
   GAG GTG AGC ACG CGG TGG GCA CTG GAT CGG GAG CTT CAG GAG AAG TAT      720
   Glu Val Ser Thr Arg Trp Ala Leu Asp Arg Glu Leu Gln Glu Lys Tyr
   690                 695                 700                 705
   GTG CTG GAG GCT GAG TGC GCA GTG GCA GGC CCT GGA GCC AAC AAG GAG      768
   Val Leu Glu Ala Glu Cys Ala Val Ala Gly Pro Gly Ala Asn Lys Glu
                   710                 715                 720
   AAG GTG GCC GTG TCC TTC CCG GTG ACG GTG TAT GAT GAA GAC GAC TCC        816
   Lys Val Ala Val Ser Phe Pro Val Thr Val Tyr Asp Glu Asp Asp Ser
               725                 730                 735
   CCG CCC ACC TTC TCC GGA GGT GTG GGC ACC GCC AGT GCT GTG GTG GAG        864
   Pro Pro Thr Phe Ser Gly Gly Val Gly Thr Ala Ser Ala Val Val Glu
           740                 745                 750
   TTT AAG CGG AAG GAG GGC ACT GTG GTA GCC ACT CTG CAG GTG TTT GAT        912
   Phe Lys Arg Lys Glu Gly Thr Val Val Ala Thr Leu Gln Val Phe Asp
       755                 760                 765
   GCA GAT GTG GTG CCA GCA TCT GGG GAG CTG GTG AGG CGG TAC ACA AGC        960
   Ala Asp Val Val Pro Ala Ser Gly Glu Leu Val Arg Arg Tyr Thr Ser
   770                 775                 780                 785
   ACA CTA CTC TCA GGG GAT TCC TGG GCC CAG CAG ACC TTC CGG GTG GAG       1008
   Thr Leu Leu Ser Gly Asp Ser Trp Ala Gln Gln Thr Phe Arg Val Glu
                   790                 795                 800
   CAC ACA CCC AAC GAG ACC TTG GTC CAG TCC AAC AAC AAC TCC GTG CGG       1056
   His Thr Pro Asn Glu Thr Leu Val Gln Ser Asn Asn Asn Ser Val Arg
               805                 810                 815
   GCA ACC ATG CAC AAT TAC AAG CTG GTT CTC AAC AGG AGC CTG TCC ATC       1104
   Ala Thr Met His Asn Tyr Lys Leu Val Leu Asn Arg Ser Leu Ser Ile
           820                 825                 830
   TCA GAG AGC CGA GTC CTG CAG CTA GTA GTC CTG GTC AAT GAC TCA GAC       1152
   Ser Glu Ser Arg Val Leu Gln Leu Val Val Leu Val Asn Asp Ser Asp
       835                 840                 845
   TTC CAG GGG CCT GGG TCA GGT GTT CTC TTC CTC CAT TTC AAC GTG TCT       1200
   Phe Gln Gly Pro Gly Ser Gly Val Leu Phe Leu His Phe Asn Val Ser
   850                 855                 860                 865
   GTG CTG CCT GTC ACC CTG AAC CTA CCC ATG GCC TAC TCC TTC CCA GTG       1248
   Val Leu Pro Val Thr Leu Asn Leu Pro Met Ala Tyr Ser Phe Pro Val
                   870                 875                 880
   AAT AGG AGA GCC CGC CGT TAT GCC CAG ATT GGG AAA GTT TGC GTG GAG       1296
   Asn Arg Arg Ala Arg Arg Tyr Ala Gln Ile Gly Lys Val Cys Val Glu
               885                 890                 895
   AAC TGC CAG GAG TTC AGC GGT GTC TCC ATC CAG TAC AAG CTG CAG CCC       1344
   Asn Cys Gln Glu Phe Ser Gly Val Ser Ile Gln Tyr Lys Leu Gln Pro
           900                 905                 910
   TCC AGC ACC AAC TGC AGT GCC CTA GGT GTG GTC ACC TCA ACA GAA GAC       1392
   Ser Ser Thr Asn Cys Ser Ala Leu Gly Val Val Thr Ser Thr Glu Asp
       915                 920                 925
   ACC TCA GGG ACC CTA TAT GTA AAT GAC ACG GAG GCC CTG CGG CGA CCT      1440
   Thr Ser Gly Thr Leu Tyr Val Asn Asp Thr Glu Ala Leu Arg Arg Pro
   930                 935                 940                 945
   GAG TGT ACC GAG CTT CAG TAC ACA GTG GTA GCC ACT GAC CGG CAG ACC      1488
   Glu Cys Thr Glu Leu Gln Tyr Thr Val Val Ala Thr Asp Arg Gln Thr
                   950                 955                 960
   CGC AGG CAG ACC CAA GCT TCG TTA GTC GTC ACA GTG GAG GGG ACA TAC      1536
   Arg Arg Gln Thr Gln Ala Ser Leu Val Val Thr Val Glu Gly Thr Tyr
               965                 970                 975
   ATT GCA GAA GAA GTG GGC TGC CCC AAG TCC TGT GCA GTA AAC AAG AGG      1584
   Ile Ala Glu Glu Val Gly Cys Pro Lys Ser Cys Ala Val Asn Lys Arg
           980                 985                 990
   CGA CCT GAG TGT GAG GAG TGT GGT GGC CTG GGT TCT CCA ACT GGC AGA      1632
   Arg Pro Glu Cys Glu Glu Cys Gly Gly Leu Gly Ser Pro Thr Gly Arg
       995                 1000                1005
   TGT GAG TGG CGT CAG GGA GAT GGT AAA GGG ATC ACC AGG AAC TTC TCC      1680
   Cys Glu Trp Arg Gln Gly Asp Gly Lys Gly Ile Thr Arg Asn Phe Ser
   1010                1015                1020                1025
   ACC TGT TCT CCT AGC ACC AGG ACC TGT CCT GAT GGC CAC TGT GAT GCT      1728
   Thr Cys Ser Pro Ser Thr Arg Thr Cys Pro Asp Gly His Cys Asp Ala
                   1030                1035                1040
   CTG GAG AGC CGG GAT ATC AAC ATT TGC CCC CAG GAC TGT CTC CGT GGC      1776
   Leu Glu Ser Arg Asp Ile Asn Ile Cys Pro Gln Asp Cys Leu Arg Gly
               1045                1050                1055
   CCC ATT GTT GGC GGG CAT GAG CGA GGG GAG CGC CAG GGG ATT AAA GCC      1824
   Pro Ile Val Gly Gly His Glu Arg Gly Glu Arg Gln Gly Ile Lys Ala
           1060                1065                1070
   GGC TAT GGC ATC TGC AAC TGT TTC CCT GAT GAG AAG AAG TGC TTC TGC      1872
   Gly Tyr Gly Ile Cys Asn Cys Phe Pro Asp Glu Lys Lys Cys Phe Cys
       1075                1080                1085
   GAG CCA GAG GAC AGC CAG GGC CCA TTG TGT GAT GCG CTG TGC CGC ACG      1920
   Glu Pro Glu Asp Ser Gln Gly Pro Leu Cys Asp Ala Leu Cys Arg Thr
   1090                1095                1100                1105
   GTCGAC                                                               1926
(2) INFORMATION FOR SEQ ID NO:7:
   (i) SEQUENCE CHARACTERISTICS:
      (A) LENGTH: 637 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Ala Lys Ala Thr Ser Gly Ala Ala Gly Leu Gly Leu Lys Leu Phe
   1 5 10 15
   Leu Leu Leu Pro Leu Leu Gly Glu Ala Pro Leu Gly Leu Tyr Phe Ser
   20 25 30
   Arg Asp Ala Tyr Trp Glu Arg Leu Tyr Val Asp Gln Pro Ala Gly Thr
   35 40 45
   Pro Leu Leu Tyr Val His Ala Leu Arg Asp Ala Pro Gly Glu Val Pro
   50 55 60
   Ser Phe Arg Leu Gly Gln Tyr Leu Tyr Gly Val Tyr Arg Thr Arg Leu
   65 70 75 80
   His Glu Asn Asp Trp Ile His Ile Asp Ala Gly Thr Gly Leu Leu Tyr
   85 90 95
   Leu Asn Gln Ser Leu Asp His Ser Ser Trp Glu Gln Leu Ser Ile Arg
   100 105 110
   Asn Gly Gly Phe Pro Leu Leu Thr Val Phe Leu Gln Val Phe Leu Gly
   115 120 125
   Ser Thr Ala Gln Arg Glu Gly Glu Cys His Trp Pro Gly Cys Ala Arg
   130 135 140
   Val Tyr Phe Ser Phe Ile Asn Asp Thr Phe Pro Asn Cys Ser Ser Phe
   145 150 155 160
   Lys Ala Arg Asp Leu Cys Thr Pro Glu Thr Gly Val Ser Phe Arg Ile
   165 170 175
   Arg Glu Asn Arg Pro Pro Gly Thr Phe Tyr Gln Phe Arg Met Leu Pro
   180 185 190
   Val Gln Phe Leu Cys Pro Asn Ile Ser Val Lys Tyr Lys Leu Leu Glu
   195 200 205
   Gly Asp Gly Leu Pro Phe Arg Cys Asp Pro Asp Cys Leu Glu Val Ser
   210 215 220
   Thr Arg Trp Ala Leu Asp Arg Glu Leu Gln Glu Lys Tyr Val Leu Glu
   225 230 235 240
   Ala Glu Cys Ala Val Ala Gly Pro Gly Ala Asn Lys Glu Lys Val Ala
   245 250 255
   Val Ser Phe Pro Val Thr Val Tyr Asp Glu Asp Asp Ser Pro Pro Thr
   260 265 270
   Phe Ser Gly Gly Val Gly Thr Ala Ser Ala Val Val Glu Phe Lys Arg
           275                 280                 285
   Lys Glu Gly Thr Val Val Ala Thr Leu Gln Val Phe Asp Ala Asp Val
       290                 295                 300
   Val Pro Ala Ser Gly Glu Leu Val Arg Arg Tyr Thr Ser Thr Leu Leu
   305                 310                 315                 320
   Ser Gly Asp Ser Trp Ala Gln Gln Thr Phe Arg Val Glu His Thr Pro
                   325                 330                 335
   Asn Glu Thr Leu Val Gln Ser Asn Asn Asn Ser Val Arg Ala Thr Met
               340                 345                 350
   His Asn Tyr Lys Leu Val Leu Asn Arg Ser Leu Ser Ile Ser Glu Ser
           355                 360                 365
   Arg Val Leu Gln Leu Val Val Leu Val Asn Asp Ser Asp Phe Gln Gly
       370                 375                 380
   Pro Gly Ser Gly Val Leu Phe Leu His Phe Asn Val Ser Val Leu Pro
   385                 390                 395                 400
   Val Thr Leu Asn Leu Pro Met Ala Tyr Ser Phe Pro Val Asn Arg Arg
                   405                 410                 415
   Ala Arg Arg Tyr Ala Gln Ile Gly Lys Val Cys Val Glu Asn Cys Gln
               420                 425                 430
   Glu Phe Ser Gly Val Ser Ile Gln Tyr Lys Leu Gln Pro Ser Ser Thr
           435                 440                 445
   Asn Cys Ser Ala Leu Gly Val Val Thr Ser Thr Glu Asp Thr Ser Gly
       450                 455                 460
   Thr Leu Tyr Val Asn Asp Thr Glu Ala Leu Arg Arg Pro Glu Cys Thr
   465                 470                 475                 480
   Glu Leu Gln Tyr Thr Val Val Ala Thr Asp Arg Gln Thr Arg Arg Gln
                   485                 490                 495
   Thr Gln Ala Ser Leu Val Val Thr Val Glu Gly Thr Tyr Ile Ala Glu
               500                 505                 510
   Glu Val Gly Cys Pro Lys Ser Cys Ala Val Asn Lys Arg Arg Pro Glu
           515                 520                 525
   Cys Glu Glu Cys Gly Gly Leu Gly Ser Pro Thr Gly Arg Cys Glu Trp
       530                 535                 540
   Arg Gln Gly Asp Gly Lys Gly Ile Thr Arg Asn Phe Ser Thr Cys Ser
   545                 550                 555                 560
   Pro Ser Thr Arg Thr Cys Pro Asp Gly His Cys Asp Ala Leu Glu Ser
                   565                 570                 575
   Arg Asp Ile Asn Ile Cys Pro Gln Asp Cys Leu Arg Gly Pro Ile Val
               580                 585                 590
   Gly Gly His Glu Arg Gly Glu Arg Gln Gly Ile Lys Ala Gly Tyr Gly
           595                 600                 605
   Ile Cys Asn Cys Phe Pro Asp Glu Lys Lys Cys Phe Cys Glu Pro Glu
       610                 615                 620
   Asp Ser Gln Gly Pro Leu Cys Asp Ala Leu Cys Arg Thr
   625                 630                 635
(2) INFORMATION FOR SEQ ID NO:8:
   (i) SEQUENCE CHARACTERISTICS:
      (A) LENGTH: 1223 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1038
   (xi) SEQUENCE DESCRIPTION: SEQ ID NO:8:
   CTG CTG GAG GAT TCC CCA TAT GAA CCA GTT AAC AGC AGA TTG TCA GAT      48
   Leu Leu Glu Asp Ser Pro Tyr Glu Pro Val Asn Ser Arg Leu Ser Asp
           640                 645                 650
   ATA TTC CGG GTG GTC CCA TTC ATA TCA GTG GAG CAC ATT CCC AAA GGG      96
   Ile Phe Arg Val Val Pro Phe Ile Ser Val Glu His Ile Pro Lys Gly
       655                 660                 665
   AAC AAC TGC CTG GAT GCA GCG AAG GCC TGC AAC CTC GAC GAC ATT TGC     144
   Asn Asn Cys Leu Asp Ala Ala Lys Ala Cys Asn Leu Asp Asp Ile Cys
   670                 675                 680                 685
   AAG AAG TAC AGG TCG GCG TAC ATC ACC CCG TGC ACC ACC AGC GTG TCC     192
   Lys Lys Tyr Arg Ser Ala Tyr Ile Thr Pro Cys Thr Thr Ser Val Ser
                   690                 695                 700
   AAC GAT GTC TGC AAC CGC CGC AAG TGC CAC AAG GCC CTC CGG CAG TTC     240
   Asn Asp Val Cys Asn Arg Arg Lys Cys His Lys Ala Leu Arg Gln Phe
               705                 710                 715
   TTT GAC AAG GTC CCG GCC AAG CAC AGC TAC GGA ATG CTC TTC TGC TCC        288
   Phe Asp Lys Val Pro Ala Lys His Ser Tyr Gly Met Leu Phe Cys Ser
           720                 725                 730
   TGC CGG GAC ATC GCC TGC ACA GAG CGG AGG CGA CAG ACC ATC GTG CCT        336
   Cys Arg Asp Ile Ala Cys Thr Glu Arg Arg Arg Gln Thr Ile Val Pro
       735                 740                 745
   GTG TGC TCC TAT GAA GAG AGG GAG AAG CCC AAC TGT TTG AAT TTG CAG        384
   Val Cys Ser Tyr Glu Glu Arg Glu Lys Pro Asn Cys Leu Asn Leu Gln
   750                 755                 760                 765
   GAC TCC TGC AAG ACG AAT TAC ATC TGC AGA TCT CGC CTT GCG GAT TTT        432
   Asp Ser Cys Lys Thr Asn Tyr Ile Cys Arg Ser Arg Leu Ala Asp Phe
                   770                 775                 780
   TTT ACC AAC TGC CAG CCA GAG TCA AGG TCT GTC AGC AGC TGT CTA AAG        480
   Phe Thr Asn Cys Gln Pro Glu Ser Arg Ser Val Ser Ser Cys Leu Lys
               785                 790                 795
   GAA AAC TAC GCT GAC TGC CTC CTC GCC TAC TCG GGG CTT ATT GGC ACA        528
   Glu Asn Tyr Ala Asp Cys Leu Leu Ala Tyr Ser Gly Leu Ile Gly Thr
           800                 805                 810
   GTC ATG ACC CCC AAC TAC ATA GAC TCC AGT AGC CTC AGT GTG GCC CCA        576
   Val Met Thr Pro Asn Tyr Ile Asp Ser Ser Ser Leu Ser Val Ala Pro
       815                 820                 825
   TGG TGT GAC TGC AGC AAC AGT GGG AAC GAC CTA GAA GAG TGC TTG AAA        624
   Trp Cys Asp Cys Ser Asn Ser Gly Asn Asp Leu Glu Glu Cys Leu Lys
   830                 835                 840                 845
   TTT TTG AAT TTC TTC AAG GAC AAT ACA TGT CTT AAA AAT GCA ATT CAA        672
   Phe Leu Asn Phe Phe Lys Asp Asn Thr Cys Leu Lys Asn Ala Ile Gln
                   850                 855                 860
   GCC TTT GGC AAT GGC TCC GAT GTG ACC GTG TGG CAG CCA GCC TTC CCA        720
   Ala Phe Gly Asn Gly Ser Asp Val Thr Val Trp Gln Pro Ala Phe Pro
               865                 870                 875
   GTA CAG ACC ACC ACT GCC ACT ACC ACC ACT GCC CTC CGG GTT AAG AAC        768
   Val Gln Thr Thr Thr Ala Thr Thr Thr Thr Ala Leu Arg Val Lys Asn
           880                 885                 890
   AAG CCC CTG GGG CCA GCA GGG TCT GAG AAT GAA ATT CCC ACT CAT GTT        816
   Lys Pro Leu Gly Pro Ala Gly Ser Glu Asn Glu Ile Pro Thr His Val
       895                 900                 905
   TTG CCA CCG TGT GCA AAT TTA CAG GCA CAG AAG CTG AAA TCC AAT GTG        864
   Leu Pro Pro Cys Ala Asn Leu Gln Ala Gln Lys Leu Lys Ser Asn Val
   910                 915                 920                 925
   TCG GGC AAT ACA CAC CTC TGT ATT TCC AAT GGT AAT TAT GAA AAA GAA        912
   Ser Gly Asn Thr His Leu Cys Ile Ser Asn Gly Asn Tyr Glu Lys Glu
                   930                 935                 940
   GGT CTC GGT GCT TCC AGC CAC ATA ACC ACA AAA TCA ATG GCT GCT CCT      960
   Gly Leu Gly Ala Ser Ser His Ile Thr Thr Lys Ser Met Ala Ala Pro
           945                 950                 955
   CCA AGC TGT GGT CTG AGC CCA CTG CTG GTC CTG GTG GTA ACC GCT CTG     1008
   Pro Ser Cys Gly Leu Ser Pro Leu Leu Val Leu Val Val Thr Ala Leu
       960                 965                 970
   TCC ACC CTA TTA TCT TTA ACA GAA ACA TCA TAGCTGCATT AAAAAAATAC       1058
   Ser Thr Leu Leu Ser Leu Thr Glu Thr Ser
   975                 980
   AATATGGACA TGTAAAAAGA CAAAAACCAA GTTATCTGTT TCCTGTTCTC TTGTATAGCT   1118
   GAAATTCCAG TTTAGGAGCT CAGTTGAGAA ACAGTTCCAT TCAACTGGAA CATTTTTTTT   1178
   TTTTCCTTTT AAGAAAGCTT CTTGTGATCC TTCGGGGCTT CTGTG                   1223
(2) INFORMATION FOR SEQ ID NO:9:
   (i) SEQUENCE CHARACTERISTICS:
      (A) LENGTH: 346 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Leu Leu Glu Asp Ser Pro Tyr Glu Pro Val Asn Ser Arg Leu Ser Asp
   1               5                   10                  15
   Ile Phe Arg Val Val Pro Phe Ile Ser Val Glu His Ile Pro Lys Gly
               20                  25                  30
   Asn Asn Cys Leu Asp Ala Ala Lys Ala Cys Asn Leu Asp Asp Ile Cys
           35                  40                  45
   Lys Lys Tyr Arg Ser Ala Tyr Ile Thr Pro Cys Thr Thr Ser Val Ser
       50                  55                  60
   Asn Asp Val Cys Asn Arg Arg Lys Cys His Lys Ala Leu Arg Gln Phe
   65                  70                  75                  80
   Phe Asp Lys Val Pro Ala Lys His Ser Tyr Gly Met Leu Phe Cys Ser
                   85                  90                  95
   Cys Arg Asp Ile Ala Cys Thr Glu Arg Arg Arg Gln Thr Ile Val Pro
               100                 105                 110
   Val Cys Ser Tyr Glu Glu Arg Glu Lys Pro Asn Cys Leu Asn Leu Gln
           115                 120                 125
   Asp Ser Cys Lys Thr Asn Tyr Ile Cys Arg Ser Arg Leu Ala Asp Phe
       130                 135                 140
   Phe Thr Asn Cys Gln Pro Glu Ser Arg Ser Val Ser Ser Cys Leu Lys
   145                 150                 155                 160
   Glu Asn Tyr Ala Asp Cys Leu Leu Ala Tyr Ser Gly Leu Ile Gly Thr
                   165                 170                 175
   Val Met Thr Pro Asn Tyr Ile Asp Ser Ser Ser Leu Ser Val Ala Pro
               180                 185                 190
   Trp Cys Asp Cys Ser Asn Ser Gly Asn Asp Leu Glu Glu Cys Leu Lys
           195                 200                 205
   Phe Leu Asn Phe Phe Lys Asp Asn Thr Cys Leu Lys Asn Ala Ile Gln
       210                 215                 220
   Ala Phe Gly Asn Gly Ser Asp Val Thr Val Trp Gln Pro Ala Phe Pro
   225                 230                 235                 240
   Val Gln Thr Thr Thr Ala Thr Thr Thr Thr Ala Leu Arg Val Lys Asn
                   245                 250                 255
   Lys Pro Leu Gly Pro Ala Gly Ser Glu Asn Glu Ile Pro Thr His Val
               260                 265                 270
   Leu Pro Pro Cys Ala Asn Leu Gln Ala Gln Lys Leu Lys Ser Asn Val
           275                 280                 285
   Ser Gly Asn Thr His Leu Cys Ile Ser Asn Gly Asn Tyr Glu Lys Glu
       290                 295                 300
   Gly Leu Gly Ala Ser Ser His Ile Thr Thr Lys Ser Met Ala Ala Pro
   305                 310                 315                 320
   Pro Ser Cys Gly Leu Ser Pro Leu Leu Val Leu Val Val Thr Ala Leu
                   325                 330                 335
   Ser Thr Leu Leu Ser Leu Thr Glu Thr Ser
               340                 345
(2) INFORMATION FOR SEQ ID NO:10:
   (i) SEQUENCE CHARACTERISTICS:
      (A) LENGTH: 1682 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 118..1497
   (xi) SEQUENCE DESCRIPTION: SEQ ID NO:10:
   GGGCGGCCAG AGCAGCACAG CTGTCCGGGG ATCGCTGCAT GCTGAGCTCC CTCGGCAAGA       60
   CCCAGCGGCG GCTCGGGATT TTTTTGGGGG GGCGGGGACC AGCCCCGCGC CGGCACC         117
   ATG TTC CTG GCG ACC CTG TAC TTC GCG CTG CCG CTC TTG GAC TTG CTC        165
   Met Phe Leu Ala Thr Leu Tyr Phe Ala Leu Pro Leu Leu Asp Leu Leu
               350                 355                 360
   CTG TCG GCC GAA GTG AGC GGC GGA GAC CGC CTG GAT TGC GTG AAA GCC        213
   Leu Ser Ala Glu Val Ser Gly Gly Asp Arg Leu Asp Cys Val Lys Ala
           365                 370                 375
   AGT GAT CAG TGC CTG AAG GAG CAG AGC TGC AGC ACC AAG TAC CGC ACG        261
   Ser Asp Gln Cys Leu Lys Glu Gln Ser Cys Ser Thr Lys Tyr Arg Thr
       380                 385                 390
   CTA AGG CAG TGC GTG GCG GGC AAG GAG ACC AAC TTC AGC CTG GCA TCC        309
   Leu Arg Gln Cys Val Ala Gly Lys Glu Thr Asn Phe Ser Leu Ala Ser
   395                 400                 405                 410
   GGC CTG GAG GCC AAG GAT GAG TGC CGC AGC GCC ATG GAG GCC CTG AAG        357
   Gly Leu Glu Ala Lys Asp Glu Cys Arg Ser Ala Met Glu Ala Leu Lys
                   415                 420                 425
   CAG AAG TCG CTC TAC AAC TGC CGC TGC AAG CGG GGT ATG AAG AAG GAG        405
   Gln Lys Ser Leu Tyr Asn Cys Arg Cys Lys Arg Gly Met Lys Lys Glu
               430                 435                 440
   AAG AAC TGC CTG CGC ATT TAC TGG AGC ATG TAC CAG AGC CTG CAG GGA        453
   Lys Asn Cys Leu Arg Ile Tyr Trp Ser Met Tyr Gln Ser Leu Gln Gly
           445                 450                 455
   AAT GAT CTG CTG GAG GAT TCC CCA TAT GAA CCA GTT AAC AGC AGA TTG        501
   Asn Asp Leu Leu Glu Asp Ser Pro Tyr Glu Pro Val Asn Ser Arg Leu
       460                 465                 470
   TCA GAT ATA TTC CGG GTG GTC CCA TTC ATA TCA GTG GAG CAC ATT CCC        549
   Ser Asp Ile Phe Arg Val Val Pro Phe Ile Ser Val Glu His Ile Pro
   475                 480                 485                 490
   AAA GGG AAC AAC TGC CTG GAT GCA GCG AAG GCC TGC AAC CTC GAC GAC        597
   Lys Gly Asn Asn Cys Leu Asp Ala Ala Lys Ala Cys Asn Leu Asp Asp
                   495                 500                 505
   ATT TGC AAG AAG TAC AGG TCG GCG TAC ATC ACC CCG TGC ACC ACC AGC        645
   Ile Cys Lys Lys Tyr Arg Ser Ala Tyr Ile Thr Pro Cys Thr Thr Ser
               510                 515                 520
   GTG TCC AAC GAT GTC TGC AAC CGC CGC AAG TGC CAC AAG GCC CTC CGG     693
   Val Ser Asn Asp Val Cys Asn Arg Arg Lys Cys His Lys Ala Leu Arg
           525                 530                 535
   CAG TTC TTT GAC AAG GTC CCG GCC AAG CAC AGC TAC GGA ATG CTC TTC     741
   Gln Phe Phe Asp Lys Val Pro Ala Lys His Ser Tyr Gly Met Leu Phe
       540                 545                 550
   TGC TCC TGC CGG GAC ATC GCC TGC ACA GAG CGG AGG CGA CAG ACC ATC     789
   Cys Ser Cys Arg Asp Ile Ala Cys Thr Glu Arg Arg Arg Gln Thr Ile
   555                 560                 565                 570
   GTG CCT GTG TGC TCC TAT GAA GAG AGG GAG AAG CCC AAC TGT TTG AAT     837
   Val Pro Val Cys Ser Tyr Glu Glu Arg Glu Lys Pro Asn Cys Leu Asn
                   575                 580                 585
   TTG CAG GAC TCC TGC AAG ACG AAT TAC ATC TGC AGA TCT CGC CTT GCG     885
   Leu Gln Asp Ser Cys Lys Thr Asn Tyr Ile Cys Arg Ser Arg Leu Ala
               590                 595                 600
   GAT TTT TTT ACC AAC TGC CAG CCA GAG TCA AGG TCT GTC AGC AGC TGT     933
   Asp Phe Phe Thr Asn Cys Gln Pro Glu Ser Arg Ser Val Ser Ser Cys
           605                 610                 615
   CTA AAG GAA AAC TAC GCT GAC TGC CTC CTC GCC TAC TCG GGG CTT ATT     981
   Leu Lys Glu Asn Tyr Ala Asp Cys Leu Leu Ala Tyr Ser Gly Leu Ile
       620                 625                 630
   GGC ACA GTC ATG ACC CCC AAC TAC ATA GAC TCC AGT AGC CTC AGT GTG    1029
   Gly Thr Val Met Thr Pro Asn Tyr Ile Asp Ser Ser Ser Leu Ser Val
   635                 640                 645                 650
   GCC CCA TGG TGT GAC TGC AGC AAC AGT GGG AAC GAC CTA GAA GAG TGC    1077
   Ala Pro Trp Cys Asp Cys Ser Asn Ser Gly Asn Asp Leu Glu Glu Cys
                   655                 660                 665
   TTG AAA TTT TTG AAT TTC TTC AAG GAC AAT ACA TGT CTT AAA AAT GCA    1125
   Leu Lys Phe Leu Asn Phe Phe Lys Asp Asn Thr Cys Leu Lys Asn Ala
               670                 675                 680
   ATT CAA GCC TTT GGC AAT GGC TCC GAT GTG ACC GTG TGG CAG CCA GCC    1173
   Ile Gln Ala Phe Gly Asn Gly Ser Asp Val Thr Val Trp Gln Pro Ala
           685                 690                 695
   TTC CCA GTA CAG ACC ACC ACT GCC ACT ACC ACC ACT GCC CTC CGG GTT    1221
   Phe Pro Val Gln Thr Thr Thr Ala Thr Thr Thr Thr Ala Leu Arg Val
       700                 705                 710
   AAG AAC AAG CCC CTG GGG CCA GCA GGG TCT GAG AAT GAA ATT CCC ACT    1269
   Lys Asn Lys Pro Leu Gly Pro Ala Gly Ser Glu Asn Glu Ile Pro Thr
   715                 720                 725                 730
   CAT GTT TTG CCA CCG TGT GCA AAT TTA CAG GCA CAG AAG CTG AAA TCC    1317
   His Val Leu Pro Pro Cys Ala Asn Leu Gln Ala Gln Lys Leu Lys Ser
                   735                 740                 745
   AAT GTG TCG GGC AAT ACA CAC CTC TGT ATT TCC AAT GGT AAT TAT GAA      1365
   Asn Val Ser Gly Asn Thr His Leu Cys Ile Ser Asn Gly Asn Tyr Glu
               750                 755                 760
   AAA GAA GGT CTC GGT GCT TCC AGC CAC ATA ACC ACA AAA TCA ATG GCT      1413
   Lys Glu Gly Leu Gly Ala Ser Ser His Ile Thr Thr Lys Ser Met Ala
           765                 770                 775
   GCT CCT CCA AGC TGT GGT CTG AGC CCA CTG CTG GTC CTG GTG GTA ACC      1461
   Ala Pro Pro Ser Cys Gly Leu Ser Pro Leu Leu Val Leu Val Val Thr
       780                 785                 790
   GCT CTG TCC ACC CTA TTA TCT TTA ACA GAA ACA TCA TAGCTGCATT           1507
   Ala Leu Ser Thr Leu Leu Ser Leu Thr Glu Thr Ser
   795                 800                 805
   AAAAAAATAC AATATGGACA TGTAAAAAGA CAAAAACCAA GTTATCTGTT TCCTGTTCTC    1567
   TTGTATAGCT GAAATTCCAG TTTAGGAGCT CAGTTGAGAA ACAGTTCCAT TCAACTGGAA    1627
   CATTTTTTTT TTTTCCTTTT AAGAAAGCTT CTTGTGATCC TTCGGGGCTT CTGTG         1682
(2) INFORMATION FOR SEQ ID NO:11:
   (i) SEQUENCE CHARACTERISTICS:
      (A) LENGTH: 460 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Phe Leu Ala Thr Leu Tyr Phe Ala Leu Pro Leu Leu Asp Leu Leu
    1               5                  10                  15
   Leu Ser Ala Glu Val Ser Gly Gly Asp Arg Leu Asp Cys Val Lys Ala
               20                  25                  30
   Ser Asp Gln Cys Leu Lys Glu Gln Ser Cys Ser Thr Lys Tyr Arg Thr
           35                  40                  45
   Leu Arg Gln Cys Val Ala Gly Lys Glu Thr Asn Phe Ser Leu Ala Ser
       50                  55                  60
   Gly Leu Glu Ala Lys Asp Glu Cys Arg Ser Ala Met Glu Ala Leu Lys
   65                  70                  75                  80
   Gln Lys Ser Leu Tyr Asn Cys Arg Cys Lys Arg Gly Met Lys Lys Glu
                   85                  90                  95
   Lys Asn Cys Leu Arg Ile Tyr Trp Ser Met Tyr Gln Ser Leu Gln Gly
               100                 105                 110
   Asn Asp Leu Leu Glu Asp Ser Pro Tyr Glu Pro Val Asn Ser Arg Leu
   115 120 125
   Ser Asp Ile Phe Arg Val Val Pro Phe Ile Ser Val Glu His Ile Pro
   130 135 140
   Lys Gly Asn Asn Cys Leu Asp Ala Ala Lys Ala Cys Asn Leu Asp Asp
   145 150 155 160
   Ile Cys Lys Lys Tyr Arg Ser Ala Tyr Ile Thr Pro Cys Thr Thr Ser
   165 170 175
   Val Ser Asn Asp Val Cys Asn Arg Arg Lys Cys His Lys Ala Leu Arg
   180 185 190
   Gln Phe Phe Asp Lys Val Pro Ala Lys His Ser Tyr Gly Met Leu Phe
   195 200 205
   Cys Ser Cys Arg Asp Ile Ala Cys Thr Glu Arg Arg Arg Gln Thr Ile
   210 215 220
   Val Pro Val Cys Ser Tyr Glu Glu Arg Glu Lys Pro Asn Cys Leu Asn
   225 230 235 240
   Leu Gln Asp Ser Cys Lys Thr Asn Tyr Ile Cys Arg Ser Arg Leu Ala
   245 250 255
   Asp Phe Phe Thr Asn Cys Gln Pro Glu Ser Arg Ser Val Ser Ser Cys
   260 265 270
   Leu Lys Glu Asn Tyr Ala Asp Cys Leu Leu Ala Tyr Ser Gly Leu Ile
   275 280 285
   Gly Thr Val Met Thr Pro Asn Tyr Ile Asp Ser Ser Ser Leu Ser Val
   290 295 300
   Ala Pro Trp Cys Asp Cys Ser Asn Ser Gly Asn Asp Leu Glu Glu Cys
   305 310 315 320
   Leu Lys Phe Leu Asn Phe Phe Lys Asp Asn Thr Cys Leu Lys Asn Ala
   325 330 335
   Ile Gln Ala Phe Gly Asn Gly Ser Asp Val Thr Val Trp Gln Pro Ala
   340 345 350
   Phe Pro Val Gln Thr Thr Thr Ala Thr Thr Thr Thr Ala Leu Arg Val
   355 360 365
   Lys Asn Lys Pro Leu Gly Pro Ala Gly Ser Glu Asn Glu Ile Pro Thr
   370 375 380
   His Val Leu Pro Pro Cys Ala Asn Leu Gln Ala Gln Lys Leu Lys Ser
   385 390 395 400
   Asn Val Ser Gly Asn Thr His Leu Cys Ile Ser Asn Gly Asn Tyr Glu
                   405                 410                 415
   Lys Glu Gly Leu Gly Ala Ser Ser His Ile Thr Thr Lys Ser Met Ala
               420                 425                 430
   Ala Pro Pro Ser Cys Gly Leu Ser Pro Leu Leu Val Leu Val Val Thr
           435                 440                 445
   Ala Leu Ser Thr Leu Leu Ser Leu Thr Glu Thr Ser
       450                 455                 460
(2) INFORMATION FOR SEQ ID NO:12:
   (i) SEQUENCE CHARACTERISTICS:
      (A) LENGTH: 1888 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 25..1416
   (xi) SEQUENCE DESCRIPTION: SEQ ID NO:12:
   AAAAAACGGT GGGATTTATT TAAC ATG ATC TTG GCA AAC GTC TTC TGC CTC         51
                              Met Ile Leu Ala Asn Val Phe Cys Leu
                                                  465
   TTC TTC TTT CTA GAC GAG ACC CTC CGC TCT TTG GCC AGC CCT TCC TCC        99
   Phe Phe Phe Leu Asp Glu Thr Leu Arg Ser Leu Ala Ser Pro Ser Ser
   470                 475                 480                 485
   CTG CAG GGC CCC GAG CTC CAC GGC TGG CGC CCC CCA GTG GAC TGT GTC       147
   Leu Gln Gly Pro Glu Leu His Gly Trp Arg Pro Pro Val Asp Cys Val
                   490                 495                 500
   CGG GCC AAT GAG CTG TGT GCC GCC GAA TCC AAC TGC AGC TCT CGC TAC       195
   Arg Ala Asn Glu Leu Cys Ala Ala Glu Ser Asn Cys Ser Ser Arg Tyr
               505                 510                 515
   CGC ACT CTG CGG CAG TGC CTG GCA GGC CGC GAC CGC AAC ACC ATG CTG       243
   Arg Thr Leu Arg Gln Cys Leu Ala Gly Arg Asp Arg Asn Thr Met Leu
           520                 525                 530
   GCC AAC AAG GAG TGC CAG GCG GCC TTG GAG GTC TTG CAG GAG AGC CCG       291
   Ala Asn Lys Glu Cys Gln Ala Ala Leu Glu Val Leu Gln Glu Ser Pro
       535                 540                 545
   CTG TAC GAC TGC CGC TGC AAG CGG GGC ATG AAG AAG GAG CTG CAG TGT        339
   Leu Tyr Asp Cys Arg Cys Lys Arg Gly Met Lys Lys Glu Leu Gln Cys
   550                 555                 560                 565
   CTG CAG ATC TAC TGG AGC ATC CAC CTG GGG CTG ACC GAG GGT GAG GAG        387
   Leu Gln Ile Tyr Trp Ser Ile His Leu Gly Leu Thr Glu Gly Glu Glu
                   570                 575                 580
   TTC TAC GAA GCC TCC CCC TAT GAG CCG GTG ACC TCC CGC CTC TCG GAC        435
   Phe Tyr Glu Ala Ser Pro Tyr Glu Pro Val Thr Ser Arg Leu Ser Asp
               585                 590                 595
   ATC TTC AGG CTT GCT TCA ATC TTC TCA GGG ACA GGG GCA GAC CCG GTG        483
   Ile Phe Arg Leu Ala Ser Ile Phe Ser Gly Thr Gly Ala Asp Pro Val
           600                 605                 610
   GTC AGC GCC AAG AGC AAC CAT TGC CTG GAT GCT GCC AAG GCC TGC AAC        531
   Val Ser Ala Lys Ser Asn His Cys Leu Asp Ala Ala Lys Ala Cys Asn
       615                 620                 625
   CTG AAT GAC AAC TGC AAG AAG CTG CGC TCC TCC TAC ATC TCC ATC TGC        579
   Leu Asn Asp Asn Cys Lys Lys Leu Arg Ser Ser Tyr Ile Ser Ile Cys
   630                 635                 640                 645
   AAC CGC GAG ATC TCG CCC ACC GAG CGC TGC AAC CGC CGC AAG TGC CAC        627
   Asn Arg Glu Ile Ser Pro Thr Glu Arg Cys Asn Arg Arg Lys Cys His
                   650                 655                 660
   AAG GCC CTG CGC CAG TTC TTC GAC CGG GTG CCC AGC GAG TAC ACC TAC        675
   Lys Ala Leu Arg Gln Phe Phe Asp Arg Val Pro Ser Glu Tyr Thr Tyr
               665                 670                 675
   CGC ATG CTC TTC TGC TCC TGC CAA GAC CAG GCG TGC GCT GAG CGC CGC        723
   Arg Met Leu Phe Cys Ser Cys Gln Asp Gln Ala Cys Ala Glu Arg Arg
           680                 685                 690
   CGG CAA ACC ATC CTG CCC AGC TGC TCC TAT GAG GAC AAG GAG AAG CCC        771
   Arg Gln Thr Ile Leu Pro Ser Cys Ser Tyr Glu Asp Lys Glu Lys Pro
       695                 700                 705
   AAC TGC CTG GAC CTG CGT GGC GTG TGC CGG ACT GAC CAC CTG TGT CGG        819
   Asn Cys Leu Asp Leu Arg Gly Val Cys Arg Thr Asp His Leu Cys Arg
   710                 715                 720                 725
   TCC CGG CTG GCC GAC TTC CAT GCC AAT TGT CGA GCC TCC TAC CAG ACG        867
   Ser Arg Leu Ala Asp Phe His Ala Asn Cys Arg Ala Ser Tyr Gln Thr
                   730                 735                 740
   GTC ACC AGC TGC CCT GCG GAC AAT TAC CAG GCG TGT CTG GGC TCT TAT        915
   Val Thr Ser Cys Pro Ala Asp Asn Tyr Gln Ala Cys Leu Gly Ser Tyr
               745                 750                 755
   GCT GGC ATG ATT GGG TTT GAC ATG ACA CCT AAC TAT GTG GAC TCC AGC        963
   Ala Gly Met Ile Gly Phe Asp Met Thr Pro Asn Tyr Val Asp Ser Ser
           760                 765                 770
   CCC ACT GGC ATC GTG GTG TCC CCC TGG TGC AGC TGT CGT GGC AGC GGG      1011
   Pro Thr Gly Ile Val Val Ser Pro Trp Cys Ser Cys Arg Gly Ser Gly
       775                 780                 785
   AAC ATG GAG GAG GAG TGT GAG AAG TTC CTC AGG GAC TTC ACC GAG AAC      1059
   Asn Met Glu Glu Glu Cys Glu Lys Phe Leu Arg Asp Phe Thr Glu Asn
   790                 795                 800                 805
   CCA TGC CTC CGG AAC GCC ATC CAG GCC TTT GGC AAC GGC ACG GAC GTG      1107
   Pro Cys Leu Arg Asn Ala Ile Gln Ala Phe Gly Asn Gly Thr Asp Val
                   810                 815                 820
   AAC GTG TCC CCA AAA GGC CCC TCG TTC CAG GCC ACC CAG GCC CCT CGG      1155
   Asn Val Ser Pro Lys Gly Pro Ser Phe Gln Ala Thr Gln Ala Pro Arg
               825                 830                 835
   GTG GAG AAG ACG CCT TCT TTG CCA GAT GAC CTC AGT GAC AGT ACC AGC      1203
   Val Glu Lys Thr Pro Ser Leu Pro Asp Asp Leu Ser Asp Ser Thr Ser
           840                 845                 850
   TTG GGG ACC AGT GTC ATC ACC ACC TGC ACG TCT GTC CAG GAG CAG GGG      1251
   Leu Gly Thr Ser Val Ile Thr Thr Cys Thr Ser Val Gln Glu Gln Gly
       855                 860                 865
   CTG AAG GCC AAC AAC TCC AAA GAG TTA AGC ATG TGC TTC ACA GAG CTC      1299
   Leu Lys Ala Asn Asn Ser Lys Glu Leu Ser Met Cys Phe Thr Glu Leu
   870                 875                 880                 885
   ACG ACA AAT ATC ATC CCA GGG AGT AAC AAG GTG ATC AAA CCT AAC TCA      1347
   Thr Thr Asn Ile Ile Pro Gly Ser Asn Lys Val Ile Lys Pro Asn Ser
                   890                 895                 900
   GGC CCC AGC AGA GCC AGA CCG TCG GCT GCC TTG ACC GTG CTG TCT GTC      1395
   Gly Pro Ser Arg Ala Arg Pro Ser Ala Ala Leu Thr Val Leu Ser Val
               905                 910                 915
   CTG ATG CTG AAA CTG GCC TTG TAGGCTGTGG GAACCGAGTC AGAAGATTTT         1446
   Leu Met Leu Lys Leu Ala Leu
           920
   TGAAAGCTAC GCAGACAAGA ACAGCCGCCT GACGAAATGG AAACACACAC AGACACACAC    1506
   ACACCTTGCA AAAAAAAAAT TGTTTTTCCC ACCTTGTCGC TGAACCTGTC TCCTCCCAGG    1566
   TTTCTTCTCT GGAGAAGTTT TTGTAAACCA AACAGACAAG CAGGCAGGCA GCCTGAGAGC    1626
   TGGCCCAGGG GTCCCCTGGC AGGGGAAACT CTGGTGCCGG GGAGGGCACG AGGCTCTAGA    1686
   AATGCCCTTC ACTTTCTCCT GGTGTTTTTC TCTCTGGACC CTTCTGAAGC AGAGACCGGA    1746
   CAAGAGCCTG CAGCGGAAGG GACTCTGGGC TGTGCCTGAG GCTGGCTGGG GGCAGGACAA    1806
   CACAGCTGCT TCCCCAGGCT GCCCACTCTG GGGACCCGCT GGGGGCTGGC AGAGGGCATC    1866
   GGTCAGCGGG GCAGCGGGGC TG                                             1888
(2) INFORMATION FOR SEQ ID NO:13:
   (i) SEQUENCE CHARACTERISTICS:
      (A) LENGTH: 464 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Ile Leu Ala Asn Val Phe Cys Leu Phe Phe Phe Leu Asp Glu Thr
    1               5                  10                  15
   Leu Arg Ser Leu Ala Ser Pro Ser Ser Leu Gln Gly Pro Glu Leu His
               20                  25                  30
   Gly Trp Arg Pro Pro Val Asp Cys Val Arg Ala Asn Glu Leu Cys Ala
           35                  40                  45
   Ala Glu Ser Asn Cys Ser Ser Arg Tyr Arg Thr Leu Arg Gln Cys Leu
       50                  55                  60
   Ala Gly Arg Asp Arg Asn Thr Met Leu Ala Asn Lys Glu Cys Gln Ala
   65                  70                  75                  80
   Ala Leu Glu Val Leu Gln Glu Ser Pro Leu Tyr Asp Cys Arg Cys Lys
                   85                  90                  95
   Arg Gly Met Lys Lys Glu Leu Gln Cys Leu Gln Ile Tyr Trp Ser Ile
               100                 105                 110
   His Leu Gly Leu Thr Glu Gly Glu Glu Phe Tyr Glu Ala Ser Pro Tyr
           115                 120                 125
   Glu Pro Val Thr Ser Arg Leu Ser Asp Ile Phe Arg Leu Ala Ser Ile
       130                 135                 140
   Phe Ser Gly Thr Gly Ala Asp Pro Val Val Ser Ala Lys Ser Asn His
   145                 150                 155                 160
   Cys Leu Asp Ala Ala Lys Ala Cys Asn Leu Asn Asp Asn Cys Lys Lys
                   165                 170                 175
   Leu Arg Ser Ser Tyr Ile Ser Ile Cys Asn Arg Glu Ile Ser Pro Thr
               180                 185                 190
   Glu Arg Cys Asn Arg Arg Lys Cys His Lys Ala Leu Arg Gln Phe Phe
           195                 200                 205
   Asp Arg Val Pro Ser Glu Tyr Thr Tyr Arg Met Leu Phe Cys Ser Cys
       210                 215                 220
   Gln Asp Gln Ala Cys Ala Glu Arg Arg Arg Gln Thr Ile Leu Pro Ser
   225                 230                 235                 240
   Cys Ser Tyr Glu Asp Lys Glu Lys Pro Asn Cys Leu Asp Leu Arg Gly
                   245                 250                 255
   Val Cys Arg Thr Asp His Leu Cys Arg Ser Arg Leu Ala Asp Phe His
               260                 265                 270
   Ala Asn Cys Arg Ala Ser Tyr Gln Thr Val Thr Ser Cys Pro Ala Asp
           275                 280                 285
   Asn Tyr Gln Ala Cys Leu Gly Ser Tyr Ala Gly Met Ile Gly Phe Asp
       290                 295                 300
   Met Thr Pro Asn Tyr Val Asp Ser Ser Pro Thr Gly Ile Val Val Ser
   305                 310                 315                 320
   Pro Trp Cys Ser Cys Arg Gly Ser Gly Asn Met Glu Glu Glu Cys Glu
                   325                 330                 335
   Lys Phe Leu Arg Asp Phe Thr Glu Asn Pro Cys Leu Arg Asn Ala Ile
               340                 345                 350
   Gln Ala Phe Gly Asn Gly Thr Asp Val Asn Val Ser Pro Lys Gly Pro
           355                 360                 365
   Ser Phe Gln Ala Thr Gln Ala Pro Arg Val Glu Lys Thr Pro Ser Leu
       370                 375                 380
   Pro Asp Asp Leu Ser Asp Ser Thr Ser Leu Gly Thr Ser Val Ile Thr
   385                 390                 395                 400
   Thr Cys Thr Ser Val Gln Glu Gln Gly Leu Lys Ala Asn Asn Ser Lys
                   405                 410                 415
   Glu Leu Ser Met Cys Phe Thr Glu Leu Thr Thr Asn Ile Ile Pro Gly
               420                 425                 430
   Ser Asn Lys Val Ile Lys Pro Asn Ser Gly Pro Ser Arg Ala Arg Pro
           435                 440                 445
   Ser Ala Ala Leu Thr Val Leu Ser Val Leu Met Leu Lys Leu Ala Leu
       450                 455                 460
(2) INFORMATION FOR SEQ ID NO:14:
   (i) SEQUENCE CHARACTERISTICS:
      (A) LENGTH: 1878 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 205..1242
   (xi) SEQUENCE DESCRIPTION: SEQ ID NO:14:
   CGCGGCGCCC AGCGCAGGCA GAGCGCTGTC GCATCCCGGG CGTCCACCCG CCATGGGGCT    60
   CTCCTGGAGC CCGCGACCTC CACTGCTGAT GATCCTGCTA CTGGTGCTGT CGTTGTGGCT   120
   GCCACTTGGA GCAGGAAACT CCCTTGCCAC AGAGAACAGG TTTGTGAACA GCTGTACCCA   180
   GGCCAGAAAG AAATGCGAGG CTAA TCC CGC TTG CAA GGC TGC CTA CCA GCA      231
                              Ser Arg Leu Gln Gly Cys Leu Pro Ala
                              465                 470
   CCT GGG CTC CTG CAC CTC CAG TTA AGC AGG CCG CTG CCC TTA GAG GAG    279
   Pro Gly Leu Leu His Leu Gln Leu Ser Arg Pro Leu Pro Leu Glu Glu
       475                 480                 485
   TCT GCC ATG TCT GCA GAC TGC CTA GAG GCA GCA GAA CAA CTC AGG AAC    327
   Ser Ala Met Ser Ala Asp Cys Leu Glu Ala Ala Glu Gln Leu Arg Asn
   490                 495                 500                 505
   AGC TCT CTG ATA GAC TGC AGG TGC CAT CGG CGC ATG AAG CAC CAA GCT    375
   Ser Ser Leu Ile Asp Cys Arg Cys His Arg Arg Met Lys His Gln Ala
                   510                 515                 520
   ACC TGT CTG GAC ATT TAT TGG ACC GTT CAC CCT GCC CGA AGC CTT GGT    423
   Thr Cys Leu Asp Ile Tyr Trp Thr Val His Pro Ala Arg Ser Leu Gly
               525                 530                 535
   GAC TAC GAG TTG GAT GTC TCA CCC TAT GAA GAC ACA GTG ACC AGC AAA    471
   Asp Tyr Glu Leu Asp Val Ser Pro Tyr Glu Asp Thr Val Thr Ser Lys
           540                 545                 550
   CCC TGG AAA ATG AAT CTT AGC AAG TTG AAC ATG CTC AAA CCA GAC TCG    519
   Pro Trp Lys Met Asn Leu Ser Lys Leu Asn Met Leu Lys Pro Asp Ser
       555                 560                 565
   GAC CTC TGC CTC AAA TTT GCT ATG CTG TGT ACT CTT CAC GAC AAG TGT    567
   Asp Leu Cys Leu Lys Phe Ala Met Leu Cys Thr Leu His Asp Lys Cys
   570                 575                 580                 585
   GAC CGC CTG CGC AAG GCC TAC GGG GAG GCA TGC TCA GGG ATC CGC TGC    615
   Asp Arg Leu Arg Lys Ala Tyr Gly Glu Ala Cys Ser Gly Ile Arg Cys
                   590                 595                 600
   CAG CGC CAC CTC TGC CTA GCC CAG CTG CGC TCC TTC TTT GAG AAG GCA    663
   Gln Arg His Leu Cys Leu Ala Gln Leu Arg Ser Phe Phe Glu Lys Ala
               605                 610                 615
   GCA GAG TCC CAC GCT CAG GGT CTG CTG CTG TGT CCC TGT GCA CCA GAA        711
   Ala Glu Ser His Ala Gln Gly Leu Leu Leu Cys Pro Cys Ala Pro Glu
           620                 625                 630
   GAT GCG GGC TGT GGG GAG CGG CGG CGT AAC ACC ATC GCC CCC AGT TGC        759
   Asp Ala Gly Cys Gly Glu Arg Arg Arg Asn Thr Ile Ala Pro Ser Cys
       635                 640                 645
   GCC CTG CCT TCT GTA ACC CCC AAT TGC CTG GAT CTG CGG AGC TTC TGC        807
   Ala Leu Pro Ser Val Thr Pro Asn Cys Leu Asp Leu Arg Ser Phe Cys
   650                 655                 660                 665
   CGT GCG GAC CCT TTG TGC AGA TCA CGC CTG ATG GAC TTC CAG ACC CAC        855
   Arg Ala Asp Pro Leu Cys Arg Ser Arg Leu Met Asp Phe Gln Thr His
                   670                 675                 680
   TGT CAT CCT ATG GAC ATC CTT GGG ACT TGT GCA ACT GAG CAG TCC AGA        903
   Cys His Pro Met Asp Ile Leu Gly Thr Cys Ala Thr Glu Gln Ser Arg
               685                 690                 695
   TGT CTG CGG GCA TAC CTG GGG CTG ATT GGG ACT GCC ATG ACC CCA AAC        951
   Cys Leu Arg Ala Tyr Leu Gly Leu Ile Gly Thr Ala Met Thr Pro Asn
           700                 705                 710
   TTC ATC AGC AAG GTC AAC ACT ACT GTT GCC TTA AGC TGC ACC TGC CGA        999
   Phe Ile Ser Lys Val Asn Thr Thr Val Ala Leu Ser Cys Thr Cys Arg
       715                 720                 725
   GGC AGC GGC AAC CTA CAG GAC GAG TGT GAA CAG CTG GAA AGG TCC TTC       1047
   Gly Ser Gly Asn Leu Gln Asp Glu Cys Glu Gln Leu Glu Arg Ser Phe
   730                 735                 740                 745
   TCC CAG AAC CCC TGC CTC GTG GAG GCC ATT GCA GCT AAG ATG CGT TTC       1095
   Ser Gln Asn Pro Cys Leu Val Glu Ala Ile Ala Ala Lys Met Arg Phe
                   750                 755                 760
   CAC AGA CAG CTC TTC TCC CAG GAC TGG GCA GAC TCT ACT TTT TCA GTG       1143
   His Arg Gln Leu Phe Ser Gln Asp Trp Ala Asp Ser Thr Phe Ser Val
               765                 770                 775
   GTG CAG CAG CAG AAC AGC AAC CCT GCT CTG AGA CTG CAG CCC AGG CTA       1191
   Val Gln Gln Gln Asn Ser Asn Pro Ala Leu Arg Leu Gln Pro Arg Leu
           780                 785                 790
   CCC ATT CTT TCT TTC TCC ATC CTT CCC TTG ATT CTG CTG CAG ACC CTC       1239
   Pro Ile Leu Ser Phe Ser Ile Leu Pro Leu Ile Leu Leu Gln Thr Leu
       795                 800                 805
   TGG TAGCTGGGCT TCCTCAGGGT CCTTTGTCCT CTCCACCACA CCCAGACTGA            1292
   Trp
   810
   TTTGCAGCCT GTGGTGGGAG AGAACTCGCC AGCCTGTGGA AGAAGACGCA GCGTGCTACA     1352
   CAGCAACCCG GAACCAACCA GGCATTCCGC AGCACATCCC GTCTGCTCCA GAAGAGGTCT 1412
   TAGAAGTGAG GGCTGTGACC CTTCCGATCC TGAGCGGCTA GTTTTCAAAC CTCCCTTGCC 1472
   CCTGCTTCCT TCTGGCTCAG GCTGCTCCTC CTTAGGACTT TGTGGGTCCA GTTTTGCCTT 1532
   CTGTTCTGAT GGTGATTAGC GGCTCACCTC CAGCGCTTCT TCCTGTTTCC CAGGACCACC 1592
   CAGAGGCTAA GGAATCAGTC ATTCCCTGTT GCCTTCTCCA GGAAGGCAGG CTAAGGGTTC 1652
   TGAGGTGACT GAGAAAAATG TTTCCTTTGT GTGGAAGGCT GGTGCTCCAG CCTCCACGTC 1712
   CCTCTGAATG GAAGATAAAA ACCTGCTGGT GTCTTGACTG CTCTGCCAGG CAATCCTGAA 1772
   CATTTGGGCA TGAAGAGCTA AAGTCTTTGG GTCTTGTTTA ACTCCTATTA CTGTCCCCAA 1832
   ATTCCCCTAG TCCCTTGGGT CATGATTAAA CATTTTGACT TAAAAA 1878
(2) INFORMATION FOR SEQ ID NO:15:
   (i) SEQUENCE CHARACTERISTICS:
      (A) LENGTH: 346 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Ser Arg Leu Gln Gly Cys Leu Pro Ala Pro Gly Leu Leu His Leu Gln
   1 5 10 15
   Leu Ser Arg Pro Leu Pro Leu Glu Glu Ser Ala Met Ser Ala Asp Cys
   20 25 30
   Leu Glu Ala Ala Glu Gln Leu Arg Asn Ser Ser Leu Ile Asp Cys Arg
   35 40 45
   Cys His Arg Arg Met Lys His Gln Ala Thr Cys Leu Asp Ile Tyr Trp
   50 55 60
   Thr Val His Pro Ala Arg Ser Leu Gly Asp Tyr Glu Leu Asp Val Ser
   65 70 75 80
   Pro Tyr Glu Asp Thr Val Thr Ser Lys Pro Trp Lys Met Asn Leu Ser
   85 90 95
   Lys Leu Asn Met Leu Lys Pro Asp Ser Asp Leu Cys Leu Lys Phe Ala
   100 105 110
   Met Leu Cys Thr Leu His Asp Lys Cys Asp Arg Leu Arg Lys Ala Tyr
   115 120 125
   Gly Glu Ala Cys Ser Gly Ile Arg Cys Gln Arg His Leu Cys Leu Ala
       130                 135                 140
   Gln Leu Arg Ser Phe Phe Glu Lys Ala Ala Glu Ser His Ala Gln Gly
   145                 150                 155                 160
   Leu Leu Leu Cys Pro Cys Ala Pro Glu Asp Ala Gly Cys Gly Glu Arg
                   165                 170                 175
   Arg Arg Asn Thr Ile Ala Pro Ser Cys Ala Leu Pro Ser Val Thr Pro
               180                 185                 190
   Asn Cys Leu Asp Leu Arg Ser Phe Cys Arg Ala Asp Pro Leu Cys Arg
           195                 200                 205
   Ser Arg Leu Met Asp Phe Gln Thr His Cys His Pro Met Asp Ile Leu
       210                 215                 220
   Gly Thr Cys Ala Thr Glu Gln Ser Arg Cys Leu Arg Ala Tyr Leu Gly
   225                 230                 235                 240
   Leu Ile Gly Thr Ala Met Thr Pro Asn Phe Ile Ser Lys Val Asn Thr
                   245                 250                 255
   Thr Val Ala Leu Ser Cys Thr Cys Arg Gly Ser Gly Asn Leu Gln Asp
               260                 265                 270
   Glu Cys Glu Gln Leu Glu Arg Ser Phe Ser Gln Asn Pro Cys Leu Val
           275                 280                 285
   Glu Ala Ile Ala Ala Lys Met Arg Phe His Arg Gln Leu Phe Ser Gln
       290                 295                 300
   Asp Trp Ala Asp Ser Thr Phe Ser Val Val Gln Gln Gln Asn Ser Asn
   305                 310                 315                 320
   Pro Ala Leu Arg Leu Gln Pro Arg Leu Pro Ile Leu Ser Phe Ser Ile
                   325                 330                 335
   Leu Pro Leu Ile Leu Leu Gln Thr Leu Trp
               340                 345
(2) INFORMATION FOR SEQ ID NO:16:
   (i) SEQUENCE CHARACTERISTICS:
      (A) LENGTH: 1889 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 41..1231
   (xi) SEQUENCE DESCRIPTION: SEQ ID NO:16:
   CGCAGGCAGA GCGCTGTCGC ATCCCGGGCG TCCACCCGCC ATG GGG CTC TCC TGG         55
                                           Met Gly Leu Ser Trp
                                                       350
   AGC CCG CGA CCT CCA CTG CTG ATG ATC CTG CTA CTG GTG CTG TCG TTG        103
   Ser Pro Arg Pro Pro Leu Leu Met Ile Leu Leu Leu Val Leu Ser Leu
               355                 360                 365
   TGG CTG CCA CTT GGA GCA GGA AAC TCC CTT GCC ACA GAG AAC AGG TTT        151
   Trp Leu Pro Leu Gly Ala Gly Asn Ser Leu Ala Thr Glu Asn Arg Phe
           370                 375                 380
   GTG AAC AGC TGT ACC CAG GCC AGA AAG AAA TGC GAG GCT AAT CCC GCT        199
   Val Asn Ser Cys Thr Gln Ala Arg Lys Lys Cys Glu Ala Asn Pro Ala
       385                 390                 395
   TGC AAG GCT GCC TAC CAG CAC CTG GGC TCC TGC ACC TCC AGT TTA AGC        247
   Cys Lys Ala Ala Tyr Gln His Leu Gly Ser Cys Thr Ser Ser Leu Ser
   400                 405                 410                 415
   AGG CCG CTG CCC TTA GAG GAG TCT GCC ATG TCT GCA GAC TGC CTA GAG        295
   Arg Pro Leu Pro Leu Glu Glu Ser Ala Met Ser Ala Asp Cys Leu Glu
                   420                 425                 430
   GCA GCA GAA CAA CTC AGG AAC AGC TCT CTG ATA GAC TGC AGG TGC CAT        343
   Ala Ala Glu Gln Leu Arg Asn Ser Ser Leu Ile Asp Cys Arg Cys His
               435                 440                 445
   CGG CGC ATG AAG CAC CAA GCT ACC TGT CTG GAC ATT TAT TGG ACC GTT        391
   Arg Arg Met Lys His Gln Ala Thr Cys Leu Asp Ile Tyr Trp Thr Val
           450                 455                 460
   CAC CCT GCC CGA AGC CTT GGT GAC TAC GAG TTG GAT GTC TCA CCC TAT        439
   His Pro Ala Arg Ser Leu Gly Asp Tyr Glu Leu Asp Val Ser Pro Tyr
       465                 470                 475
   GAA GAC ACA GTG ACC AGC AAA CCC TGG AAA ATG AAT CTT AGC AAG TTG        487
   Glu Asp Thr Val Thr Ser Lys Pro Trp Lys Met Asn Leu Ser Lys Leu
   480                 485                 490                 495
   AAC ATG CTC AAA CCA GAC TCG GAC CTC TGC CTC AAA TTT GCT ATG CTG        535
   Asn Met Leu Lys Pro Asp Ser Asp Leu Cys Leu Lys Phe Ala Met Leu
                   500                 505                 510
   TGT ACT CTT CAC GAC AAG TGT GAC CGC CTG CGC AAG GCC TAC GGG GAG        583
   Cys Thr Leu His Asp Lys Cys Asp Arg Leu Arg Lys Ala Tyr Gly Glu
               515                 520                 525
   GCA TGC TCA GGG ATC CGC TGC CAG CGC CAC CTC TGC CTA GCC CAG CTG       631
   Ala Cys Ser Gly Ile Arg Cys Gln Arg His Leu Cys Leu Ala Gln Leu
           530                 535                 540
   CGC TCC TTC TTT GAG AAG GCA GCA GAG TCC CAC GCT CAG GGT CTG CTG       679
   Arg Ser Phe Phe Glu Lys Ala Ala Glu Ser His Ala Gln Gly Leu Leu
       545                 550                 555
   CTG TGT CCC TGT GCA CCA GAA GAT GCG GGC TGT GGG GAG CGG CGG CGT       727
   Leu Cys Pro Cys Ala Pro Glu Asp Ala Gly Cys Gly Glu Arg Arg Arg
   560                 565                 570                 575
   AAC ACC ATC GCC CCC AGT TGC GCC CTG CCT TCT GTA ACC CCC AAT TGC       775
   Asn Thr Ile Ala Pro Ser Cys Ala Leu Pro Ser Val Thr Pro Asn Cys
                   580                 585                 590
   CTG GAT CTG CGG AGC TTC TGC CGT GCG GAC CCT TTG TGC AGA TCA CGC       823
   Leu Asp Leu Arg Ser Phe Cys Arg Ala Asp Pro Leu Cys Arg Ser Arg
               595                 600                 605
   CTG ATG GAC TTC CAG ACC CAC TGT CAT CCT ATG GAC ATC CTT GGG ACT       871
   Leu Met Asp Phe Gln Thr His Cys His Pro Met Asp Ile Leu Gly Thr
           610                 615                 620
   TGT GCA ACT GAG CAG TCC AGA TGT CTG CGG GCA TAC CTG GGG CTG ATT       919
   Cys Ala Thr Glu Gln Ser Arg Cys Leu Arg Ala Tyr Leu Gly Leu Ile
       625                 630                 635
   GGG ACT GCC ATG ACC CCA AAC TTC ATC AGC AAG GTC AAC ACT ACT GTT       967
   Gly Thr Ala Met Thr Pro Asn Phe Ile Ser Lys Val Asn Thr Thr Val
   640                 645                 650                 655
   GCC TTA AGC TGC ACC TGC CGA GGC AGC GGC AAC CTA CAG GAC GAG TGT      1015
   Ala Leu Ser Cys Thr Cys Arg Gly Ser Gly Asn Leu Gln Asp Glu Cys
                   660                 665                 670
   GAA CAG CTG GAA AGG TCC TTC TCC CAG AAC CCC TGC CTC GTG GAG GCC      1063
   Glu Gln Leu Glu Arg Ser Phe Ser Gln Asn Pro Cys Leu Val Glu Ala
               675                 680                 685
   ATT GCA GCT AAG ATG CGT TTC CAC AGA CAG CTC TTC TCC CAG GAC TGG      1111
   Ile Ala Ala Lys Met Arg Phe His Arg Gln Leu Phe Ser Gln Asp Trp
           690                 695                 700
   GCA GAC TCT ACT TTT TCA GTG GTG CAG CAG CAG AAC AGC AAC CCT GCT      1159
   Ala Asp Ser Thr Phe Ser Val Val Gln Gln Gln Asn Ser Asn Pro Ala
       705                 710                 715
   CTG AGA CTG CAG CCC AGG CTA CCC ATT CTT TCT TTC TCC ATC CTT CCC      1207
   Leu Arg Leu Gln Pro Arg Leu Pro Ile Leu Ser Phe Ser Ile Leu Pro
   720                 725                 730                 735
   TTG ATT CTG CTG CAG ACC CTC TGG TAGCTGGGCT TCCTCAGGGT CCTTTGTCCT     1261
   Leu Ile Leu Leu Gln Thr Leu Trp
                   740
   CTCCACCACA CCCAGACTGA TTTGCAGCCT GTGGTGGGAG AGAACTCGCC AGCCTGTGGA   1321
   AGAAGACGCA GCGTGCTACA CAGCAACCCG GAACCAACCA GGCATTCCGC AGCACATCCC   1381
   GTCTGCTCCA GAAGAGGTCT TAGAAGTGAG GGCTGTGACC CTTCCGATCC TGAGCGGCTA   1441
   GTTTTCAAAC CTCCCTTGCC CCTGCTTCCT TCTGGCTCAG GCTGCTCCTC CTTAGGACTT   1501
   TGTGGGTCCA GTTTTGCCTT CTGTTCTGAT GGTGATTAGC GGCTCACCTC CAGCGCTTCT   1561
   TCCTGTTTCC CAGGACCACC CAGAGGCTAA GGAATCAGTC ATTCCCTGTT GCCTTCTCCA   1621
   GGAAGGCAGG CTAAGGGTTC TGAGGTGACT GAGAAAAATG TTTCCTTTGT GTGGAAGGCT   1681
   GGTGCTCCAG CCTCCACGTC CCTCTGAATG GAAGATAAAA ACCTGCTGGT GTCTTGACTG   1741
   CTCTGCCAGG CAATCCTGAA CATTTGGGCA TGAAGAGCTA AAGTCTTTGG GTCTTGTTTA   1801
   ACTCCTATTA CTGTCCCCAA ATTCCCCTAG TCCCTTGGGT CATGATTAAA CATTTTGACT   1861
   TAAAAAAAAA AAAAAAAAAA AAAAAAAA                                      1889
(2) INFORMATION FOR SEQ ID NO:17:
   (i) SEQUENCE CHARACTERISTICS:
      (A) LENGTH: 397 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Gly Leu Ser Trp Ser Pro Arg Pro Pro Leu Leu Met Ile Leu Leu
    1               5                  10                  15
   Leu Val Leu Ser Leu Trp Leu Pro Leu Gly Ala Gly Asn Ser Leu Ala
               20                  25                  30
   Thr Glu Asn Arg Phe Val Asn Ser Cys Thr Gln Ala Arg Lys Lys Cys
           35                  40                  45
   Glu Ala Asn Pro Ala Cys Lys Ala Ala Tyr Gln His Leu Gly Ser Cys
       50                  55                  60
   Thr Ser Ser Leu Ser Arg Pro Leu Pro Leu Glu Glu Ser Ala Met Ser
   65                  70                  75                  80
   Ala Asp Cys Leu Glu Ala Ala Glu Gln Leu Arg Asn Ser Ser Leu Ile
                   85                  90                  95
   Asp Cys Arg Cys His Arg Arg Met Lys His Gln Ala Thr Cys Leu Asp
               100                 105                 110
   Ile Tyr Trp Thr Val His Pro Ala Arg Ser Leu Gly Asp Tyr Glu Leu
           115                 120                 125
   Asp Val Ser Pro Tyr Glu Asp Thr Val Thr Ser Lys Pro Trp Lys Met
       130                 135                 140
   Asn Leu Ser Lys Leu Asn Met Leu Lys Pro Asp Ser Asp Leu Cys Leu
   145                 150                 155                 160
   Lys Phe Ala Met Leu Cys Thr Leu His Asp Lys Cys Asp Arg Leu Arg
                   165                 170                 175
   Lys Ala Tyr Gly Glu Ala Cys Ser Gly Ile Arg Cys Gln Arg His Leu
               180                 185                 190
   Cys Leu Ala Gln Leu Arg Ser Phe Phe Glu Lys Ala Ala Glu Ser His
           195                 200                 205
   Ala Gln Gly Leu Leu Leu Cys Pro Cys Ala Pro Glu Asp Ala Gly Cys
       210                 215                 220
   Gly Glu Arg Arg Arg Asn Thr Ile Ala Pro Ser Cys Ala Leu Pro Ser
   225                 230                 235                 240
   Val Thr Pro Asn Cys Leu Asp Leu Arg Ser Phe Cys Arg Ala Asp Pro
                   245                 250                 255
   Leu Cys Arg Ser Arg Leu Met Asp Phe Gln Thr His Cys His Pro Met
               260                 265                 270
   Asp Ile Leu Gly Thr Cys Ala Thr Glu Gln Ser Arg Cys Leu Arg Ala
           275                 280                 285
   Tyr Leu Gly Leu Ile Gly Thr Ala Met Thr Pro Asn Phe Ile Ser Lys
       290                 295                 300
   Val Asn Thr Thr Val Ala Leu Ser Cys Thr Cys Arg Gly Ser Gly Asn
   305                 310                 315                 320
   Leu Gln Asp Glu Cys Glu Gln Leu Glu Arg Ser Phe Ser Gln Asn Pro
                   325                 330                 335
   Cys Leu Val Glu Ala Ile Ala Ala Lys Met Arg Phe His Arg Gln Leu
               340                 345                 350
   Phe Ser Gln Asp Trp Ala Asp Ser Thr Phe Ser Val Val Gln Gln Gln
           355                 360                 365
   Asn Ser Asn Pro Ala Leu Arg Leu Gln Pro Arg Leu Pro Ile Leu Ser
       370                 375                 380
   Phe Ser Ile Leu Pro Leu Ile Leu Leu Gln Thr Leu Trp
   385                 390                 395
(2) INFORMATION FOR SEQ ID NO:18:
   (i) SEQUENCE CHARACTERISTICS:
      (A) LENGTH: 1271 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 2..946
   (xi) SEQUENCE DESCRIPTION: SEQ ID NO:18:
   C GGC TAC TGT GAA ACA CCT CAA CTC AGG AAC AGC TCT CTG ATA GGC        46
     Gly Tyr Cys Glu Thr Pro Gln Leu Arg Asn Ser Ser Leu Ile Gly
             400                 405                 410
   TGC ATG TGC CAC CGG CGC ATG AAG AAC CAG GTT GCC TGC TTG GAC ATC      94
   Cys Met Cys His Arg Arg Met Lys Asn Gln Val Ala Cys Leu Asp Ile
           415                 420                 425
   TAT TGG ACC GTT CAC CGT GCC CGC AGC CTT GGT AAC TAT GAG CTG GAT     142
   Tyr Trp Thr Val His Arg Ala Arg Ser Leu Gly Asn Tyr Glu Leu Asp
       430                 435                 440
   GTC TCC CCC TAT GAA GAC ACA GTG ACC AGC AAA CCC TGG AAA ATG AAT     190
   Val Ser Pro Tyr Glu Asp Thr Val Thr Ser Lys Pro Trp Lys Met Asn
   445                 450                 455                 460
   CTC AGC AAA CTG AAC ATG CTC AAA CCA GAC TCA GAC CTC TGC CTC AAG     238
   Leu Ser Lys Leu Asn Met Leu Lys Pro Asp Ser Asp Leu Cys Leu Lys
                   465                 470                 475
   TTT GCC ATG CTG TGT ACT CTC AAT GAC AAG TGT GAC CGG CTG CGC AAG     286
   Phe Ala Met Leu Cys Thr Leu Asn Asp Lys Cys Asp Arg Leu Arg Lys
               480                 485                 490
   GCC TAC GGG GAG GCG TGC TCC GGG CCC CAC TGC CAG CGC CAC GTC TGC     334
   Ala Tyr Gly Glu Ala Cys Ser Gly Pro His Cys Gln Arg His Val Cys
           495                 500                 505
   CTC AGG CAG CTG CTC ACT TTC TTC GAG AAG GCC GCC GAG CCC CAC GCG     382
   Leu Arg Gln Leu Leu Thr Phe Phe Glu Lys Ala Ala Glu Pro His Ala
       510                 515                 520
   CAG GGC CTG CTA CTG TGC CCA TGT GCC CCC AAC GAC CGG GGC TGC GGG     430
   Gln Gly Leu Leu Leu Cys Pro Cys Ala Pro Asn Asp Arg Gly Cys Gly
   525                 530                 535                 540
   GAG CGC CGG CGC AAC ACC ATC GCC CCC AAC TGC GCG CTG CCG CCT GTG      478
   Glu Arg Arg Arg Asn Thr Ile Ala Pro Asn Cys Ala Leu Pro Pro Val
                   545                 550                 555
   GCC CCC AAC TGC CTG GAG CTG CGG CGC CTC TGC TTC TCC GAC CCG CTT      526
   Ala Pro Asn Cys Leu Glu Leu Arg Arg Leu Cys Phe Ser Asp Pro Leu
               560                 565                 570
   TGC AGA TCA CGC CTG GTG GAT TTC CAG ACC CAC TGC CAT CCC ATG GAC      574
   Cys Arg Ser Arg Leu Val Asp Phe Gln Thr His Cys His Pro Met Asp
           575                 580                 585
   ATC CTA GGA ACT TGT GCA ACA GAG CAG TCC AGA TGT CTA CGA GCA TAC      622
   Ile Leu Gly Thr Cys Ala Thr Glu Gln Ser Arg Cys Leu Arg Ala Tyr
       590                 595                 600
   CTG GGG CTG ATT GGG ACT GCC ATG ACC CCC AAC TTT GTC AGC AAT GTC      670
   Leu Gly Leu Ile Gly Thr Ala Met Thr Pro Asn Phe Val Ser Asn Val
   605                 610                 615                 620
   AAC ACC AGT GTT GCC TTA AGC TGC ACC TGC CGA GGC AGT GGC AAC CTG      718
   Asn Thr Ser Val Ala Leu Ser Cys Thr Cys Arg Gly Ser Gly Asn Leu
                   625                 630                 635
   CAG GAG GAG TGT GAA ATG CTG GAA GGG TTC TTC TCC CAC AAC CCC TGC      766
   Gln Glu Glu Cys Glu Met Leu Glu Gly Phe Phe Ser His Asn Pro Cys
               640                 645                 650
   CTC ACG GAG GCC ATT GCA GCT AAG ATG CGT TTT CAC AGC CAA CTC TTC      814
   Leu Thr Glu Ala Ile Ala Ala Lys Met Arg Phe His Ser Gln Leu Phe
           655                 660                 665
   TCC CAG GAC TGG CCA CAC CCT ACC TTT GCT GTG ATG GCA CAC CAG AAT      862
   Ser Gln Asp Trp Pro His Pro Thr Phe Ala Val Met Ala His Gln Asn
       670                 675                 680
   GAA AAC CCT GCT GTG AGG CCA CAG CCC TGG GTG CCC TCT CTT TTC TCC      910
   Glu Asn Pro Ala Val Arg Pro Gln Pro Trp Val Pro Ser Leu Phe Ser
   685                 690                 695                 700
   TGC ACG CTT CCC TTG ATT CTG CTC CTG AGC CTA TGG TAGCTGGACT           956
   Cys Thr Leu Pro Leu Ile Leu Leu Leu Ser Leu Trp
                   705                 710
   TCCCCAGGGC CCTCTTCCCC TCCACCACAC CCAGGTGGAC TTGCAGCCCA CAAGGGGTGA   1016
   GGAAAGGACA GCAGCAGGAA GGAGGTGCAG TGCGCAGATG AGGGCACAGG AGAAGCTAAG   1076
   GGTTATGACC TCCAGATCCT TACTGGTCCA GTCCTCATTC CCTCCACCCC ATCTCCACTT   1136
   CTGATTCATG CTGCCCCTCC TTGGTGGCCA CAATTTAGCC ATGTCATCTG GTGCCTGTGG   1196
   GCCTTGCTTT ATTCCTATTA TTGTCCTAAA GTCTCTCTGG GCTCTTGGAT CATGATTAAA   1256
   CCTTTGACTT AAAAA                                                    1271
(2) INFORMATION FOR SEQ ID NO:19:
   (i) SEQUENCE CHARACTERISTICS:
      (A) LENGTH: 315 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Gly Tyr Cys Glu Thr Pro Gln Leu Arg Asn Ser Ser Leu Ile Gly Cys
    1               5                  10                  15
   Met Cys His Arg Arg Met Lys Asn Gln Val Ala Cys Leu Asp Ile Tyr
               20                  25                  30
   Trp Thr Val His Arg Ala Arg Ser Leu Gly Asn Tyr Glu Leu Asp Val
           35                  40                  45
   Ser Pro Tyr Glu Asp Thr Val Thr Ser Lys Pro Trp Lys Met Asn Leu
       50                  55                  60
   Ser Lys Leu Asn Met Leu Lys Pro Asp Ser Asp Leu Cys Leu Lys Phe
   65                  70                  75                  80
   Ala Met Leu Cys Thr Leu Asn Asp Lys Cys Asp Arg Leu Arg Lys Ala
                   85                  90                  95
   Tyr Gly Glu Ala Cys Ser Gly Pro His Cys Gln Arg His Val Cys Leu
               100                 105                 110
   Arg Gln Leu Leu Thr Phe Phe Glu Lys Ala Ala Glu Pro His Ala Gln
           115                 120                 125
   Gly Leu Leu Leu Cys Pro Cys Ala Pro Asn Asp Arg Gly Cys Gly Glu
       130                 135                 140
   Arg Arg Arg Asn Thr Ile Ala Pro Asn Cys Ala Leu Pro Pro Val Ala
   145                 150                 155                 160
   Pro Asn Cys Leu Glu Leu Arg Arg Leu Cys Phe Ser Asp Pro Leu Cys
                   165                 170                 175
   Arg Ser Arg Leu Val Asp Phe Gln Thr His Cys His Pro Met Asp Ile
               180                 185                 190
   Leu Gly Thr Cys Ala Thr Glu Gln Ser Arg Cys Leu Arg Ala Tyr Leu
           195                 200                 205
   Gly Leu Ile Gly Thr Ala Met Thr Pro Asn Phe Val Ser Asn Val Asn
       210                 215                 220
   Thr Ser Val Ala Leu Ser Cys Thr Cys Arg Gly Ser Gly Asn Leu Gln
   225                 230                 235                 240
   Glu Glu Cys Glu Met Leu Glu Gly Phe Phe Ser His Asn Pro Cys Leu
                   245                 250                 255
   Thr Glu Ala Ile Ala Ala Lys Met Arg Phe His Ser Gln Leu Phe Ser
               260                 265                 270
   Gln Asp Trp Pro His Pro Thr Phe Ala Val Met Ala His Gln Asn Glu
           275                 280                 285
   Asn Pro Ala Val Arg Pro Gln Pro Trp Val Pro Ser Leu Phe Ser Cys
       290                 295                 300
   Thr Leu Pro Leu Ile Leu Leu Leu Ser Leu Trp
   305                 310                 315
(2) INFORMATION FOR SEQ ID NO:20:
   (i) SEQUENCE CHARACTERISTICS:
      (A) LENGTH: 1699 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 175..1374
   (xi) SEQUENCE DESCRIPTION: SEQ ID NO:20:
   TGTGGACGCG CGCTTCGGAG TTGGAGGGCG GCGCCCAGGA CCCTGGTGGG AGAGTGTGTG      60
   CGTCGCGCTG GAGGGCGGGA GGCGGGGGCG GGAGGTGCCG GTCGAGGGAG CCCCGCTCTC     120
   AGAGCTCCAG GGGAGGAGCG AGGGGAGCGC GGAGCCCGGC GCCTACAGCT CGCC ATG       177
                                                               Met
   GTG CGC CCC CTG AAC CCG CGA CCG CTG CCG CCC GTA GTC CTG ATG TTG       225
   Val Arg Pro Leu Asn Pro Arg Pro Leu Pro Pro Val Val Leu Met Leu
           320                 325                 330
   CTG CTG CTG CTG CCG CCG TCG CCG CTG CCT CTC GCA GCC GGA GAC CCC       273
   Leu Leu Leu Leu Pro Pro Ser Pro Leu Pro Leu Ala Ala Gly Asp Pro
       335                 340                 345
   CTT CCC ACA GAA AGC CGA CTC ATG AAC AGC TGT CTC CAG GCC AGG AGG       321
   Leu Pro Thr Glu Ser Arg Leu Met Asn Ser Cys Leu Gln Ala Arg Arg
   350                 355                 360
   AAG TGC CAG GCT GAT CCC ACC TGC AGT GCT GCC TAC CAC CAC CTG GAT      369
   Lys Cys Gln Ala Asp Pro Thr Cys Ser Ala Ala Tyr His His Leu Asp
   365                 370                 375                 380
   TCC TGC ACC TCT AGC ATA AGC ACC CCA CTG CCC TCA GAG GAG CCT TCG      417
   Ser Cys Thr Ser Ser Ile Ser Thr Pro Leu Pro Ser Glu Glu Pro Ser
                   385                 390                 395
   GTC CCT GCT GAC TGC CTG GAG GCA GCA CAG CAA CTC AGG AAC AGC TCT      465
   Val Pro Ala Asp Cys Leu Glu Ala Ala Gln Gln Leu Arg Asn Ser Ser
               400                 405                 410
   CTG ATA GGC TGC ATG TGC CAC CGG CGC ATG AAG AAC CAG GTT GCC TGC      513
   Leu Ile Gly Cys Met Cys His Arg Arg Met Lys Asn Gln Val Ala Cys
           415                 420                 425
   TTG GAC ATC TAT TGG ACC GTT CAC CGT GCC CGC AGC CTT GGT AAC TAT      561
   Leu Asp Ile Tyr Trp Thr Val His Arg Ala Arg Ser Leu Gly Asn Tyr
       430                 435                 440
   GAG CTG GAT GTC TCC CCC TAT GAA GAC ACA GTG ACC AGC AAA CCC TGG      609
   Glu Leu Asp Val Ser Pro Tyr Glu Asp Thr Val Thr Ser Lys Pro Trp
   445                 450                 455                 460
   AAA ATG AAT CTC AGC AAA CTG AAC ATG CTC AAA CCA GAC TCA GAC CTC      657
   Lys Met Asn Leu Ser Lys Leu Asn Met Leu Lys Pro Asp Ser Asp Leu
                   465                 470                 475
   TGC CTC AAG TTT GCC ATG CTG TGT ACT CTC AAT GAC AAG TGT GAC CGG      705
   Cys Leu Lys Phe Ala Met Leu Cys Thr Leu Asn Asp Lys Cys Asp Arg
               480                 485                 490
   CTG CGC AAG GCC TAC GGG GAG GCG TGC TCC GGG CCC CAC TGC CAG CGC      753
   Leu Arg Lys Ala Tyr Gly Glu Ala Cys Ser Gly Pro His Cys Gln Arg
           495                 500                 505
   CAC GTC TGC CTC AGG CAG CTG CTC ACT TTC TTC GAG AAG GCC GCC GAG      801
   His Val Cys Leu Arg Gln Leu Leu Thr Phe Phe Glu Lys Ala Ala Glu
       510                 515                 520
   CCC CAC GCG CAG GGC CTG CTA CTG TGC CCA TGT GCC CCC AAC GAC CGG      849
   Pro His Ala Gln Gly Leu Leu Leu Cys Pro Cys Ala Pro Asn Asp Arg
   525                 530                 535                 540
   GGC TGC GGG GAG CGC CGG CGC AAC ACC ATC GCC CCC AAC TGC GCG CTG      897
   Gly Cys Gly Glu Arg Arg Arg Asn Thr Ile Ala Pro Asn Cys Ala Leu
                   545                 550                 555
   CCG CCT GTG GCC CCC AAC TGC CTG GAG CTG CGG CGC CTC TGC TTC TCC      945
   Pro Pro Val Ala Pro Asn Cys Leu Glu Leu Arg Arg Leu Cys Phe Ser
               560                 565                 570
   GAC CCG CTT TGC AGA TCA CGC CTG GTG GAT TTC CAG ACC CAC TGC CAT         993
   Asp Pro Leu Cys Arg Ser Arg Leu Val Asp Phe Gln Thr His Cys His
           575                 580                 585
   CCC ATG GAC ATC CTA GGA ACT TGT GCA ACA GAG CAG TCC AGA TGT CTA        1041
   Pro Met Asp Ile Leu Gly Thr Cys Ala Thr Glu Gln Ser Arg Cys Leu
       590                 595                 600
   CGA GCA TAC CTG GGG CTG ATT GGG ACT GCC ATG ACC CCC AAC TTT GTC        1089
   Arg Ala Tyr Leu Gly Leu Ile Gly Thr Ala Met Thr Pro Asn Phe Val
   605                 610                 615                 620
   AGC AAT GTC AAC ACC AGT GTT GCC TTA AGC TGC ACC TGC CGA GGC AGT        1137
   Ser Asn Val Asn Thr Ser Val Ala Leu Ser Cys Thr Cys Arg Gly Ser
                   625                 630                 635
   GGC AAC CTG CAG GAG GAG TGT GAA ATG CTG GAA GGG TTC TTC TCC CAC        1185
   Gly Asn Leu Gln Glu Glu Cys Glu Met Leu Glu Gly Phe Phe Ser His
               640                 645                 650
   AAC CCC TGC CTC ACG GAG GCC ATT GCA GCT AAG ATG CGT TTT CAC AGC        1233
   Asn Pro Cys Leu Thr Glu Ala Ile Ala Ala Lys Met Arg Phe His Ser
           655                 660                 665
   CAA CTC TTC TCC CAG GAC TGG CCA CAC CCT ACC TTT GCT GTG ATG GCA        1281
   Gln Leu Phe Ser Gln Asp Trp Pro His Pro Thr Phe Ala Val Met Ala
       670                 675                 680
   CAC CAG AAT GAA AAC CCT GCT GTG AGG CCA CAG CCC TGG GTG CCC TCT        1329
   His Gln Asn Glu Asn Pro Ala Val Arg Pro Gln Pro Trp Val Pro Ser
   685                 690                 695                 700
   CTT TTC TCC TGC ACG CTT CCC TTG ATT CTG CTC CTG AGC CTA TGG            1374
   Leu Phe Ser Cys Thr Leu Pro Leu Ile Leu Leu Leu Ser Leu Trp
                   705                 710                 715
   TAGCTGGACT TCCCCAGGGC CCTCTTCCCC TCCACCACAC CCAGGTGGAC TTGCAGCCCA      1434
   CAAGGGGTGA GGAAAGGACA GCAGCAGGAA GGAGGTGCAG TGCGCAGATG AGGGCACAGG      1494
   AGAAGCTAAG GGTTATGACC TCCAGATCCT TACTGGTCCA GTCCTCATTC CCTCCACCCC      1554
   ATCTCCACTT CTGATTCATG CTGCCCCTCC TTGGTGGCCA CAATTTAGCC ATGTCATCTG      1614
   GTGCCTGTGG GCCTTGCTTT ATTCCTATTA TTGTCCTAAA GTCTCTCTGG GCTCTTGGAT      1674
   CATGATTAAA CCTTTGACTT AAAAA                                            1699
(2) INFORMATION FOR SEQ ID NO:21:
   (i) SEQUENCE CHARACTERISTICS:
      (A) LENGTH: 400 amino acids
      (B) TYPE: amino acid
      (D) TOPOLOGY: linear
   (ii) MOLECULE TYPE: protein
   (xi) SEQUENCE DESCRIPTION: SEQ ID NO:21:

### SEQUENCE LISTING

(1) GENERAL INFORMATION:
   (i) APPLICANT: BIOGEN, INC.
   (ii) TITLE OF INVENTION: Ret Ligand (RetL) for Stimulating Neural and Renal Growth
   (iii) NUMBER OF SEQUENCES: 21
   (iv) CORRESPONDENCE ADDRESS:
      (A) ADDRESSEE: Biogen, Inc.
      (B) STREET: 14 Cambridge Center
      (C) CITY: Cambridge
      (D) STATE: MA
      (E) COUNTRY: USA
      (F) ZIP: 02142
   (v) COMPUTER READABLE FORM:
      (A) MEDIUM TYPE: Floppy disk
      (B) COMPUTER: IBM PC compatible
      (C) OPERATING SYSTEM: PC-DDS/MS-DOS
      (D) SOFTWARE: PatentIn Release #1.0, Version #1.30
   (vi) CURRENT APPLICATION DATA:
      (A) APPLICATION NUMBER:
      (B) FILING DATE: 07-MAY-1997
      (C) CLASSIFICATION:
   (vii) PRIOR APPLICATION DATA:
      (A) APPLICATION NUMBER: US 08/999,999
      (B) FILING DATE: 01-JAN 1996
   (viii) ATTORNEY/AGENT INFORMATION:
      (A) NAME: Levine, Leslie M.
      (B) REGISTRATION NUMBER: 35,245
      (C) REFERENCE/DOCKET NUMBER: A008 PCT CIP
   (ix) TELECOMMUNICATION INFORMATION:
      (A) TELEPHONE: 617-679-2400
      (B) TELEFAX: 617-679-2838
(2) INFORMATION-FOR SEQ ID NO:1:
   (i) SEQUENCE CHARACTERISTICS:
      (A) LENGTH: 3616 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KBY: CDS
      (B) LOCATION: 257..1660
   (xi) SEQUENCE DESCRIPTION: SEQ ID NO:1
(2) INFORMATION FOR SEQ ID NO: 2 :
   (i) SEQUENCE CHARACTERISTICS:
      (A) LENGTH: 468 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Phe Leu Ala Thr Leu Tyr Phe Ala Leu Pro Leu Leu Asp Leu Leu
    1               5                  10                  15
   Met Ser Ala Glu Val Ser Gly Gly Asp Arg Leu Asp Cys Val Lys Ala
               20                  25                  30
   Ser Asp Gln Cys Leu Lys Glu Gln Ser Cys Ser Thr Lys Tyr Arg Thr
           35                  40                  45
   Leu Arg Gln Cys Val Ala Gly Lys Glu Thr Asn Phe Ser Leu Thr Ser
       50                  55                  60
   Gly Leu Glu Ala Lys Asp Glu Cys Arg Ser Ala Met Glu Ala Leu Lys
   65                  70                  75                  80
   Gln Lys Ser Leu Tyr Asn Cys Arg Cys Lys Arg Gly Met Lys Lys Glu
                   85                  90                  95
   Lys Asn Cys Leu Arg Ile Tyr Trp Ser Met Tyr Gln Ser Leu Gln Gly
               100                 105                 110
   Asn Asp Leu Leu Glu Asp Ser Pro Tyr Glu Pro Val Asn Ser Arg Leu
           115                 120                 125
   Ser Asp Ile Phe Arg Ala Val Pro Phe Ile Ser Asp Val Phe Gln Gln
       130                 135                 140
   Val Glu His Ile Ser Lys Gly Asn Asn Cys Leu Asp Ala Ala Lys Ala
   145                 150                 155                 160
   Cys Asn Leu Asp Asp Thr Cys Lys Lys Tyr Arg Ser Ala Tyr Ile Thr
                   165                 170                 175
   Pro Cys Thr Thr Ser Met Ser Asn Glu Val Cys Asn Arg Arg Lys Cys
               180                 185                 190
   His Lys Ala Leu Arg Gln Phe Phe Asp Lys Val Pro Ala Lys His Ser
           195                 200                 205
   Tyr Gly Met Leu Phe Cys Ser Cys Arg Asp Ile Ala Cys Thr Glu Arg
       210                 215                 220
   Arg Arg Gln Thr Ile Val Pro Val Cys Ser Tyr Glu Glu Arg Glu Arg
   225                 230                 235                 240
   Pro Asn Cys Leu Ser Leu Gln Asp Ser Cys Lys Thr Asn Tyr Ile Cys
                   245                 250                 255
   Arg Ser Arg Leu Ala Asp Phe Phe Thr Asn Cys Gln Pro Glu Ser Arg
                   260                          265                          270
   Ser Val Ser Asn Cys Leu Lys Glu Asn Tyr Ala Asp Cys Leu Leu Ala
           275                     280                     285
   Tyr Ser Gly Leu Ile Gly Thr Val Met Thr Pro Asn Tyr Val Asp Ser
       290                     295                     300
   Ser Ser Leu Ser Val Ala Pro Trp Cys Asp Cys Ser Asn Ser Gly Asn
   305                     310                     315                     320
   Asp Leu Glu Asp Cys Leu Lys Phe Leu Asn Phe Phe Lys Asp Asn Thr
                       325                     330                     335
   Cys Leu Lys Asn Ala Ile Gln Ala Phe Gly Asn Gly Ser Asp Val Thr
                   340                     345                     350
   Met Trp Gln Pro Ala Pro Pro Val Gln Thr Thr Thr Ala Thr Thr Thr
               355                     360                     365
   Thr Ala Phe Arg Val Lys Asn Lys Pro Leu Gly Pro Ala Gly Ser Glu
       370                     375                     380
   Asn Glu Ile Pro Thr His Val Leu Pro Pro Cys Ala Asn Leu Gln Ala
   385                     390                     395                     400
   Gln Lys Leu Lys Ser Asn Val Ser Gly Ser Thr His Leu Cys Leu Ser
                   405                     410                     415
   Asp Ser Asp Phe Gly Lys Asp Gly Leu Ala Gly Ala Ser Ser His Ile
               420                     425                     430
   Thr Thr Lys Ser Met Ala Ala Pro Pro Ser Cys Ser Leu Ser Ser Leu
           435                     440                     445
   Pro Val Leu Met Leu Thr Ala Leu Ala Ala Leu Leu Ser Val Ser Leu
       450                     455                     460
   Ala Glu Thr Ser
   465
(2) INFORMATION FOR SEQ ID NO:3:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
      AAGGAAAAAA GCGGCCGCCA TGGCCAAGGC GACGTCCGG 39
(2) INFORMATION FOR SEQ ID NO:4:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
      AGTTTTGTCG ACCGTGCGGC ACAGCTCGTC GCA 33
(2) INFORMATION FOR SEQ ID NO:5:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
      AGTTTTGTCG ACCGTGCGGC ACAGCGCATC ACA 33
(2) INFORMATION FOR SEQ ID NO:6:
   (i) SEQUENCE CHARACTERISTICS:
      (A) LENGTH: 1926 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 10..1920
   (xi) SEQUENCE DESCRIPTION: SEQ ID NO:6:
        GCGGCCGCC ATG GCG AAG GCG ACG TCC GGC GCC GCA GGG CTG GGG CTG
   48
                  Met Ala Lys Ala Thr Ser Gly Ala Ala Gly Leu Gly Leu
                      470                 475                 480
        AAG CTG TTT TTG CTG CTG CCG CTA CTG GGA GAA GCC CCG CTG GGT CTC
   96
        Lys Leu Phe Leu Leu Leu Pro Leu Leu Gly Glu Ala Pro Leu Gly Leu
                    485                 490                 495
        TAC TTC TCA AGG GAT GCT TAC TGG GAG AGG CTG TAT GTG GAC CAG CCA
   144
        Tyr Phe Ser Arg Asp Ala Tyr Trp Glu Arg Leu Tyr Val Asp Gln Pro
                500                 505                 510
        GCT GGC ACA CCT CTG CTC TAT GTC CAT GCC CTA CGG GAT GCC CCT GGA
   192
       Ala Gly Thr Pro Leu Leu Tyr Val His Ala Leu Arg Asp Ala Pro Gly
           515                 520                 525
       GAA GTG CCC AGC TTC CGC CTG GGC CAG TAT CTC TAT GGC GTC TAC CGC
   240
       Glu Val Pro Ser Phe Arg Leu Gly Gln Tyr Leu Tyr Gly Val Tyr Arg
       530                 535                 540                 545
       ACG CGT CTG CAT GAG AAT GAC TGG ATC CAC ATC GAT GCG GGC ACT GGC
   288
       Thr Arg Leu His Glu Asn Asp Trp Ile His Ile Asp Ala Gly Thr Gly
                       550                 555                 560
       CTC CTC TAC CTC AAT CAG AGC CTG GAC CAT AGT TCC TGG GAG CAG CTC
   336
       Leu Leu Tyr Leu Asn Gln Ser Leu Asp His Ser Ser Trp Glu Gln Leu
                   565                 570                 575
       AGC ATC CGA AAT GGC GGC TTC CCC TTG CTC ACC GTC TTC CTC CAG GTC
   384
       Ser Ile Arg Asn Gly Gly Phe Pro Leu Leu Thr Val Phe Leu Gln Val
               580                 585                 590
       TTC CTG GGG TCC ACA GCC CAG AGA GAG GGA GAG TGT CAT TGG CCA GGC
   432
       Phe Leu Gly Ser Thr Ala Gln Arg Glu Gly Glu Cys His Trp Pro Gly
           595                 600                 605
       TGT GCC CGT GTG TAC TTC TCC TTC ATC AAC GAC ACC TTC CCA AAT TGT
   480
       Cys Ala Arg Val Tyr Phe Ser Phe Ile Asn Asp Thr Phe Pro Asn Cys
       610                 615                 620                 625
       AGC TCC TTC AAA GCC CGG GAT CTC TGC ACC CCA GAG ACG GGT GTG TCC
   528
       Ser Ser Phe Lys Ala Arg Asp Leu Cys Thr Pro Glu Thr Gly Val Ser
                       630                 635                 640
       TTC CGC ATC AGG GAG AAC AGG CCC CCT GGC ACC TTC TAC CAG TTC CGC
   576
       Phe Arg Ile Arg Glu Asn Arg Pro Pro Gly Thr Phe Tyr Gln Phe Arg
                   645                 650                 655
       ATG CTA CCT GTG CAG TTC CTT TGT CCT AAC ATC AGT GTG AAG TAC AAA
   624
       Met Leu Pro Val Gln Phe Leu Cys Pro Asn Ile Ser Val Lys Tyr Lys
               660                 665                 670
       CTC TTA GAA GGG GAC GGT CTG CCC TTC CGT TGT GAC CCC GAC TGT CTG
   672
       Leu Leu Glu Gly Asp Gly Leu Pro Phe Arg Cys Asp Pro Asp Cys Leu
           675                 680                 685
       GAG GTG AGC ACG CGG TGG GCA CTG GAT CGG GAG CTT CAG GAG AAG TAT
   720
       Glu Val Ser Thr Arg Trp Ala Leu Asp Arg Glu Leu Gln Glu Lys Tyr
       690                 695                 700                 705
       GTG CTG GAG GCT GAG TGC GCA GTG GCA GGC CCT GGA GCC AAC AAG GAG
   768
       Val Leu Glu Ala Glu Cys Ala Val Ala Gly Pro Gly Ala Asn Lys Glu
                       710                 715                 720
       AAG GTG GCC GTG TCC TTC CCG GTG ACG GTG TAT GAT GAA GAC GAC TCC
   816
   Lys Val Ala Val Ser Phe Pro Val Thr Val Tyr Asp Glu Asp Asp Ser
               725                 730                 735
   CCG CCC ACC TTC TCC GGA GGT GTG GGC ACC GCC AGT GCT GTG GTG GAG
   864
   Pro Pro Thr Phe Ser Gly Gly Val Gly Thr Ala Ser Ala Val Val Glu
           740                 745                 750
   TTT AAG CGG AAG GAG GGC ACT GTG GTA GCC ACT CTG CAG GTG TTT GAT
   912
   Phe Lys Arg Lys Glu Gly Thr Val Val Ala Thr Leu Gln Val Phe Asp
       755                 760                 765
   GCA GAT GTG GTG CCA GCA TCT GGG GAG CTG GTG AGG CGG TAC ACA AGC
   960
   Ala Asp Val Val Pro Ala Ser Gly Glu Leu Val Arg Arg Tyr Thr Ser
   770                 775                 780                 785
   ACA CTA CTC TCA GGG GAT TCC TGG GCC CAG CAG ACC TTC CGG GTG GAG
   1008
   Thr Leu Leu Ser Gly Asp Ser Trp Ala Gln Gln Thr Phe Arg Val Glu
                   790                 795                 800
   CAC ACA CCC AAC GAG ACC TTG GTC CAG TCC AAC AAC AAC TCC GTG CGG
   1056
   His Thr Pro Asn Glu Thr Leu Val Gln Ser Asn Asn Asn Ser Val Arg
               805                 810                 815
   GCA ACC ATG CAC AAT TAC AAG CTG GTT CTC AAC AGG AGC CTG TCC ATC
   1104
   Ala Thr Met His Asn Tyr Lys Leu Val Leu Asn Arg Ser Leu Ser Ile
           820                 825                 830
   TCA GAG AGC CGA GTC CTG CAG CTA GTA GTC CTG GTC AAT GAC TCA GAC
   1152
   Ser Glu Ser Arg Val Leu Gln Leu Val Val Leu Val Asn Asp Ser Asp
       835                 840                 845
   TTC CAG GGG CCT GGG TCA GGT GTT CTC TTC CTC CAT TTC AAC GTG TCT
   1200
   Phe Gln Gly Pro Gly Ser Gly Val Leu Phe Leu His Phe Asn Val Ser
   850                 855                 860                 865
   GTG CTG CCT GTC ACC CTG AAC CTA CCC ATG GCC TAC TCC TTC CCA GTG
   1248
   Val Leu Pro Val Thr Leu Asn Leu Pro Met Ala Tyr Ser Phe Pro Val
                   870                 875                 880
   AAT AGG AGA GCC CGC CGT TAT GCC CAG ATT GGG AAA GTT TGC GTG GAG
   1296
   Asn Arg Arg Ala Arg Arg Tyr Ala Gln Ile Gly Lys Val Cys Val Glu
               885                 890                 895
   AAC TGC CAG GAG TTC AGC GGT GTC TCC ATC CAG TAC AAG CTG CAG CCC
   1344
   Asn Cys Gln Glu Phe Ser Gly Val Ser Ile Gln Tyr Lys Leu Gln Pro
           900                 905                 910
   TCC AGC ACC AAC TGC AGT GCC CTA GGT GTG GTC ACC TCA ACA GAA GAC
   1392
   Ser Ser Thr Asn Cys Ser Ala Leu Gly Val Val Thr Ser Thr Glu Asp
       915                 920                 925
       ACC TCA GGG ACC CTA TAT GTA AAT GAC ACG GAG GCC CTG CGG CGA CCT
   1440
   Thr Ser Gly Thr Leu Tyr Val Asn Asp Thr Glu Ala Leu Arg Arg Pro
   930                 935                 940                 945
   GAG TGT ACC GAG CTT CAG TAC ACA GTG GTA GCC ACT GAC CGG CAG ACC
   1488
   Glu Cys Thr Glu Leu Gln Tyr Thr Val Val Ala Thr Asp Arg Gln Thr
                   950                 955                 960
   CGC AGG CAG ACC CAA GCT TCG TTA GTC GTC ACA GTG GAG GGG ACA TAC
   1536
   Arg Arg Gln Thr Gln Ala Ser Leu Val Val Thr Val Glu Gly Thr Tyr
               965                 970                 975
   ATT GCA GAA GAA GTG GGC TGC CCC AAG TCC TCT GCA CTA AAC AAG AGG
   1584
   Ile Ala Glu Glu Val Gly Cys Pro Lys Ser Cys Ala Val Asn Lys Arg
           980                 985                 990
   CGA CCT GAG TGT GAG GAG TGT GGT GGC CTG GGT TCT CCA ACT GGC AGA
   1632
   Arg Pro Glu Cys Glu Glu Cys Gly Gly Leu Gly Ser Pro Thr Gly Arg
       995                 1000                1005
   TGT GAG TGG CGT CAG GGA GAT GGT AAA GGG ATC ACC AGG AAC TTC TCC
   1680
   Cys Glu Trp Arg Gln Gly Asp Gly Lys Gly Ile Thr Arg Asn Phe Ser
   1010                1015                1020                1025
   ACC TGT TCT CCT AGC ACC AGG ACC TGT CCT GAT GGC CAC TGT GAT GCT
   1728
   Thr Cys Ser Pro Ser Thr Arg Thr Cys Pro Asp Gly His Cys Asp Ala
                   1030                1035                1040
   CTG GAG AGC CGG GAT ATC AAC ATT TGC CCC CAG GAC TGT CTC CGT GGC
   1776
   Leu Glu Ser Arg Asp Ile Asn Ile Cys Pro Gln Asp Cys Leu Arg Gly
               1045                1050                1055
   CCC ATT GTT GGC GGG CAT GAG CGA GGG GAG CGC CAG GGG ATT AAA GCC
   1824
   Pro Ile Val Gly Gly His Glu Arg Gly Glu Arg Gln Gly Ile Lys Ala
           1060                1065                1070
   GGC TAT GGC ATC TGC AAC TGT TTC CCT GAT GAG AAG AAG TGC TTC TGC
   1872
   Gly Tyr Gly Ile Cys Asn Cys Phe Pro Asp Glu Lys Lys Cys Phe Cys
       1075                1080                1085
   GAG CCA GAG GAC AGC CAG GGC CCA TTG TGT GAT GCG CTG TGC CGC ACG
   1920
   Glu Pro Glu Asp Ser Gln Gly Pro Leu Cys Asp Ala Leu Cys Arg Thr
   1090                1095                1100                1105
   GTCGAC
   1926
(2) INFORMATION FOR SEQ ID NO:7:
   (i) SEQUENCE CHARACTERISTICS:
      (A) LENGTH: 637 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Ala Lys Ala Thr Ser Gly Ala Ala Gly Leu Gly Leu Lys Leu Phe
   1               5                   10                  15
   Leu Leu Leu Pro Leu Leu Gly Glu Ala Pro Leu Gly Leu Tyr Phe Ser
               20                  25                  30
   Arg Asp Ala Tyr Trp Glu Arg Leu Tyr Val Asp Gln Pro Ala Gly Thr
           35                  40                  45
   Pro Leu Leu Tyr Val His Ala Leu Arg Asp Ala Pro Gly Glu Val Pro
       50                  55                  60
   Ser Phe Arg Leu Gly Gln Tyr Leu Tyr Gly Val Tyr Arg Thr Arg Leu
   65                  70                  75                  80
   His Glu Asn Asp Trp Ile His Ile Asp Ala Gly Thr Gly Leu Leu Tyr
                   85                  90                  95
   Leu Asn Gln Ser Leu Asp His Ser Ser Trp Glu Gln Leu Ser Ile Arg
               100                 105                 110
   Asn Gly Gly Phe Pro Leu Leu Thr Val Phe Leu Gln Val Phe Leu Gly
           115                 120                 125
   Ser Thr Ala Gln Arg Glu Gly Glu Cys His Trp Pro Gly Cys Ala Arg
       130                 135                 140
   Val Tyr Phe Ser Phe Ile Asn Asp Thr Phe Pro Asn Cys Ser Ser Phe
   145                 150                 155                 160
   Lys Ala Arg Asp Leu Cys Thr Pro Glu Thr Gly Val Ser Phe Arg Ile
                   165                 170                 175
   Arg Glu Asn Arg Pro Pro Gly Thr Phe Tyr Gln Phe Arg Met Leu Pro
               180                 185                 190
   Val Gln Phe Leu Cys Pro Asn Ile Ser Val Lys Tyr Lys Leu Leu Glu
           195                 200                 205
   Gly Asp Gly Leu Pro Phe Arg Cys Asp Pro Asp Cys Leu Glu Val Ser
       210                 215                 220
   Thr Arg Trp Ala Leu Asp Arg Glu Leu Gln Glu Lys Tyr Val Leu Glu
   225                 230                 235                 240
   Ala Glu Cys Ala Val Ala Gly Pro Gly Ala Asn Lys Glu Lys Val Ala
                   245                 250                 255
   Val Ser Phe Pro Val Thr Val Tyr Asp Glu Asp Asp Ser Pro Pro Thr
               260                 265                 270
   Phe Ser Gly Gly Val Gly Thr Ala Ser Ala Val Val Glu Phe Lys Arg
           275                 280                 285
   Lys Glu Gly Thr Val Val Ala Thr Leu Gln Val Phe Asp Ala Asp Val
       290                 295                 300
   Val Pro Ala Ser Gly Glu Leu Val Arg Arg Tyr Thr Ser Thr Leu Leu
   305                 310                 315                 320
   Ser Gly Asp Ser Trp Ala Gln Gln Thr Phe Arg Val Glu His Thr Pro
                   325                 330                 335
   Asn Glu Thr Leu Val Gln Ser Asn Asn Asn Ser Val Arg Ala Thr Met
               340                 345                 350
   His Asn Tyr Lys Leu Val Leu Asn Arg Ser Leu Ser Ile Ser Glu Ser
           355                 360                 365
   Arg Val Leu Gln Leu Val Val Leu Val Asn Asp Ser Asp Phe Gln Gly
       370                 375                 380
   Pro Gly Ser Gly Val Leu Phe Leu His Phe Asn Val Ser Val Leu Pro
   385                 390                 395                 400
   Val Thr Leu Asn Leu Pro Met Ala Tyr Ser Phe Pro Val Asn Arg Arg
                   405                 410                 415
   Ala Arg Arg Tyr Ala Gln Ile Gly Lys Val Cys Val Glu Asn Cys Gln
               420                 425                 430
   Glu Phe Ser Gly Val Ser Ile Gln Tyr Lys Leu Gln Pro Ser Ser Thr
           435                 440                 445
   Asn Cys Ser Ala Leu Gly Val Val Thr Ser Thr Glu Asp Thr Ser Gly
       450                 455                 460
   Thr Leu Tyr Val Asn Asp Thr Glu Ala Leu Arg Arg Pro Glu Cys Thr
   465                 470                 475                 480
   Glu Leu Gln Tyr Thr Val Val Ala Thr Asp Arg Gln Thr Arg Arg Gln
                   485                 490                 495
   Thr Gln Ala Ser Leu Val Val Thr Val Glu Gly Thr Tyr Ile Ala Glu
               500                 505                 510
   Glu Val Gly Cys Pro Lys Ser Cys Ala Val Asn Lys Arg Arg Pro Glu
           515                 520                 525
   Cys Glu Glu Cys Gly Gly Leu Gly Ser Pro Thr Gly Arg Cys Glu Trp
       530                 535                 540
   Arg Gln Gly Asp Gly Lys Gly Ile Thr Arg Asn Phe Ser Thr Cys Ser
   545                 550                 555                 560
   Pro Ser Thr Arg Thr Cys Pro Asp Gly His Cys Asp Ala Leu Glu Ser
                   565                 570                 575
   Arg Asp Ile Asn Ile Cys Pro Gln Asp Cys Leu Arg Gly Pro Ile Val
               580                 585                 590
   Gly Gly His Glu Arg Gly Glu Arg Gln Gly Ile Lys Ala Gly Tyr Gly
           595                 600                 605
   Ile Cys Asn Cys Phe Pro Asp Glu Lys Lys Cys Phe Cys Glu Pro Glu
       610                 615                 620
   Asp Ser Gln Gly Pro Leu Cys Asp Ala Leu Cys Arg Thr
   625                 630                 635
(2) INFORMATION FOR SEQ ID NO:8:
   (i) SEQUENCE CHARACTERISTICS:
      (A) LENGTH: 1223 base pairs
      (2) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1038
   (xi) SEQUENCE DESCRIPTION: SEQ ID NO:8:
   CTG CTG GAG GAT TCC CCA TAT GAA CCA GTT AAC AGC AGA TTG TCA GAT      48
   Leu Leu Glu Asp Ser Pro Tyr Glu Pro Val Asn Ser Arg Leu Ser Asp
           640                 645                 650
   ATA TTC CGG GTG GTC CCA TTC ATA TCA GTG GAG CAC ATT CCC AAA GGG      96
   Ile Phe Arg Val Val Pro Phe Ile Ser Val Glu His Ile Pro Lys Gly
       655                 660                 665
   AAC AAC TGC CTG GAT GCA GCG AAG GCC TGC AAC CTC GAC GAC ATT TGC     144
   Asn Asn Cys Leu Asp Ala Ala Lys Ala Cys Asn Leu Asp Asp Ile Cys
   670                 675                 680                 685
   AAG AAG TAC AGG TCG GCG TAC ATC ACC CCG TGC ACC ACC AGC GTG TCC     192
   Lys Lys Tyr Arg Ser Ala Tyr Ile Thr Pro Cys Thr Thr Ser Val Ser
                   690                 695                 700
   AAC GAT GTC TGC AAC CGC CGC AAG TGC CAC AAG GCC CTC CGG CAG TTC     240
   Asn Asp Val Cys Asn Arg Arg Lys Cys His Lys Ala Leu Arg Gln Phe
               705                 710                 715
   TTT GAC AAG GTC CCG GCC AAG CAC AGC TAC GGA ATG CTC TTC TGC TCC     288
   Phe Asp Lys Val Pro Ala Lys His Ser Tyr Gly Met Leu Phe Cys Ser
           720                 725                 730
   TGC CGG GAC ATC GCC TGC ACA GAG CGG AGG CGA CAG ACC ATC GTG CCT     336
   Cys Arg Asp Ile Ala Cys Thr Glu Arg Arg Arg Gln Thr Ile Val Pro
       735                 740                 745
   GTG TGC TCC TAT GAA GAG AGG GAG AAG CCC AAC TGT TTG AAT TTG CAG     384
   Val Cys Ser Tyr Glu Glu Arg Glu Lys Pro Asn Cys Leu Asn Leu Gln
   750                 755                 760                 765
   GAC TCC TGC AAG ACG AAT TAC ATC TGC AGA TCT CGC CTT GCG GAT TTT     432
   Asp Ser Cys Lys Thr Asn Tyr Ile Cys Arg Ser Arg Leu Ala Asp Phe
                   770                 775                 780
   TTT ACC AAC TGC CAG CCA GAG TCA AGG TCT GTC AGC AGC TGT CTA AAG     480
   Phe Thr Asn Cys Gln Pro Glu Ser Arg Ser Val Ser Ser Cys Leu Lys
               785                 790                 795
   GAA AAC TAC GCT GAC TGC CTC CTC GCC TAC TCG GGG CTT ATT GGC ACA     528
   Glu Asn Tyr Ala Asp Cys Leu Leu Ala Tyr Ser Gly Leu Ile Gly Thr
           800                 805                 810
        GTC ATG ACC CCC AAC TAC ATA GAC TCC ACT AGC CTC AGT GTG CCC CCA
   576
        Val Met Thr Pro Asn Tyr Ile Asp Ser Ser Ser Leu Ser Val Ala Pro
            815                 820                 825
        TGG TGT GAC TGC AGC AAC AGT GGG AAC GAC CTA GAA GAG TGC TTG AAA
   624
        Trp Cys Asp Cys Ser Asn Ser Gly Asn Asp Leu Glu Glu Cys Leu Lys
        830                 835                 840                 845
        TTT TTG AAT TTC TTC AAG GAC AAT ACA TGT CTT AAA AAT GCA ATT CAA
   672
        Phe Leu Asn Phe Phe Lys Asp Asn Thr Cys Leu Lys Asn Ala Ile Gln
                        850                 855                 860
        GCC TTT GGC AAT GGC TCC GAT GTG ACC GTG TGG CAG CCA GCC TTC CCA
   720
        Ala Phe Gly Asn Gly Ser Asp Val Thr Val Trp Gln Pro Ala Phe Pro
                    865                 870                 875
        GTA CAG ACC ACC ACT GCC ACT ACC ACC ACT GCC CTC CGG GTT AAG AAC
   768
        Val Gln Thr Thr Thr Ala Thr Thr Thr Thr Ala Leu Arg Val Lys Asn
                880                 885                 890
        AAG CCC CTG GGG CCA GCA GGG TCT GAG AAT GAA ATT CCC ACT CAT GTT
   816
        Lys Pro Leu Gly Pro Ala Gly Ser Glu Asn Glu Ile Pro Thr His Val
            895                 900                 905
        TTG CCA CCG TGT GCA AAT TTA CAG GCA CAG AAG CTG AAA TCC AAT GTG
   864
        Leu Pro Pro Cys Ala Asn Leu Gln Ala Gln Lys Leu Lys Ser Asn Val
        910                 915                 920                 925
        TCC GGC AAT ACA CAC CTC TGT ATT TCC AAT GGT AAT TAT GAA AAA GAA
   912
        Ser Gly Asn Thr His Leu Cys Ile Ser Asn Gly Asn Tyr Glu Lys Glu
                        930                 935                 940
        GGT CTC GGT GCT TCC AGC CAC ATA ACC ACA AAA TCA ATG GCT GCT CCT
   960
        Gly Leu Gly Ala Ser Ser His Ile Thr Thr Lys Ser Met Ala Ala Pro
                    945                 950                 955
        CCA AGC TGT GGT CTG AGC CCA CTG CTG GTC CTG GTG GTA ACC GCT CTG
   1008
        Pro Ser Cys Gly Leu Ser Pro Leu Leu Val Leu Val Val Thr Ala Leu
                960                 965                 970
        TCC ACC CTA TTA TCT TTA ACA GAA ACA TCA TAGCTGCATT AAAAAAATAC
   1058
        Ser Thr Leu Leu Ser Leu Thr Glu Thr Ser
            975                 980
        AATATGGACA TGTAAAAAGA CAAAAACCAA GTTATCTGTT TCCTGTTCTC TTGTATAGCT
   1118
        GAAATTCCAG TTTAGGAGCT CAGTTGAGAA ACAGTTCCAT TCAACTGGAA CATTTTTTTT
   1178
        TTTTCCTTTT AAGAAAGCTT CTTGTGATCC TTCGGGGCTT CTGTG
   1223
(2) INFORMATION FOR SEQ ID NO:9:
   (i) SEQUENCE CHARACTERISTICS:
      (A) LENGTH: 346 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Leu Leu Glu Asp Ser Pro Tyr Glu Pro Val Asn Ser Arg Leu Ser Asp
    1               5                  10                  15
   Ile Phe Arg Val Val Pro Phe Ile Ser Val Glu His Ile Pro Lys Gly
               20                  25                  30
   Asn Asn Cys Leu Asp Ala Ala Lys Ala Cys Asn Leu Asp Asp Ile Cys
           35                  40                  45
   Lys Lys Tyr Arg Ser Ala Tyr Ile Thr Pro Cys Thr Thr Ser Val Ser
       50                  55                  60
   Asn Asp Val Cys Asn Arg Arg Lys Cys His Lys Ala Leu Arg Gln Phe
   65                  70                  75                  80
   Phe Asp Lys Val Pro Ala Lys His Ser Tyr Gly Met Leu Phe Cys Ser
                   85                  90                  95
   Cys Arg Asp Ile Ala Cys Thr Glu Arg Arg Arg Gln Thr Ile Val Pro
               100                 105                 110
   Val Cys Ser Tyr Glu Glu Arg Glu Lys Pro Asn Cys Leu Asn Leu Gln
           115                 120                 125
   Asp Ser Cys Lys Thr Asn Tyr Ile Cys Arg Ser Arg Leu Ala Asp Phe
       130                 135                 140
   Phe Thr Asn Cys Gln Pro Glu Ser Arg Ser Val Ser Ser Cys Leu Lys
   145                 150                 155                 160
   Glu Asn Tyr Ala Asp Cys Leu Leu Ala Tyr Ser Gly Leu Ile Gly Thr
                   165                 170                 175
   Val Met Thr Pro Asn Tyr Ile Asp Ser Ser Ser Leu Ser Val Ala Pro
               180                 185                 190
   Trp Cys Asp Cys Ser Asn Ser Gly Asn Asp Leu Glu Glu Cys Leu Lys
           195                 200                 205
   Phe Leu Asn Phe Phe Lys Asp Asn Thr Cys Leu Lys Asn Ala Ile Gln
       210                 215                 220
   Ala Phe Gly Asn Gly Ser Asp Val Thr Val Trp Gln Pro Ala Phe Pro
   225                 230                 235                 240
   Val Gln Thr Thr Thr Ala Thr Thr Thr Thr Ala Leu Arg Val Lys Asn
                   245                 250                 255
   Lys Pro Leu Gly Pro Ala Gly Ser Glu Asn Glu Ile Pro Thr His Val
               260                 265                 270
   Leu Pro Pro Cys Ala Asn Leu Gln Ala Gln Lys Leu Lys Ser Asn Val
           275                 280                 285
   Ser Gly Asn Thr His Leu Cys Ile Ser Asn Gly Asn Tyr Glu Lys Glu
       290                 295                 300
   Gly Leu Gly Ala Ser Ser His Ile Thr Thr Lys Ser Met Ala Ala Pro
   305                 310                 315                 320
   Pro Ser Cys Gly Leu Ser Pro Leu Leu Val Leu Val Val Thr Ala Leu
                   325                 330                 335
   Ser Thr Leu Leu Ser Leu Thr Glu Thr Ser
               340                 345
(2) INFORMATION FOR SEQ ID NO: 10:
   (i) SEQUENCE CHARACTERISTICS:
      (A) LENGTH: 1682 base pairs
      (8) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 118..1497
   (xi) SEQUENCE DESCRIPTION: SEQ ID NO:10:
   GGGCGGCCAG AGCAGCACAG CTGTCCGGGG ATCGCTGCAT GCTGAGCTCC CTCGGCAAGA
   60
   CCCAGCGGCG GCTCGGGATT TTTTTGGGGG GGCGGGGACC AGCCCCGCGC CGGCACC
   117
   ATG TTC CTG GCG ACC CTG TAC TTC GCG CTG CCG CTC TTG GAC TTG CTC
   165
   Met Phe Leu Ala Thr Leu Tyr Phe Ala Leu Pro Leu Leu Asp Leu Leu
   350 355 360
   CTG TCG GCC GAA GTG AGC GGC GGA GAC CGC CTG GAT TGC GTG AAA GCC
   213
   Leu Ser Ala Glu Val Ser Gly Gly Asp Arg Leu Asp Cys Val Lys Ala
   365 370 375
   AGT GAT CAG TGC CTG AAG GAG CAG AGC TGC AGC ACC AAG TAC CGC ACG
   261
   Ser Asp Gln Cys Leu Lys Glu Gln Ser Cys Ser Thr Lys Tyr Arg Thr
   380 385 390
   CTA AGG CAG TGC GTG GCG GGC AAG GAG ACC AAC TTC AGC CTG GCA TCC
   309
   Leu Arg Gln Cys Val Ala Gly Lys Glu Thr Asn Phe Ser Leu Ala Ser
   395 400 405 410
   GGC CTG GAG GCC AAG GAT GAG TGC CGC AGC GCC ATG GAG GCC CTG AAG
   357
   Gly Leu Glu Ala Lys Asp Glu Cys Arg Ser Ala Met Glu Ala Leu Lys
   415 420 425
   CAG AAG TCG CTC TAC AAC TGC CGC TGC AAG CGG GGT ATG AAG AAG GAG
   405
   Gln Lys Ser Leu Tyr Asn Cys Arg Cys Lys Arg Gly Met Lys Lys Glu
                   430                       435                       440
           AAG AAC TGC CTG CGC ATT TAC TGG AGC ATG TAC CAG AGC CTG CAG GGA
   453
           Lys Asn Cys Leu Arg Ile Tyr Trp Ser Met Tyr Gln Ser Leu Gln Gly
                   445                     450                     455
           AAT GAT CTG CTG GAG GAT TCC CCA TAT GAA CCA GTT AAC AGC AGA TTG
   501
           Asn Asp Leu Leu Glu Asp Ser Pro Tyr Glu Pro Val Asn Ser Arg Leu
               460                 465                     470
           TCA GAT ATA TTC CGG GTG GTC CCA TTC ATA TCA GTG GAG CAC ATT CCC
   549
           Ser Asp Ile Phe Arg Val Val Pro Phe Ile Ser Val Glu His Ile Pro
           475                 480                 485                 490
           AAA GGG AAC AAC TGC CTG GAT GCA GCG AAG GCC TGC AAC CTC GAC GAC
   597
           Lys Gly Asn Asn Cys Leu Asp Ala Ala Lys Ala Cys Asn Leu Asp Asp
                           495                 500                 505
           ATT TGC AAG AAG TAC AGG TCG GCG TAC ATC ACC CCG TGC ACC ACC AGC
   645
           Ile Cys Lys Lys Tyr Arg Ser Ala Tyr Ile Thr Pro Cys Thr Thr Ser
                       510                 515                 520
           GTG TCC AAC GAT GTC TGC AAC CGC CGC AAG TGC CAC AAG GCC CTC CGG
   693
           Val Ser Asn Asp Val Cys Asn Arg Arg Lys Cys His Lys Ala Leu Arg
                   525                 530                 535
           CAG TTC TTT GAC AAG GTC CCG GCC AAG CAC AGC TAC GGA ATG CTC TTC
   741
           Gln Phe Phe Asp Lys Val Pro Ala Lys His Ser Tyr Gly Met Leu Phe
               540                 545                 550
           TGC TCC TGC CGG GAC ATC GCC TGC ACA GAG CGG AGG CGA CAG ACC ATC
   789
           Cys Ser Cys Arg Asp Ile Ala Cys Thr Glu Arg Arg Arg Gln Thr Ile
           555                 560                 565                 570
           GTG CCT GTG TGC TCC TAT GAA GAG AGG GAG AAG CCC AAC TGT TTG AAT
   837
           Val Pro Val Cys Ser Tyr Glu Glu Arg Glu Lys Pro Asn Cys Leu Asn
                           575                 580                 585
           TTG CAG GAC TCC TGC AAG ACG AAT TAC ATC TGC AGA TCT CGC CTT GCG
   885
           Leu Gln Asp Ser Cys Lys Thr Asn Tyr Ile Cys Arg Ser Arg Leu Ala
                       590                 595                 600
           GAT TTT TTT ACC AAC TGC CAG CCA GAG TCA AGC TCT GTC AGC AGC TGT
   933
           Asp Phe Phe Thr Asn Cys Gln Pro Glu Ser Arg Ser Val Ser Ser Cys
                   605                 610                 615
           CTA AAG GAA AAC TAC GCT GAC TGC CTC CTC GCC TAC TCG GGG CTT ATT
   981
           Leu Lys Glu Asn Tyr Ala Asp Cys Leu Leu Ala Tyr Ser Gly Leu Ile
               620                 625                 630
           GGC ACA GTC ATG ACC CCC AAC TAC ATA GAC TCC AGT AGC CTC AGT GTG
   1029
           Gly Thr Val Met Thr Pro Asn Tyr Ile Asp Ser Ser Ser Leu Ser Val
   635                 640                 645                 650
   GCC CCA TGG TGT GAC TGC AGC AAC AGT GGG AAC GAC CTA GAA GAG TGC
   1077
   Ala Pro Trp Cys Asp Cys Ser Asn Ser Gly Asn Asp Leu Glu Glu Cys
                   655                 660                 665
   TTG AAA TTT TTG AAT TTC TTC AAG GAC AAT ACA TCT CTT AAA AAT GCA
   1125
   Leu Lys Phe Leu Asn Phe Phe Lys Asp Asn Thr Cys Leu Lys Asn Ala
               670                 675                 680
   ATT CAA GCC TTT GGC AAT GGC TCC GAT GTG ACC GTG TGG CAG CCA GCC
   1173
   Ile Gln Ala Phe Gly Asn Gly Ser Asp Val Thr Val Trp Gln Pro Ala
           685                 690                 695
   TTC CCA GTA CAG ACC ACC ACT GCC ACT ACC ACC ACT GCC CTC CGG GTT
   1221
   Phe Pro Val Gln Thr Thr Thr Ala Thr Thr Thr Thr Ala Leu Arg Val
       700                 705                 710
   AAG AAC AAG CCC CTG GGG CCA GCA GGG TCT GAG AAT GAA ATT CCC ACT
   1269
   Lys Asn Lys Pro Leu Gly Pro Ala Gly Ser Glu Asn Glu Ile Pro Thr
   715                 720                 725                 730
   CAT GTT TTG CCA CCG TGT GCA AAT TTA CAG GCA CAG AAG CTG AAA TCC
   1317
   His Val Leu Pro Pro Cys Ala Asn Leu Gln Ala Gln Lys Leu Lys Ser
                   735                 740                 745
   AAT GTG TCG GGC AAT ACA CAC CTC TGT ATT TCC AAT GGT AAT TAT GAA
   1365
   Asn Val Ser Gly Asn Thr His Leu Cys Ile Ser Asn Gly Asn Tyr Glu
               750                 755                 760
   AAA GAA GGT CTC GGT GCT TCC AGC CAC ATA ACC ACA AAA TCA ATG GCT
   1413
   Lys Glu Gly Leu Gly Ala Ser Ser His Ile Thr Thr Lys Ser Met Ala
           765                 770                 775
   GCT CCT CCA AGC TGT GGT CTG AGC CCA CTG CTG GTC CTG GTG GTA ACC
   1461
   Ala Pro Pro Ser Cys Gly Leu Ser Pro Leu Leu Val Leu Val Val Thr
       780                 785                 790
   GCT CTG TCC ACC CTA TTA TCT TTA ACA GAA ACA TCA TAGCTGCATT
   1507
   Ala Leu Ser Thr Leu Leu Ser Leu Thr Glu Thr Ser
   795                 800                 805
   AAAAAAATAC AATATGGACA TGTAAAAAGA CAAAAACCAA GTTATCTGTT TCCTGTTCTC
   1567
   TTGTATAGCT GAAATTCCAG TTTAGGAGCT CAGTTGAGAA ACAGTTCCAT TCAACTGGAA
   1627
   CATTTTTTTT TTTTCCTTTT AAGAAAGCTT CTTGTGATCC TTCGGGGCTT CTGTG
   1682
(2) INFORMATION FOR SEQ ID NO:11:
   (i) SEQUENCE CHARACTERISTICS:
      (A) LENGTH: 460 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Phe Leu Ala Thr Leu Tyr Phe Ala Leu Pro Leu Leu Asp Leu Leu
    1               5                  10                  15
   Leu Ser Ala Glu Val Ser Gly Gly Asp Arg Leu Asp Cys Val Lys Ala
               20                  25                  30
   Ser Asp Gln Cys Leu Lys Glu Gln Ser Cys Ser Thr Lys Tyr Arg Thr
           35                  40                  45
   Leu Arg Gln Cys Val Ala Gly Lys Glu Thr Asn Phe Ser Leu Ala Ser
       50                  55                  60
   Gly Leu Glu Ala Lys Asp Glu Cys Arg Ser Ala Met Glu Ala Leu Lys
   65                  70                  75                  80
   Gln Lys Ser Leu Tyr Asn Cys Arg Cys Lys Arg Gly Met Lys Lys Glu
                   85                  90                  95
   Lys Asn Cys Leu Arg Ile Tyr Trp Ser Met Tyr Gln Ser Leu Gln Gly
               100                 105                 110
   Asn Asp Leu Leu Glu Asp Ser Pro Tyr Glu Pro Val Asn Ser Arg Leu
           115                 120                 125
   Ser Asp Ile Phe Arg Val Val Pro Phe Ile Ser Val Glu His Ile Pro
       130                 135                 140
   Lys Gly Asn Asn Cys Leu Asp Ala Ala Lys Ala Cys Asn Leu Asp Asp
   145                 150                 155                 160
   Ile Cys Lys Lys Tyr Arg Ser Ala Tyr Ile Thr Pro Cys Thr Thr Ser
                   165                 170                 175
   Val Ser Asn Asp Val Cys Asn Arg Arg Lys Cys His Lys Ala Leu Arg
               180                 185                 190
   Gln Phe Phe Asp Lys Val Pro Ala Lys His Ser Tyr Gly Met Leu Phe
           195                 200                 205
   Cys Ser Cys Arg Asp Ile Ala Cys Thr Glu Arg Arg Arg Gln Thr Ile
       210                 215                 220
   Val Pro Val Cys Ser Tyr Glu Glu Arg Glu Lys Pro Asn Cys Leu Asn
   225                 230                 235                 240
   Leu Gln Asp Ser Cys Lys Thr Asn Tyr Ile Cys Arg Ser Arg Leu Ala
                   245                 250                 255
   Asp Phe Phe Thr Asn Cys Gln Pro Glu Ser Arg Ser Val Ser Ser Cys
               260                 265                 270
   Leu Lys Glu Asn Tyr Ala Asp Cys Leu Leu Ala Tyr Ser Gly Leu Ile
           275                 280                 285
   Gly Thr Val Met Thr Pro Asn Tyr Ile Asp Ser Ser Ser Leu Ser Val
       290                 295                 300
   Ala Pro Trp Cys Asp Cys Ser Asn Ser Gly Asn Asp Leu Glu Glu Cys
   305                 310                 315                 320
   Leu Lys Phe Leu Asn Phe Phe Lys Asp Asn Thr Cys Leu Lys Asn Ala
                   325                 330                 335
   Ile Gln Ala Phe Gly Asn Gly Ser Asp Val Thr Val Trp Gln Pro Ala
               340                 345                 350
   Phe Pro Val Gln Thr Thr Thr Ala Thr Thr Thr Thr Ala Leu Arg Val
           355                 360                 365
   Lys Asn Lys Pro Leu Gly Pro Ala Gly Ser Glu Asn Glu Ile Pro Thr
       370                 375                 380
   His Val Leu Pro Pro Cys Ala Asn Leu Gln Ala Gln Lys Leu Lys Ser
   385                 390                 395                 400
   Asn Val Ser Gly Asn Thr His Leu Cys Ile Ser Asn Gly Asn Tyr Glu
                   405                 410                 415
   Lys Glu Gly Leu Gly Ala Ser Ser His Ile Thr Thr Lys Ser Met Ala
               420                 425                 430
   Ala Pro Pro Ser Cys Gly Leu Ser Pro Leu Leu Val Leu Val Val Thr
           435                 440                 445
   Ala Leu Ser Thr Leu Leu Ser Leu Thr Glu Thr Ser
       450                 455                 460
(2) INFORMATION FOR SEQ ID NO:12:
   (i) SEQUENCE CHARACTERISTICS:
      (A) LENGTH: 1888 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 25..1416
   (xi) SEQUENCE DESCRIPTION: SEQ ID NO:1.2:
(2) INFORMATION FOR SEQ ID NO:13:
   (i) SEQUENCE CHARACTERISTICS:
      (A) LENGTH: 454 amino acids
      (B) TYPE: amino acid
      (D) TOPOLOGY: linear
   (ii) MOLECULE TYPE: protein
   (xi) SEQUENCE DESCRIPTION: SEQ ID NO:13:
(2) INFORMATION FOR SEQ ID NO:14:
   (i) SEQUENCE CHARACTERISTICS:
      (A) LENGTH: 1878 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 205. 1242
   (xi) SEQUENCE DESCRIPTION: SEQ ID NO:14:
   CGCGGCGCCC AGCGCAGGCA GAGCGCTGTC GCATCCCGGG CGTCCACCCG CCATGGGGCT
   60
   CTCCTGGAGC CCGCGACCTC CACTGCTGAT GATCCTGCTA CTGGTGCTGT CGTTGTGGCT
   120
   GCCACTTGGA GCAGGAAACT CCCTTGCCAC AGAGAACAGG TTTGTGAACA GCTGTACCCA
   180
   GGCCAGAAAG AAATGCGAGG CTAA TCC CGC TTG CAA GGC TGC CTA CCA GCA
   231
                              Ser Arg Leu Gln Gly Cys Leu Pro Ala
                              465                 470
   CCT GGG CTC CTG CAC CTC CAG TTA AGC AGG CCG CTG CCC TTA GAG GAG
   279
   Pro Gly Leu Leu His Leu Gln Leu Ser Arg Pro Leu Pro Leu Glu Glu
       475                 480                 485
   TCT GCC ATG TCT GCA GAC TGC CTA GAG GCA GCA GAA CAA CTC AGG AAC
   327
   Ser Ala Met Ser Ala Asp Cys Leu Glu Ala Ala Glu Gln Leu Arg Asn
   490                 495                 500                 505
   AGC TCT CTG ATA GAC TGC AGG TGC CAT CGG CGC ATG AAG CAC CAA GCT
   375
   Ser Ser Leu Ile Asp Cys Arg Cys His Arg Arg Met Lys His Gln Ala
                   510                 515                 520
   ACC TGT CTG GAC ATT TAT TGG ACC GTT CAC CCT GCC CGA AGC CTT GGT
   423
   Thr Cys Leu Asp Ile Tyr Trp Thr Val His Pro Ala Arg Ser Leu Gly
               525                 530                 535
   GAC TAC GAG TTG GAT GTC TCA CCC TAT GAA GAC ACA GTG ACC AGC AAA
   471
   Asp Tyr Glu Leu Asp Val Ser Pro Tyr Glu Asp Thr Val Thr Ser Lys
           540                 545                 550
   CCC TGG AAA ATG AAT CTT AGC AAG TTG AAC ATG CTC AAA CCA GAC TCG
   519
   Pro Trp Lys Met Asn Leu Ser Lys Leu Asn Met Leu Lys Pro Asp Ser
       555                 560                 565
   GAC CTC TGC CTC AAA TTT GCT ATG CTG TGT ACT CTT CAC GAC AAG TGT
   567
   Asp Leu Cys Leu Lys Phe Ala Met Leu Cys Thr Leu His Asp Lys Cys
   570                 575                 580                 585
        GAC CGC CTG CGC AAG GCC TAC GGG GAG GCA TGC TCA GGC ATC CGC TGC
   615
        Asp Arg Leu Arg Lys Ala Tyr Gly Glu Ala Cys Ser Gly Ile Arg Cys
                        590                 595                 600
        CAG CGC CAC CTC TGC CTA GCC CAG CTG CGC TCC TTC TTT GAG AAG GCA
   663
        Gln Arg His Leu Cys Leu Ala Gln Leu Arg Ser Phe Phe Glu Lys Ala
                    605                 610                 615
        GCA GAG TCC CAC GCT CAG GGT CTG CTG CTG TGT CCC TGT GCA CCA GAA
   711
        Ala Glu Ser His Ala Gln Gly Leu Leu Leu Cys Pro Cys Ala Pro Glu
                620                 625                 630
        GAT GCG GGC TGT GGG GAG CGG CGG CGT AAC ACC ATC GCC CCC AGT TGC
   759
        Asp Ala Gly Cys Gly Glu Arg Arg Arg Asn Thr Ile Ala Pro Ser Cys
            635                 640                 645
        GCC CTG CCT TCT GTA ACC CCC AAT TGC CTG GAT CTG CGG AGC TTC TGC
   807
        Ala Leu Pro Ser Val Thr Pro Asn Cys Leu Asp Leu Arg Ser Phe Cys
        650                 655                 660                 665
        CGT GCG GAC CCT TTG TGC AGA TCA CGC CTG ATG GAC TTC CAG ACC CAC
   855
        Arg Ala Asp Pro Leu Cys Arg Ser Arg Leu Met Asp Phe Gln Thr His
                        670                 675                 680
        TGT CAT CCT ATG GAC ATC CTT GGG ACT TGT GCA ACT GAG CAG TCC AGA
   903
        Cys His Pro Met Asp Ile Leu Gly Thr Cys Ala Thr Glu Gln Ser Arg
                    685                 690                 695
        TGT CTG CGG GCA TAC CTG GGG CTG ATT GGG ACT GCC ATG ACC CCA AAC
   951
        Cys Leu Arg Ala Tyr Leu Gly Leu Ile Gly Thr Ala Met Thr Pro Asn
                700                 705                 710
        TTC ATC AGC AAG GTC AAC ACT ACT GTT GCC TTA AGC TGC ACC TGC CGA
   999
        Phe Ile Ser Lys Val Asn Thr Thr Val Ala Leu Ser Cys Thr Cys Arg
            715                 720                 725
        GGC AGC GGC AAC CTA CAG GAC GAG TGT GAA CAG CTG GAA AGG TCC TTC
   1047
        Gly Ser Gly Asn Leu Gln Asp Glu Cys Glu Gln Leu Glu Arg Ser Phe
        730                 735                 740                 745
        TCC CAG AAC CCC TGC CTC GTG GAG GCC ATT GCA GCT AAG ATG CGT TTC
   1095
        Ser Gln Asn Pro Cys Leu Val Glu Ala Ile Ala Ala Lys Met Arg Phe
                        750                 755                 760
        CAC AGA CAG CTC TTC TCC CAG GAC TGG GCA GAC TCT ACT TTT TCA GTG
   1143
        His Arg Gln Leu Phe Ser Gln Asp Trp Ala Asp Ser Thr Phe Ser Val
                    765                 770                 775
        GTG CAG CAG CAG AAC AGC AAC CCT GCT CTG AGA CTG CAG CCC AGG CTA
   1191
        Val Gln Gln Gln Asn Ser Asn Pro Ala Leu Arg Leu Gln Pro Arg Leu
                780                 785                 790
   CCC ATT CTT TCT TTC TCC ATC CTT CCC TTG ATT GTG CTG CAG ACC CTC
   1239
   Pro Ile Leu Ser Phe Ser Ile Leu Pro Leu Ile Leu Leu Gln Thr Leu
   795 800 805
   TGG TAGCTGGGCT TCCTCAGGGT CCTTTGTCCT CTCCACCACA CCCAGACTGA
   1292
   Trp
   810
   TTTGCAGCCT GTGGTGGGAG AGAACTCGCC AGCCTGTGGA AGAAGACGCA GCGTGCTACA
   1352
   CAGCAACCCG GAACCAACCA GGCATTCCCC AGCACATCCC GTCTGCTCCA GAAGAGGTCT
   1412
   TAGAAGTGAG GGCTGTGACC CTTCCGATCC TGAGCGGCTA GTTTTCAAAC CTCCCTTGCC
   1472
   CCTGCTTCCT TCTGGCTCAG GCTGCTCCTC CTTAGGACTT TGTGGGTCCA GTTTTGCCTT
   1532
   CTGTTCTGAT GGTGATTAGC GGCTCACCTC CAGCGCTTCT TCCTGTTTCC CAGGACCACC
   1592
   CAGAGGCTAA GGAATCAGTC ATTCCCTGTT GCCTTCTCCA GGAAGGCAGG CTAAGGGTTC
   1652
   TGAGGTGACT GAGAAAAATG TTTCCTTTGT GTGGAAGGCT GGTGCTCCAG CCTCCACGTC
   1712
   CCTCTGAATG GAAGATAAAA ACCTGCTGGT GTCTTGACTG CTCTGCCAGG CAATCCTGAA
   1772
   CATTTGGGCA TGAAGAGCTA AAGTCTTTGG GTCTTGTTTA ACTCCTATTA CTGTCCCCAA
   1832
   ATTCCCCTAG TCCCTTGGGT CATGATTAAA CATTTTGACT TAAAAA
   1878
(2) INFORMATION FOR SEQ ID NO:15:
   (i) SEQUENCE CHARACTERISTICS:
      (A) LENGTH: 346 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Ser Arg Leu Gln Gly Cys Leu Pro Ala Pro Gly Leu Leu His Leu Gln
   1               5                   10                  15
   Leu Ser Arg Pro Leu Pro Leu Glu Glu Ser Ala Met Ser Ala Asp Cys
               20                  25                  30
   Leu Glu Ala Ala Glu Gln Leu Arg Asn Ser Ser Leu Ile Asp Cys Arg
           35                  40                  45
   Cys His Arg Arg Met Lys His Gln Ala Thr Cys Leu Asp Ile Tyr Trp
       50                  55                  60
   Thr Val His Pro Ala Arg Ser Leu Gly Asp Tyr Glu Leu Asp Val Ser
   65                  70                  75                  80
   Pro Tyr Glu Asp Thr Val Thr Ser Lys Pro Trp Lys Met Asn Leu Ser
                   85                  90                  95
   Lys Leu Asn Met Leu Lys Pro Asp Ser Asp Leu Cys Leu Lys Phe Ala
               100                 105                 110
   Met Leu Cys Thr Leu His Asp Lys Cys Asp Arg Leu Arg Lys Ala Tyr
           115                 120                 125
   Gly Glu Ala Cys Ser Gly Ile Arg Cys Gln Arg His Leu Cys Leu Ala
       130                 135                 140
   Gln Leu Arg Ser Phe Phe Glu Lys Ala Ala Glu Ser His Ala Gln Gly
   145                 150                 155                 160
   Leu Leu Leu Cys Pro Cys Ala Pro Glu Asp Ala Gly Cys Gly Glu Arg
                   165                 170                 175
   Arg Arg Asn Thr Ile Ala Pro Ser Cys Ala Leu Pro Ser Val Thr Pro
               180                 185                 190
   Asn Cys Leu Asp Leu Arg Ser Phe Cys Arg Ala Asp Pro Leu Cys Arg
           195                 200                 205
   Ser Arg Leu Met Asp Phe Gln Thr His Cys His Pro Met Asp Ile Leu
       210                 215                 220
   Gly Thr Cys Ala Thr Glu Gln Ser Arg Cys Leu Arg Ala Tyr Leu Gly
   225                 230                 235                 240
   Leu Ile Gly Thr Ala Met Thr Pro Asn Phe Ile Ser Lys Val Asn Thr
                   245                 250                 255
   Thr Val Ala Leu Ser Cys Thr Cys Arg Gly Ser Gly Asn Leu Gln Asp
               260                 265                 270
   Glu Cys Glu Gln Leu Glu Arg Ser Phe Ser Gln Asn Pro Cys Leu Val
           275                 280                 285
   Glu Ala Ile Ala Ala Lys Met Arg Phe His Arg Gln Leu Phe Ser Gln
       290                 295                 300
   Asp Trp Ala Asp Ser Thr Phe Ser Val Val Gln Gln Gln Asn Ser Asn
   305                 310                 315                 320
   Pro Ala Leu Arg Leu Gln Pro Arg Leu Pro Ile Leu Ser Phe Ser Ile
                   325                 330                 335
   Leu Pro Leu Ile Leu Leu Gln Thr Leu Trp
               340                 345
(2) INFORMATION FOR SEQ ID NO:16:
   (i) SEQUENCE CHARACTERISTICS:
      (A) LENGTH: 1889 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 41..1231
   (xi) SEQUENCE DESCRIPTION: SEQ ID NO:16:
       CGCAGGCAGA GCGCTGTCGC ATCCCGGGCG TCCACCCGCC ATG GGG CTC TCC TGG
   55
                                                   Met Gly Leu Ser Trp
                                                               350
       AGC CCG CGA CCT CCA CTG CTG ATG ATC CTG CTA CTG GTG CTG TCG TTG
   103
       Ser Pro Arg Pro Pro Leu Leu Met Ile Leu Leu Leu Val Leu Ser Leu
                   355                 360                 365
       TGG CTG CCA CTT GGA GCA GGA AAC TCC CTT GCC ACA GAG AAC AGG TTT
   151
       Trp Leu Pro Leu Gly Ala Gly Asn Ser Leu Ala Thr Glu Asn Arg Phe
               370                 375                 380
       GTG AAC AGC TGT ACC CAG GCC AGA AAG AAA TGC GAG GCT AAT CCC GCT
   199
       Val Asn Ser Cys Thr Gln Ala Arg Lys Lys Cys Glu Ala Asn Pro Ala
           385                 390                 395
       TGC AAG GCT GCC TAC CAG CAC CTG GGC TCC TGC ACC TCC AGT TTA AGC
   247
       Cys Lys Ala Ala Tyr Gln His Leu Gly Ser Cys Thr Ser Ser Leu Ser
       400                 405                 410                 415
       AGG CCG CTG CCC TTA GAG GAG TCT GCC ATG TCT GCA GAC TGC CTA GAG
   295
       Arg Pro Leu Pro Leu Glu Glu Ser Ala Met Ser Ala Asp Cys Leu Glu
                       420                 425                 430
       GCA GCA GAA CAA CTC AGG AAC AGC TCT CTG ATA GAC TGC AGG TGC CAT
   343
       Ala Ala Glu Gln Leu Arg Asn Ser Ser Leu Ile Asp Cys Arg Cys His
                   435                 440                 445
       CGG CGC ATG AAG CAC CAA GCT ACC TGT CTG GAC ATT TAT TGG ACC GTT
   391
       Arg Arg Met Lys His Gln Ala Thr Cys Leu Asp Ile Tyr Trp Thr Val
               450                 455                 460
       CAC CCT GCC CGA AGC CTT GGT GAC TAC GAG TTG GAT GTC TCA CCC TAT
   439
       His Pro Ala Arg Ser Leu Gly Asp Tyr Glu Leu Asp Val Ser Pro Tyr
           465                 470                 475
       GAA GAC ACA GTG ACC AGC AAA CCC TGG AAA ATG AAT CTT AGC AAG TTG
   487
       Glu Asp Thr Val Thr Ser Lys Pro Trp Lys Met Asn Leu Ser Lys Leu
       480                 485                 490                 495
       AAC ATG CTC AAA CCA GAC TCG GAC CTC TGC CTC AAA TTT GCT ATG CTG
   535
       Asn Met Leu Lys Pro Asp Ser Asp Leu Cys Leu Lys Phe Ala Met Leu
                       500                 505                 510
       TGT ACT CTT CAC GAC AAG TGT GAC CGC CTG CGC AAG GCC TAC GGG GAG
   583
       Cys Thr Leu His Asp Lys Cys Asp Arg Leu Arg Lys Ala Tyr Gly Glu
                   515                 520                 525
       GCA TGC TCA GGG ATC CGC TGC CAG CGC CAC CTC TGC CTA GCC CAG CTG
   631
       Ala Cys Ser Gly Ile Arg Cys Gln Arg His Leu Cys Leu Ala Gln Leu
               530                 535                 540
       CGC TCC TTC TTT GAG AAG GCA GCA GAG TCC CAC GCT CAG GGT CTG CTG
   679
       Arg Ser Phe Phe Glu Lys Ala Ala Glu Ser His Ala Gln Gly Leu Leu
           545                 550                 555
       CTG TGT CCC TGT GCA CCA GAA GAT GCG GGC TGT GGG GAG CGG CGG CGT
   727
       Leu Cys Pro Cys Ala Pro Glu Asp Ala Gly Cys Gly Glu Arg Arg Arg
       560                 565                 570                 575
       AAC ACC ATC GCC CCC AGT TGC GCC CTG CCT TCT GTA ACC CCC AAT TGC
   775
       Asn Thr Ile Ala Pro Ser Cys Ala Leu Pro Ser Val Thr Pro Asn Cys
                       580                 585                 590
       CTG GAT CTG CGG AGC TTC TGC CGT GCG GAC CCT TTG TGC AGA TCA CGC
   823
       Leu Asp Leu Arg Ser Phe Cys Arg Ala Asp Pro Leu Cys Arg Ser Arg
                   595                 600                 605
       CTG ATG GAC TTC CAG ACC CAC TGT CAT CCT ATG GAC ATC CTT GGG ACT
   871
       Leu Met Asp Phe Gln Thr His Cys His Pro Met Asp Ile Leu Gly Thr
               610                 615                 620
       TGT GCA ACT GAG CAG TCC AGA TGT CTG CGG GCA TAC CTG GGG CTG ATT
   919
       Cys Ala Thr Glu Gln Ser Arg Cys Leu Arg Ala Tyr Leu Gly Leu Ile
           625                 630                 635
       GGG ACT GCC ATG ACC CCA AAC TTC ATC AGC AAG GTC AAC ACT ACT GTT
   967
       Gly Thr Ala Met Thr Pro Asn Phe Ile Ser Lys Val Asn Thr Thr Val
       640                 645                 650                 655
       GCC TTA AGC TGC ACC TGC CGA GGC AGC GGC AAC CTA CAG GAC GAG TGT
   1015
       Ala Leu Ser Cys Thr Cys Arg Gly Ser Gly Asn Leu Gln Asp Glu Cys
                       660                 665                 670
       GAA CAG CTG GAA AGG TCC TTC TCC CAG AAC CCC TGC CTC GTG GAG GCC
   1063
       Glu Gln Leu Glu Arg Ser Phe Ser Gln Asn Pro Cys Leu Val Glu Ala
                   675                 680                 685
       ATT GCA GCT AAG ATG CGT TTC CAC AGA CAG CTC TTC TCC CAG GAC TGG
   1111
       Ile Ala Ala Lys Met Arg Phe His Arg Gln Leu Phe Ser Gln Asp Trp
               690                 695                 700
       GCA GAC TCT ACT TTT TCA GTG GTG CAG CAG CAG AAC AGC AAC CCT GCT
   1159
       Ala Asp Ser Thr Phe Ser Val Val Gln Gln Gln Asn Ser Asn Pro Ala
           705                 710                 715
       CTG AGA CTG CAG CCC AGG CTA CCC ATT CTT TCT TTC TCC ATC CTT CCC
   1207
       Leu Arg Leu Gln Pro Arg Leu Pro Ile Leu Ser Phe Ser Ile Leu Pro
       720                 725                 730                 735
        TTG ATT CTG CTG CAG ACC CTC TGG TAGCTGGGCT TCCTCAGGGT CCTTTGTCCT
   1261
        Leu Ile Leu Leu Gln Thr Leu Trp
                            740
        CTCCACCACA CCCAGACTGA TTTGCAGCCT GTGGTGGGAG AGAACTCGCC AGCCTGTGGA
   1321
        AGAAGACGCA GCGTGCTACA CAGCAACCCG GAACCAACCA GGCATTCCGC AGCACATCCC
   1381
        GTCTGCTCCA GAAGAGGTCT TAGAAGTGAG GGCTGTGACC CTTCCGATCC TGAGCGGCTA
   1441
        GTTTTCAAAC CTCCCTTGCC CCTGCTTCCT TCTGGCTCAG GCTGCTCCTC CTTAGGACTT
   1501
        TGTGGGTCCA GTTTTGCCTT CTGTTCTGAT GGTGATTAGC GGCTCACCTC CAGCGCTTCT
   1561
        TCCTGTTTCC CAGGACCACC CAGAGGCTAA GGAATCAGTC ATTCCCTGTT GCCTTCTCCA
   1621
        GGAAGGCAGG CTAAGGGTTC TGAGGTGACT GAGAAAAATG TTTCCTTTGT GTGGAAGGCT
   1681
        GGTGCTCCAG CCTCCACGTC CCTCTGAATG GAAGATAAAA ACCTGCTGGT GTCTTGACTG
   1741
        CTCTGCCAGG CAATCCTGAA CATTTGGGCA TGAAGAGCTA AAGTCTTTGG GTCTTGTTTA
   1801
        ACTCCTATTA CTGTCCCCAA ATTCCCCTAG TCCCTTGGGT CATGATTAAA CATTTTGACT
   1861
        TAAAAAAAAA AAAAAAAAAA AAAAAAAA
   1889
(2) INFORMATION FOR SEQ ID NO:17:
   (i) SEQUENCE CHARACTERISTICS:
      (A) LENGTH: 397 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Gly Leu Ser Trp Ser Pro Arg Pro Pro Leu Leu Met Ile Leu Leu
    1               5                  10                  15
   Leu Val Leu Ser Leu Trp Leu Pro Leu Gly Ala Gly Asn Ser Leu Ala
               20                  25                  30
   Thr Glu Asn Arg Phe Val Asn Ser Cys Thr Gln Ala Arg Lys Lys Cys
           35                  40                  45
   Glu Ala Asn Pro Ala Cys Lys Ala Ala Tyr Gln His Leu Gly Ser Cys
       50                  55                  60
   Thr Ser Ser Leu Ser Arg Pro Leu Pro Leu Glu Glu Ser Ala Met Ser
   65                  70                  75                  80
   Ala Asp Cys Leu Glu Ala Ala Glu Gln Leu Arg Asn Ser Ser Leu Ile
                   85                  90                  95
   Asp Cys Arg Cys His Arg Arg Met Lys His Gln Ala Thr Cys Leu Asp
               100                 105                 110
   Ile Tyr Trp Thr Val His Pro Ala Arg Ser Leu Gly Asp Tyr Glu Leu
           115                 120                 125
   Asp Val Ser Pro Tyr Glu Asp Thr Val Thr Ser Lys Pro Trp Lys Met
       130                 135                 140
   Asn Leu Ser Lys Leu Asn Met Leu Lys Pro Asp Ser Asp Leu Cys Leu
   145                 150                 155                 160
   Lys Phe Ala Met Leu Cys Thr Leu His Asp Lys Cys Asp Arg Leu Arg
                   165                 170                 175
   Lys Ala Tyr Gly Glu Ala Cys Ser Gly Ile Arg Cys Gln Arg His Leu
               180                 185                 190
   Cys Leu Ala Gln Leu Arg Ser Phe Phe Glu Lys Ala Ala Glu Ser His
           195                 200                 205
   Ala Gln Gly Leu Leu Leu Cys Pro Cys Ala Pro Glu Asp Ala Gly Cys
       210                 215                 220
   Gly Glu Arg Arg Arg Asn Thr Ile Ala Pro Ser Cys Ala Leu Pro Ser
   225                 230                 235                 240
   Val Thr Pro Asn Cys Leu Asp Leu Arg Ser Phe Cys Arg Ala Asp Pro
                   245                 250                 255
   Leu Cys Arg Ser Arg Leu Met Asp Phe Gln Thr His Cys His Pro Met
               260                 265                 270
   Asp Ile Leu Gly Thr Cys Ala Thr Glu Gln Ser Arg Cys Leu Arg Ala
           275                 280                 285
   Tyr Leu Gly Leu Ile Gly Thr Ala Met Thr Pro Asn Phe Ile Ser Lys
       290                 295                 300
   Val Asn Thr Thr Val Ala Leu Ser Cys Thr Cys Arg Gly Ser Gly Asn
   305                 310                 315                 320
   Leu Gln Asp Glu Cys Glu Gln Leu Glu Arg Ser Phe Ser Gln Asn Pro
                   325                 330                 335
   Cys Leu Val Glu Ala Ile Ala Ala Lys Met Arg Phe His Arg Gln Leu
               340                 345                 350
   Phe Ser Gln Asp Trp Ala Asp Ser Thr Phe Ser Val Val Gln Gln Gln
           355                 360                 365
   Asn Ser Asn Pro Ala Leu Arg Leu Gln Pro Arg Leu Pro Ile Leu Ser
       370                 375                 380
   Phe Ser Ile Leu Pro Leu Ile Leu Leu Gln Thr Leu Trp
   385                 390                 395
(2) INFORMATION FOR SEQ ID NO:18:
   (i) SEQUENCE CHARACTERISTICS:
      (A) LENGTH: 1271 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 2...946
   (xi) SEQUENCE DESCRIPTION: SEQ ID NO:18:
        C GGC TAC TGT GAA ACA CCT CAA CTC AGG AAC AGC TCT CTG ATA GGC
   46
          Gly Tyr Cys Glu Thr Pro Gln Leu Arg Asn Ser Ser Leu Ile Gly
                  400                 405                 410
        TGC ATG TGC CAC CGG CGC ATG AAG AAC CAG GTT GCC TGC TTG GAC ATC
   94
        Cys Met Cys His Arg Arg Met Lys Asn Gln Val Ala Cys Leu Asp Ile
                415                 420                 425
        TAT TGG ACC GTT CAC CGT GCC CGC AGC CTT GGT AAC TAT GAG CTG GAT
   142
        Tyr Trp Thr Val His Arg Ala Arg Ser Leu Gly Asn Tyr Glu Leu Asp
            430                 435                 440
        GTC TCC CCC TAT GAA GAC ACA GTG ACC AGC AAA CCC TGG AAA ATG AAT
   190
        Val Ser Pro Tyr Glu Asp Thr Val Thr Ser Lys Pro Trp Lys Met Asn
        445                 450                 455                 460
        CTC AGC AAA CTG AAC ATG CTC AAA CCA GAC TCA GAC CTC TGC CTC AAG
   238
        Leu Ser Lys Leu Asn Met Leu Lys Pro Asp Ser Asp Leu Cys Leu Lys
                        465                 470                 475
        TTT GCC ATG CTG TGT ACT CTC AAT GAC AAG TGT GAC CGG CTG CGC AAG
   286
        Phe Ala Met Leu Cys Thr Leu Asn Asp Lys Cys Asp Arg Leu Arg Lys
                    480                 485                 490
        GCC TAC GGG GAG GCG TGC TCC GGG CCC CAC TGC CAG CGC CAC GTC TGC
   334
        Ala Tyr Gly Glu Ala Cys Ser Gly Pro His Cys Gln Arg His Val Cys
                495                 500                 505
        CTC AGG CAG CTG CTC ACT TTC TTC GAG AAG GCC GCC GAG CCC CAC GCG
   382
        Leu Arg Gln Leu Leu Thr Phe Phe Glu Lys Ala Ala Glu Pro His Ala
            510                 515                 520
        CAG GGC CTG CTA CTG TGC CCA TGT GCC CCC AAC GAC CGG GGC TGC GGG
   430
        Gln Gly Leu Leu Leu Cys Pro Cys Ala Pro Asn Asp Arg Gly Cys Gly
        525                 530                 535                 540
        GAG CGC CGG CGC AAC ACC ATC GCC CCC AAC TGC GCG CTG CCG CCT GTG
   478
        Glu Arg Arg Arg Asn Thr Ile Ala Pro Asn Cys Ala Leu Pro Pro Val
                        545                 550                 555
       GCC CCC AAC TGC CTG GAG CTG CGG CGC CTC TGC TTC TCC GAC CCG CTT
   526
       Ala Pro Asn Cys Leu Glu Leu Arg Arg Leu Cys Phe Ser Asp Pro Leu
                   560                 565                 570
       TGC AGA TCA CGC CTG GTG GAT TTC CAG ACC CAC TGC CAT CCC ATG GAC
   574
       Cys Arg Ser Arg Leu Val Asp Phe Gln Thr His Cys His Pro Met Asp
               575                 580                 585
       ATC CTA GGA ACT TGT GCA ACA GAG CAG TCC AGA TGT CTA CGA GCA TAC
   622
       Ile Leu Gly Thr Cys Ala Thr Glu Gln Ser Arg Cys Leu Arg Ala Tyr
           590                 595                 600
       CTG GGG CTG ATT GGG ACT GCC ATG ACC CCC AAC TTT GTC AGC AAT GTC
   670
       Leu Gly Leu Ile Gly Thr Ala Met Thr Pro Asn Phe Val Ser Asn Val
       605                 610                 615                 620
       AAC ACC AGT GTT GCC TTA AGC TGC ACC TGC CGA GGC AGT GGC AAC CTG
   718
       Asn Thr Ser Val Ala Leu Ser Cys Thr Cys Arg Gly Ser Gly Asn Leu
                       625                 630                 635
       CAG GAG GAG TGT GAA ATG CTG GAA GGG TTC TTC TCC CAC AAC CCC TGC
   766
       Gln Glu Glu Cys Glu Met Leu Glu Gly Phe Phe Ser His Asn Pro Cys
                   640                 645                 650
       CTC ACG GAG GCC ATT GCA GCT AAG ATG CGT TTT CAC AGC CAA CTC TTC
   814
       Leu Thr Glu Ala Ile Ala Ala Lys Met Arg Phe His Ser Gln Leu Phe
               655                 660                 665
       TCC CAG GAC TGG CCA CAC CCT ACC TTT GCT GTG ATG GCA CAC CAG AAT
   862
       Ser Gln Asp Trp Pro His Pro Thr Phe Ala Val Met Ala His Gln Asn
           670                 675                 680
       GAA AAC CCT GCT GTG AGG CCA CAG CCC TGG GTG CCC TCT CTT TTC TCC
   910
       Glu Asn Pro Ala Val Arg Pro Gln Pro Trp Val Pro Ser Leu Phe Ser
       685                 690                 695                 700
       TGC ACG CTT CCC TTG ATT CTG CTC CTG AGC CTA TGG TAGCTGGACT
   956
       Cys Thr Leu Pro Leu Ile Leu Leu Leu Ser Leu Trp
                       705                 710
       TCCCCAGGGC CCTCTTCCCC TCCACCACAC CCAGGTGGAC TTGCAGCCCA CAAGGGGTGA
   1016
       GGAAAGGACA GCAGCAGGAA GGAGGTGCAG TGCGCAGATG AGGGCACAGG AGAAGCTAAG
   1076
       GGTTATGACC TCCAGATCCT TACTGGTCCA CTCCTCATTC CCTCCACCCC ATCTCCACTT
   1136
       CTGATTCATG CTGCCCCTCC TTGGTGGCCA CAATTTAGCC ATGTCATCTG GTGCCTGTGG
   1196
       GCCTTGCTTT ATTCCTATTA TTGTCCTAAA GTCTCTCTGG GCTCTTGGAT CATGATTAAA
   1256
   CCTTTGACTT AAAAA
   1271
(2) INFORMATION FOR SEQ ID NO:19:
   (i) SEQUENCE CHARACTERISTICS:
      (A) LENGTH: 315 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Gly Tyr Cys Glu Thr Pro Gln Leu Arg Asn Ser Ser Leu Ile Gly Cys
    1               5                  10                  15
   Met Cys His Arg Arg Met Lys Asn Gln Val Ala Cys Leu Asp Ile Tyr
               20                  25                  30
   Trp Thr Val His Arg Ala Arg Ser Leu Gly Asn Tyr Glu Leu Asp Val
           35                  40                  45
   Ser Pro Tyr Glu Asp Thr Val Thr Ser Lys Pro Trp Lys Met Asn Leu
       50                  55                  60
   Ser Lys Leu Asn Met Leu Lys Pro Asp Ser Asp Leu Cys Leu Lys Phe
   65                  70                  75                  80
   Ala Met Leu Cys Thr Leu Asn Asp Lys Cys Asp Arg Leu Arg Lys Ala
                   85                  90                  95
   Tyr Gly Glu Ala Cys Ser Gly Pro His Cys Gln Arg His Val Cys Leu
               100                 105                 110
   Arg Gln Leu Leu Thr Phe Phe Glu Lys Ala Ala Glu Pro His Ala Gln
           115                 120                 125
   Gly Leu Leu Leu Cys Pro Cys Ala Pro Asn Asp Arg Gly Cys Gly Glu
       130                 135                 140
   Arg Arg Arg Asn Thr Ile Ala Pro Asn Cys Ala Leu Pro Pro Val Ala
   145                 150                 155                 160
   Pro Asn Cys Leu Glu Leu Arg Arg Leu Cys Phe Ser Asp Pro Leu Cys
                   165                 170                 175
   Arg Ser Arg Leu Val Asp Phe Gln Thr His Cys His Pro Met Asp Ile
               180                 185                 190
   Leu Gly Thr Cys Ala Thr Glu Gln Ser Arg Cys Leu Arg Ala Tyr Leu
           195                 200                 205
   Gly Leu Ile Gly Thr Ala Met Thr Pro Asn Phe Val Ser Asn Val Asn
       210                 215                 220
   Thr Ser Val Ala Leu Ser Cys Thr Cys Arg Gly Ser Gly Asn Leu Gln
   225                 230                 235                 240
   Glu Glu Cys Glu Met Leu Glu Gly Phe Phe Ser His Asn Pro Cys Leu
                   245                 250                 255
   Thr Glu Ala Ile Ala Ala Lys Met Arg Phe His Ser Gln Leu Phe Ser
               260                 265                 270
   Gln Asp Trp Pro His Pro Thr Phe Ala Val Met Ala His Gln Asn Glu
           275                 280                 285
   Asn Pro Ala Val Arg Pro Gln Pro Trp Val Pro Ser Leu Phe Ser Cys
       290                 295                 300
   Thr Leu Pro Leu Ile Leu Leu Leu Ser Leu Trp
   305                 310                 315
(2) INFORMATION FOR SEQ ID NO:20:
   (i) SEQUENCE CHARACTERISTICS:
      (A) LENGTH: 1699 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 175..1374
   (xi) SEQUENCE DESCRIPTION: SEQ ID NO:20:
   TGTGGACGCG CGCTTCGGAG TTGGAGGGCG GCGCCCAGGA CCCTGGTGGG AGAGTGTGTG
   60
   CGTCGCGCTG GAGGGCGGGA GGCGGGGGCG GGAGGTGCCG GTCGAGGGAG CCCCGCTCTC
   120
   AGAGCTCCAG GGGAGGAGCG AGGGGAGCGC GGAGCCCGGC GCCTACAGCT CGCC ATG
   177
                                                               Met
   GTG CGC CCC CTG AAC CCG CGA CCG CTG CCG CCC GTA GTC CTG ATG TTG
   225
   Val Arg Pro Leu Asn Pro Arg Pro Leu Pro Pro Val Val Leu Met Leu
               320                 325                 330
   CTG CTG CTG CTG CCG CCG TCG CCG CTG CCT CTC GCA GCC GGA GAC CCC
   273
   Leu Leu Leu Leu Pro Pro Ser Pro Leu Pro Leu Ala Ala Gly Asp Pro
           335                 340                 345
   CTT CCC ACA GAA AGC CGA CTC ATG AAC AGC TGT CTC CAG GCC AGG AGG
   321
   Leu Pro Thr Glu Ser Arg Leu Met Asn Ser Cys Leu Gln Ala Arg Arg
       350                 355                 360
   AAG TGC CAG GCT GAT CCC ACC TGC AGT GCT GCC TAC CAC CAC CTG GAT
   369
   Lys Cys Gln Ala Asp Pro Thr Cys Ser Ala Ala Tyr His His Leu Asp
   365                 370                 375                 380
   TCC TGC ACC TCT AGC ATA AGC ACC CCA CTG CCC TCA GAG GAG CCT TCG
   417
   Ser Cys Thr Ser Ser Ile Ser Thr Pro Leu Pro Ser Glu Glu Pro Ser
                   385                 390                 395
   GTC CCT GCT GAC TGC CTG GAG GCA GCA CAG CAA CTC AGG AAC AGC TCT
   465
   Val Pro Ala Asp Cys Leu Glu Ala Ala Gln Gln Leu Arg Asn Ser Ser
   400 405 410
   CTG ATA GGC TGC ATG TGC CAC CGG CGC ATG AAG AAC CAG GTT GCC TGC
   513
   Leu Ile Gly Cys Met Cys His Arg Arg Met Lys Asn Gln Val Ala Cys
   415 420 425
   TTG GAC ATC TAT TGG ACC GTT CAC CGT GCC CGC AGC CTT GGT AAC TAT
   561
   Leu Asp Ile Tyr Trp Thr Val His Arg Ala Arg Ser Leu Gly Asn Tyr
   430 435 440
   GAG CTG GAT GTC TCC CCC TAT GAA GAC ACA GTG ACC AGC AAA CCC TGG
   609
   Glu Leu Asp Val Ser Pro Tyr Glu Asp Thr Val Thr Ser Lys Pro Trp
   445 450 455 460
   AAA ATG AAT CTC AGC AAA CTG AAC ATG CTC AAA CCA GAC TCA GAC CTC
   657
   Lys Met Asn Leu Ser Lys Leu Asn Met Leu Lys Pro Asp Ser Asp Leu
   465 470 475
   TGC CTC AAG TTT GCC ATG CTG TGT ACT CTC AAT GAC AAG TGT GAC CGG
   705
   Cys Leu Lys Phe Ala Met Leu Cys Thr Leu Asn Asp Lys Cys Asp Arg
   480 485 490
   CTG CGC AAG GCC TAC GGG GAG GCG TGC TCC GGG CCC CAC TGC CAG CGC
   753
   Leu Arg Lys Ala Tyr Gly Glu Ala Cys Ser Gly Pro His Cys Gln Arg
   495 500 505
   CAC GTC TGC CTC AGG CAG CTG CTC ACT TTC TTC GAG AAG GCC GCC GAG
   801
   His Val Cys Leu Arg Gln Leu Leu Thr Phe Phe Glu Lys Ala Ala Glu
   510 515 520
   CCC CAC GCG CAG GGC CTG CTA CTG TGC CCA TGT GCC CCC AAC GAC CGG
   849
   Pro His Ala Gln Gly Leu Leu Leu Cys Pro Cys Ala Pro Asn Asp Arg
   525 530 535 540
   GGC TGC GGG GAG CGC CGG CGC AAC ACC ATC GCC CCC AAC TGC GCG CTG
   897
   Gly Cys Gly Glu Arg Arg Arg Asn Thr Ile Ala Pro Asn Cys Ala Leu
   545 550 555
   CCG CCT GTG GCC CCC AAC TGC CTG GAG CTG CGG CGC CTC TGC TTC TCC
   945
   Pro Pro Val Ala Pro Asn Cys Leu Glu Leu Arg Arg Leu Cys Phe Ser
   560 565 570
   GAC CCG CTT TGC AGA TCA CGC CTG GTG GAT TTC CAG ACC CAC TGC CAT
   993
   Asp Pro Leu Cys Arg Ser Arg Leu Val Asp Phe Gln Thr His Cys His
   575 580 585
   CCC ATG GAC ATC CTA GGA ACT TGT GCA ACA GAG CAG TCC AGA TGT CTA
   1041
   Pro Met Asp Ile Leu Gly Thr Cys Ala Thr Glu Gln Ser Arg Cys Leu
   590 595 600
       CGA GCA TAC CTG GGG CTG ATT GGG ACT GCC ATG ACC CCC AAC TTT GTC
   1089
       Arg Ala Tyr Leu Gly Leu Ile Gly Thr Ala Met Thr Pro Asn Phe Val
       605                 610                 615                 620
       AGC AAT GTC AAC ACC AGT GTT GCC TTA AGC TGC ACC TGC CGA GGC AGT
   1137
       Ser Asn Val Asn Thr Ser Val Ala Leu Ser Cys Thr Cys Arg Gly Ser
                       625                 630                 635
       GGC AAC CTG CAG GAG GAG TGT GAA ATG CTG GAA GGG TTC TTC TCC CAC
   1185
       Gly Asn Leu Gln Glu Glu Cys Glu Met Leu Glu Gly Phe Phe Ser His
                   640                 645                 650
       AAC CCC TGC CTC ACG GAG GCC ATT GCA GCT AAG ATG CGT TTT CAC AGC
   1233
       Asn Pro Cys Leu Thr Glu Ala Ile Ala Ala Lys Met Arg Phe His Ser
               655                 660                 665
       CAA CTC TTC TCC CAG GAC TGG CCA CAC CCT ACC TTT GCT GTG ATG GCA
   1281
       Gln Leu Phe Ser Gln Asp Trp Pro His Pro Thr Phe Ala Val Met Ala
           670                 675                 680
       CAC CAG AAT GAA AAC CCT GCT GTG AGG CCA CAG CCC TGG GTG CCC TCT
   1329
       His Gln Asn Glu Asn Pro Ala Val Arg Pro Gln Pro Trp Val Pro Ser
       685                 690                 695                 700
       CTT TTC TCC TGC ACG CTT CCC TTG ATT CTG CTC CTG AGC CTA TGG
   1374
       Leu Phe Ser Cys Thr Leu Pro Leu Ile Leu Leu Leu Ser Leu Trp
                       705                 710                 715
       TAGCTGGACT TCCCCAGGGC CCTCTTCCCC TCCACCACAC CCAGGTGGAC TTGCAGCCCA
   1434
       CAAGGGGTGA GGAAAGGACA GCAGCAGGAA GGAGGTGCAG TGCGCAGATG AGGGCACAGG
   1494
       AGAAGCTAAG GGTTATGACC TCCAGATCCT TACTGGTCCA GTCCTCATTC CCTCCACCCC
   1554
       ATCTCCACTT CTGATTCATG CTGCCCCTCC TTGGTGGCCA CAATTTAGCC ATGTCATCTG
   1614
       GTGCCTGTGG GCCTTGCTTT ATTCCTATTA TTGTCCTAAA GTCTCTCTGG GCTCTTGGAT
   1674
       CATGATTAAA CCTTTGACTT AAAAA
   1699
(2) INFORMATION FOR SEQ ID NO:21:
   (i) SEQUENCE CHARACTERISTICS:
      (A) LENGTH: 400 amino acids
      (B) TYPE: amino acid
      (D) TOPOLOGY: linear
   (ii) MOLECULE TYPE: protein
   (xi) SEQUENCE DESCRIPTION: SEQ ID NO:21:
   Met Val Arg Pro Leu Asn Pro Arg Pro Leu Pro Pro Val Val Leu Met
    1               5                  10                  15
   Leu Leu Leu Leu Leu Pro Pro Ser Pro Leu Pro Leu Ala Ala Gly Asp
               20                  25                  30
   Pro Leu Pro Thr Glu Ser Arg Leu Met Asn Ser Cys Leu Gln Ala Arg
           35                  40                  45
   Arg Lys Cys Gln Ala Asp Pro Thr Cys Ser Ala Ala Tyr His His Leu
       50                  55                  60
   Asp Ser Cys Thr Ser Ser Ile Ser Thr Pro Leu Pro Ser Glu Glu Pro
   65                  70                  75                  80
   Ser Val Pro Ala Asp Cys Leu Glu Ala Ala Gln Gln Leu Arg Asn Ser
                   85                  90                  95
   Ser Leu Ile Gly Cys Met Cys His Arg Arg Met Lys Asn Gln Val Ala
               100                 105                 110
   Cys Leu Asp Ile Tyr Trp Thr Val His Arg Ala Arg Ser Leu Gly Asn
           115                 120                 125
   Tyr Glu Leu Asp Val Ser Pro Tyr Glu Asp Thr Val Thr Ser Lys Pro
       130                 135                 140
   Trp Lys Met Asn Leu Ser Lys Leu Asn Met Leu Lys Pro Asp Ser Asp
   145                 150                 155                 160
   Leu Cys Leu Lys Phe Ala Met Leu Cys Thr Leu Asn Asp Lys Cys Asp
                   165                 170                 175
   Arg Leu Arg Lys Ala Tyr Gly Glu Ala Cys Ser Gly Pro His Cys Gln
               180                 185                 190
   Arg His Val Cys Leu Arg Gln Leu Leu Thr Phe Phe Glu Lys Ala Ala
           195                 200                 205
   Glu Pro His Ala Gln Gly Leu Leu Leu Cys Pro Cys Ala Pro Asn Asp
       210                 215                 220
   Arg Gly Cys Gly Glu Arg Arg Arg Asn Thr Ile Ala Pro Asn Cys Ala
   225                 230                 235                 240
   Leu Pro Pro Val Ala Pro Asn Cys Leu Glu Leu Arg Arg Leu Cys Phe
                   245                 250                 255
   Ser Asp Pro Leu Cys Arg Ser Arg Leu Val Asp Phe Gln Thr His Cys
               260                 265                 270
   His Pro Met Asp Ile Leu Gly Thr Cys Ala Thr Glu Gln Ser Arg Cys
           275                 280                 285
   Leu Arg Ala Tyr Leu Gly Leu Ile Gly Thr Ala Met Thr Pro Asn Phe
       290                 295                 300
   Val Ser Asn Val Asn Thr Ser Val Ala Leu Ser Cys Thr Cys Arg Gly
   305                 310                 315                 320
   Ser Gly Asn Leu Gln Glu Glu Cys Glu Met Leu Glu Gly Phe Phe Ser
                   325                 330                 335
   His Asn Pro Cys Leu Thr Glu Ala Ile Ala Ala Lys Met Arg Phe His
               340                 345                 350
   Ser Gln Leu Phe Ser Gln Asp Trp Pro His Pro Thr Phe Ala Val Met
   355 360 365
   Ala His Gln Asn Glu Asn Pro Ala Val Arg Pro Gln Pro Trp Val Pro
   370 375 380
   Ser Leu Phe Ser Cys Thr Leu Pro Leu Ile Leu Leu Leu Ser Leu Trp
   385 390 395 400